# EUROPÄISCHE PATENTSCHRIFT

(11) **EP 0 827 499 B1**
(45) Veröffentlichungstag und Bekanntmachung des Hinweises auf die Patenterteilung: **01.09.2004**
(21) Anmeldenummer: 96914186.0
(22) Anmeldetag: 09.05.1996
(51) Int. Cl.: C07D 265/08, C07D 295/125, A01N 43/86

(54) **VERFAHREN ZUR HERSTELLUNG VON 5,6-DIHYDRO-1,3-OXAZINEN**
PROCESS FOR PRODUCING 5,6-DIHYDRO-1,3-OXAZINES
PROCEDE DE PRODUCTION DE 5,6-DIHYDRO-1,3-OXAZINES

(30) Priorität: 22.05.1995 DE 19518681
(43) Veröffentlichungstag der Anmeldung: 11.03.1998
(73) Patentinhaber: Bayer CropScience AG, 40789 Monheim (DE)
(72) Erfinder: ALIG, Bernd, D-53639 Königswinter (DE)
(86) Internationale Anmeldenummer: PCT/EP1996/001954
(87) Internationale Veröffentlichungsnummer: WO 1996/037484

(56) Entgegenhaltungen:
- EP-A- 0 394 850
- WO-A-93/22296
- DE-A- 1 545 671
- DE-A- 1 670 475
- DE-A- 2 049 160

## Beschreibung

Die Erfindung betrifft ein neues Verfahren zur Herstellung von teilweise bekannten 5,6-Dihydro-1,3-oxazinen, neue 5,6-Dihydro-1,3-oxazine, sowie neue Zwischenprodukte zu ihrer Herstellung und ihre Verwendung zur Bekämpfung tierischer Schädlinge.

Es ist bekannt, daß man 5,6-Dihydro-1,3-oxazine erhält, wenn man Aminoalkohole der Formel (A) mit Carbonsäuren der Formel (B) in Gegenwart eines wasserentziehenden Mittels gemäß dem folgenden Reaktionsschema umsetzt: 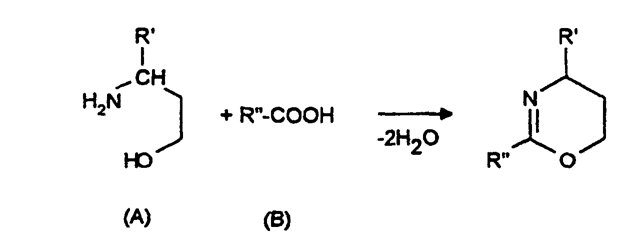 (vgl. hierzu EP-A 0 635 500 und WO-A 94/14783)

Weiter ist bekannt, daß man 5,6-Dihydro-1,3-oxazine erhält, wenn man Aminoalkohole der Formel (A) zunächst mit Säurechloriden der Formel (C) umsetzt und anschließend die entstehenden Amidalkohole der Formel (D) mit einem wasserentziehenden Mittel gemäß dem folgenden Reaktionsschema cyclisiert: 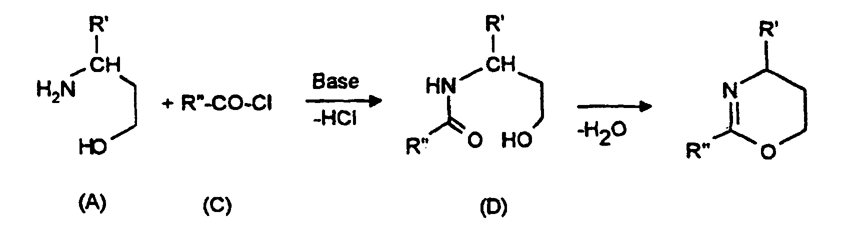 (vgl. hierzu EP-A 0 635 500 und WO-A 94/14783)

Ferner ist bekannt, daß man 5,6-Dihydro-1,3-oxazine erhält, wenn man Aminoalkohole der Formel (E) mit Nitrilen der Formel (F) in Gegenwart von RhodiumKomplexen, gegebenenfalls unter Zusatz von Diphosphinen gemäß dem folgenden Reaktionsschema umsetzt: 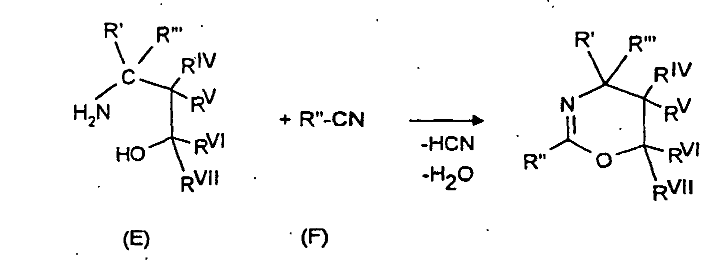 (vgl. hierzu JP-A 06 298 746)

Bei diesen bekannten Synthesemethoden sind jedoch - vor allem mit Blick auf das Gesamtverfahren, d.h. unter Einschluß der Herstellung der als Ausgangsstoffe benötigten Aminoalkohole der Formeln (A) bzw. (E) - die erzielbaren Ausbeuten der Produkte nicht zufriedenstellend: Die Aminoalkohole sind nur über mehrere Stufen, unter Verwendung teurer Vorprodukte und Reduktionsmittel in unbefriedigenden Ausbeuten erhältlich, wobei aufgrund größerer Mengen an Reduktions- und Lösungsmitteln und dem teilweisen Arbeiten unter Inertgas zusätzliche technische Schwierigkeiten auftreten.

Es wurde nun gefunden, daß man 5,6-Dihydro-1,3-oxazine der Formel (I) 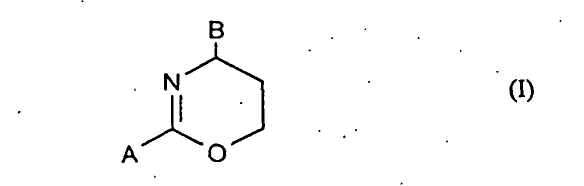 in welcher
- A: für substituiertes Phenyl oder für jeweils gegebenenfalls substituiertes Naphthyl, Pyridyl, Thienyl oder Pyrazolyl steht und
- B: für Alkyl, Alkoxyalkyl, Alkylthioalkyl, Halogenalkyl, Alkenyl, gegebenenfalls substituiertes Cycloalkyl oder jeweils gegebenenfalls substituiertes Phenyl, Phenylalkyl, Phenylalkenyl, Phenoxyalkyl, Phenylthioalkyl, Pyridyl, 2- oder 3-Pyrrolyl steht,
erhält,
wenn man Amid-Derivate der Formel (II) 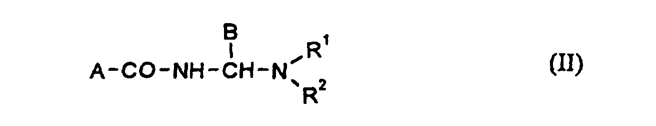 in welcher
- A und B: die oben angegebene Bedeutung haben und
- R¹ und R²: gleich oder verschieden sind und jeweils für Alkyl stehen oder gemeinsam mit dem N-Atom, an das sie gebunden sind, für einen gegebenenfalls substituierten Heterocyclus stehen,
mit Ethylen in Gegenwart von Chlorwasserstoff-Gas und eines Katalysators, gegebenenfalls in Gegenwart eines Verdünnungsmittels umsetzt.

Überraschenderweise können nach dem erfindungsgemäßen Verfahren 5,6-Dihydro-1,3-oxazine der Formel (I) in guten Ausbeuten und in hoher Reinheit erhalten werden, obwohl aufgrund der geringen Reaktivität des Ethylens keine merkliche Reaktion zu erwarten war.

Die erfindungsgemäße Umsetzung hat somit den Vorteil einer günstigeren Verfahrensdurchführung bei gleichzeitiger Ausbeuteverbesserung, insbesondere im Hinblick auf das Gesamtverfahren, d.h. unter Einbeziehung der Vorproduktherstellung (vgl. auch die Herstellungsbeispiele).

Die erfindungsgemäß herzustellenden Verbindungen sind durch die Formel (I) allgemein definiert. Bevorzugte Substituenten bzw. Bereiche der in den oben und nachstehend erwähnten Formeln aufgeführten Reste werden im folgenden erläutert.
- A: steht bevorzugt für Phenyl, das einfach bis fünffach, gleich oder verschieden substituiert ist durch Halogen, C₁-C₆-Alkyl, C₁-C₆-Alkoxy, C₁-C₆-Alkylthio, C₁-C₆-Halogenalkyl, C₁-C₆-Halogenalkoxy, C₁-C₆-Halogenalkylthio, Nitro, Cyano oder durch gegebenenfalls einfach bis fünffach, gleich oder verschieden durch Halogen, C₁-C₆-Alkyl, C₁-C₆-Halogenalkyl, C₁-C₆-Alkoxy, C₁-C₆-Halogenalkoxy, C₁-C₆-Alkylthio oder C₁-C₆-Halogenalkylthio substituiertes Phenyl,
für gegebenenfalls einfach bis dreifach, gleich oder verschieden durch Halogen, C₁-C₆-Alkyl, C₁-C₆-Alkoxy oder C₁-C₆-Halogenalkoxy substituiertes Naphthyl,
für gegebenenfalls einfach bis dreifach, gleich oder verschieden durch Halogen, C₁-C₆-Alkyl, C₁-C₆-Alkoxy, C₁-C₆-Halogenalkyl, C₁-C₆-Halogenalkoxy oder Cyano substituiertes Pyridyl,
für gegebenenfalls einfach bis dreifach, gleich oder verschieden durch Halogen oder C₁-C₆-Alkyl substituiertes Thienyl,
oder für gegebenenfalls einfach bis dreifach, gleich oder verschieden durch Halogen oder C₁-C₃-Alkyl substituiertes Pyrazolyl.
- B: steht bevorzugt für C₁-C₆-Alkyl, C₁-C₆-Alkoxy-C₁-C₆-alkyl, C₁-C₆-Alkylthio-C₁-C₆-alkyl, C₁-C₆-Halogenalkyl oder C₂-C₆-Alkenyl,
für gegebenenfalls einfach bis fünffach, gleich oder verschieden durch Halogen, C₁-C₆-Alkyl, C₁-C₆-Alkoxy, C₁-C₆-Halogenalkyl oder C₁-C₆-Halogenalkoxy substituiertes C₃-C₇-Cycloalkyl,
für gegebenenfalls einfach bis dreifach, gleich oder verschieden durch Halogen, C₁-C₆-Alkyl, C₁-C₆-Alkoxy, C₁-C₆-Halogenalkyl, C₁-C₆-Halogenalkoxy oder Cyano substituiertes Pyridyl,
für jeweils gegebenenfalls einfach oder zweifach, gleich oder verschieden durch Halogen oder C₁-C₄-Alkyl substituiertes α-Pyrrolyl oder β-Pyrrolyl,
für durch Hydroxy, CHO, Cyano, Carboxyl, Poly-C₁-C₈-alkoxy-C₁-C₈-alkyl oder C₁-C₈-Alkoxycarbonyl substituiertes Phenyl oder
für jeweils gegebenenfalls einfach bis fünffach, gleich oder verschieden im Phenylring substituiertes Phenyl, Benzyl, Pheneth-1-yl, Pheneth-2-yl, Phenoxymethyl, Phenylthiomethyl, Phenylthioeth-1-yl, Phenoxyeth-1-yl, Phenoxyeth-2-yl oder Styryl, wobei jeweils als Substituenten in Frage kommen Halogen,
C₁-C₁₈-Alkyl,
C₁-C₈-Alkoxy-C₁-C₈-alkyl,
C₁-C₈-Halogenalkoxy,
C₁-C₄-Halogenalkyl,
C₁-C₁₈-Alkoxy, das gegebenenfalls durch weitere 1 bis 3 Sauerstoffatome unterbrochen ist,
C₁-C₁₈-Alkylthio,
C₁-C₈-Halogenalkylthio,
Tri-(C₁-C₈)-alkylsilyl,
Phenyl-di-(C₁-C₈)-alkylsilyl,
3,4-Difluormethylendioxo,
3,4-Tetrafluorethylendioxo,
eine anellierte Benzogruppe,
eine anellierte C₃-C₄-Alkandiylgruppe,
gegebenenfalls durch Halogen, C₁-C₄-Alkyl oder C₃-C₆-Cycloalkyl substituiertes Benzyliminooxymethyl,
jeweils gegebenenfalls durch C₁-C₆-Alkyl, C₁-C₆-Alkoxy, Cyclohexyl oder Phenyl substituiertes Cyclohexyl oder Cyclohexyloxy,
gegebenenfalls einfach oder zweifach, gleich oder verschieden durch Halogen, C₁-C₄-Alkyl oder C₁-C₄-Halogenalkyl substituiertes Pyridyloxy,
jeweils gegebenenfalls einfach bis fünffach, gleich oder verschieden durch Halogen, C₁-C₁₂-Alkyl, C₁-C₄-Halogenalkyl, C₁-C₁₂-Alkoxy, C₁-C₆-Halogenalkoxy, C₁-C₆-Alkoxy-C₁-C₆-alkyl, C₁-C₆-Alkoxy-ethylenoxy, C₁-C₆-Alkylthio oder C₁-C₆-Halogenalkylthio substituiertes Phenyl, Phenyl-C₁-C₆-alkyl, Phenoxy, Phenylthio, Phenyl-C₁-C₆-alkyloxy oder Benzylthio.
- A: steht besonders bevorzugt für Phenyl, das einfach bis vierfach, gleich oder verschieden substituiert ist durch Fluor, Chlor, Brom, C₁-C₃-Alkyl, C₁-C₃-Alkoxy, C₁-C₃-Alkylthio, einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃, SCHF₂, Nitro, Cyano, oder durch gegebenenfalls einfach bis vierfach, gleich oder verschieden durch Fluor, Chlor, Brom, C₁-C₄-Alkyl, C₁-C₄-Alkoxy, C₁-C₄-Alkylthio, einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃ oder SCHF₂ substituiertes Phenyl,
für gegebenenfalls einfach bis dreifach, gleich oder verschieden durch Fluor, Chlor, C₁-C₆-Alkyl, C₁-C₆-Alkoxy oder einfach bis dreifach, gleich oder verschieden durch Fluor oder Chlor substituiertes C₁-C₆-Alkoxy substituiertes Naphthyl,
für gegebenenfalls einfach oder zweifach, gleich oder verschieden durch Fluor, Chlor, C₁-C₃-Alkyl, C₁-C₃-Alkoxy, CF₃, OCF₃ oder Cyano substituiertes Pyridyl,
für gegebenenfalls einfach oder zweifach, gleich oder verschieden durch Chlor, Brom, Methyl oder Ethyl substituiertes Thienyl oder
für gegebenenfalls einfach bis dreifach, gleich oder verschieden durch Fluor, Chlor, Brom oder C₁-C₃-Alkyl substituiertes Pyrazolyl.
- B: steht besonders bevorzugt für C₁-C₄-Alkyl, C₁-C₄-Alkoxy-C₁-C₄-alkyl, C₁-C₄-Alkylthio-C₁-C₄-alkyl, C₁-C₄-Halogenalkyl oder C₂-C₆-Alkenyl,
für jeweils gegebenenfalls einfach bis dreifach, gleich oder verschieden durch Fluor, Chlor, Brom, C₁-C₃-Alkyl, C₁-C₃-Alkoxy; CF₃ oder OCF₃ substituiertes Cyclopropyl, Cyclopentyl oder Cyclohexyl,
für gegebenenfalls einfach oder zweifach, gleich oder verschieden durch Fluor, Chlor, C₁-C₃-Alkyl, C₁-C₃-Alkoxy, CF₃, OCF₃ oder Cyano substituiertes Pyridyl,
für jeweils gegebenenfalls einfach oder zweifach, gleich oder verschieden durch Fluor, Chlor oder C₁-C₃-Alkyl substituiertes α-Pyrrolyl oder β-Pyrrolyl,
für durch CHO, Cyano, Hydroxy, Carboxyl, Poly-C₁-C₈-alkoxy-C₁-C₆-alkyl oder C₁-C₈-Alkoxycarbonyl substituiertes Phenyl oder
für jeweils gegebenenfalls einfach bis vierfach, gleich oder verschieden im Phenylring substituiertes Phenyl, Benzyl, Pheneth-1-yl, Pheneth-2-yl, Phenoxymethyl, Phenylthiomethyl, Phenylthioeth-1-yl, Phenoxyeth-1-yl, Phenoxyeth-2-yl, oder Styryl, wobei jeweils als Substituenten in Frage kommen Fluor, Chlor, Brom,
C₁-C₁₈-Alkyl,
C₁-C₆-Alkoxy-C₁-C₈-alkyl,
einfach bis sechsfach, gleich oder verschieden durch Fluor oder Chlor substituiertes C₁-C₈-Alkoxy,
einfach bis fünffach, gleich oder verschieden durch Fluor oder Chlor substituiertes C₁-C₂-Alkyl,
C₁-C₁₈-Alkoxy oder -(OC₂H₄)₁₋₃-O-C₁-C₆-alkyl,
C₁-C₁₅-Alkylthio,
einfach bis sechsfach, gleich oder verschieden durch Fluor oder Chlor substituiertes C₁-C₈-Alkylthio,
Tri-(C₁-C₆)-alkylsilyl,
Phenyl-di-(C₁-C₆)-alkylsilyl,
3,4-Difluormethylendioxo,
3,4-Tetrafluorethylendioxo,
eine anellierte Benzogruppe,
eine anellierte C₄-Alkandiylgruppe,
die Gruppen 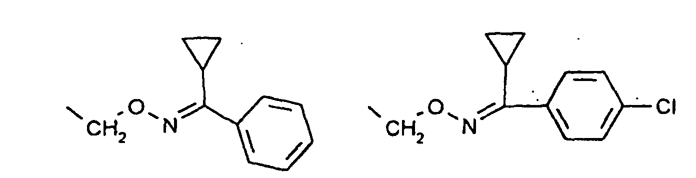 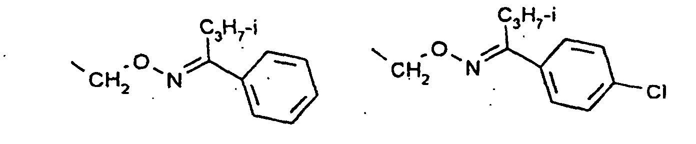 jeweils gegebenenfalls durch C₁-C₄-Alkyl, C₁-C₄-Alkoxy, Cyclohexyl oder Phenyl substituiertes Cyclohexyl oder Cyclohexyloxy,
gegebenenfalls einfach oder zweifach, gleich oder verschieden durch Fluor, Chlor oder CF₃ substituiertes Pyridyloxy,
jeweils gegebenenfalls einfach bis vierfach, gleich oder verschieden durch Fluor, Chlor, Brom, C₁-C₁₂-Alkyl, einfach bis sechsfach, gleich oder verschieden durch Fluor oder Chlor substituiertes C₁-C₄-Alkyl, C₁-C₁₂-Alkoxy, einfach bis sechsfach, gleich oder verschieden durch Fluor oder Chlor substituiertes C₁-C₄-Alkoxy, C₁-C₄-Alkoxy-C₁-C₄-alkyl, C₁-C₄-Alkoxyethylenoxy, C₁-C₄-Alkylthio oder einfach bis sechsfach, gleich oder verschieden durch Fluor oder Chlor substituiertes C₁-C₄-Alkylthio substituiertes Phenyl, Phenyl-C₁-C₆-alkyl, Phenoxy, Phenylthio, Phenyl-C₁-C₆-alkyloxy oder Benzylthio.
- A: steht ganz besonders bevorzugt für Phenyl, das einfach bis dreifach, gleich oder verschieden substituiert ist durch Fluor, Chlor, Brom, C₁-C₃-Alkyl, C₁-C₃-Alkoxy, C₁-C₃-Alkylthio, einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃, SCHF₂, Nitro, Cyano, oder durch gegebenenfalls einfach bis vierfach, gleich oder verschieden durch Fluor, Chlor, Brom, C₁-C₃-Alkyl, C₁-C₃-Alkoxy, C₁-C₃-Alkylthio, einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃ oder SCHF₂ substituiertes Phenyl.
- B: steht ganz besonders bevorzugt für C₁-C₄-Alkyl, C₁-C₂-Alkoxy-C₁-C₄-alkyl, C₁-C₂-Alkylthio-C₁-C₄-alkyl, C₁-C₂-Halogenalkyl oder C₂-C₄-Alkenyl,
für jeweils gegebenenfalls einfach bis dreifach, gleich oder verschieden durch Fluor, Chlor, CH₃, C₂H₅, OCH₃, CF₃ oder OCF₃ substituiertes Cyclopropyl, Cyclopentyl oder Cyclohexyl,
für gegebenenfalls einfach oder zweifach, gleich oder verschieden durch Fluor, Chlor, CH₃, C₂H₅, OCH₃, CF₃, OCF₃ oder Cyano substituiertes Pyridyl,
für jeweils gegebenenfalls einfach oder zweifach, gleich oder verschieden durch Fluor, Chlor, CH₃ oder C₂H₅ substituiertes α-Pyrrolyl oder β-Pyrrolyl,
für durch CHO, Hydroxy, Cyano, Carboxyl, Di-C₁-C₆-alkoxy-C₁-C₄-alkyl oder C₁-C₆-Alkoxycarbonyl substituiertes Phenyl oder
für jeweils gegebenenfalls einfach bis dreifach, gleich oder verschieden im Phenylring substituiertes Phenyl, Benzyl, Pheneth-1-yl, Pheneth-2-yl, Phenoxymethyl, Phenylthiomethyl, Phenylthioeth-1-yl, Phenoxyeth-1-yl, Phenoxyeth-2-yl, oder Styryl, wobei jeweils als Substituenten in Frage kommen Fluor, Chlor, Brom,
C₁-C₁₈-Alkyl,
C₁-C₆-Alkoxy-C₁-C₈-alkyl,
einfach bis sechsfach, gleich oder verschieden durch Fluor oder Chlor substituiertes C₁-C₈-Alkoxy,
einfach bis fünffach, gleich oder verschieden durch Fluor oder Chlor substituiertes C₁-C₂-Alkyl,
C₁-C₁₈-Alkoxy oder -(OC₂H₄)₁₋₃-O-C₁-C₆-alkyl,
C₁-C₁₅-Alkylthio,
einfach bis sechsfach, gleich oder verschieden durch Fluor oder Chlor substituiertes C₁-C₈-Alkylthio,
Tri-(C₁-C₆)-alkylsilyl,
Phenyl-di-(C₁-C₆)-alkylsilyl,
3,4-Difluormethylendioxo,
3,4-Tetrafluorethylendioxo,
eine anellierte Benzogruppe,
eine anellierte C₄-Alkandiylgruppe,
die Gruppen 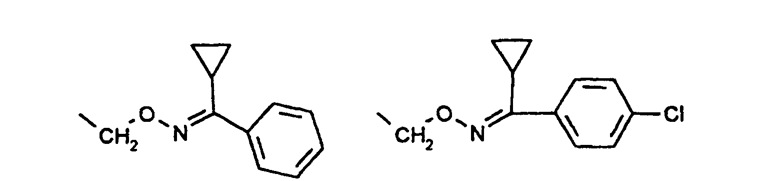 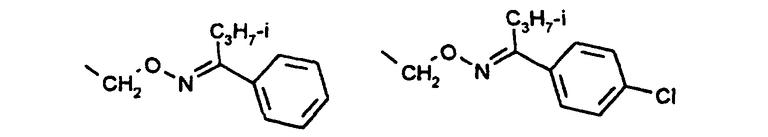 jeweils gegebenenfalls durch C₁-C₄-Alkyl, C₁-C₄-Alkoxy, Cyclohexyl oder Phenyl substituiertes Cyclohexyl oder Cyclohexyloxy,
gegebenenfalls einfach oder zweifach, gleich oder verschieden durch Fluor, Chlor oder CF₃ substituiertes Pyridyloxy,
jeweils gegebenenfalls einfach bis vierfach, gleich oder verschieden durch Fluor, Chlor, Brom, C₁-C₁₂-Alkyl, einfach bis sechsfach durch Fluor oder Chlor substituiertes C₁-C₄-Alkyl, C₁-C₁₂-Alkoxy, einfach bis sechsfach, gleich oder verschieden durch Fluor oder Chlor substituiertes C₁-C₄-Alkoxy, C₁-C₄-Alkoxy-C₁-C₄-alkyl, C₁-C₄-Alkoxy-ethylenoxy, C₁-C₄-Alkylthio oder einfach bis sechsfach, gleich oder verschieden durch Fluor oder Chlor substituiertes C₁-C₄-Alkylthio substituiertes Phenyl, Phenyl-C₁-C₆-alkyl, Phenoxy, Phenylthio, Phenyl-C₁-C₆-alkyloxy oder Benzylthio.

Bevorzugte, erfindungsgemäß herstellbare Verbindungen sind Stoffe der Formel (Ia) 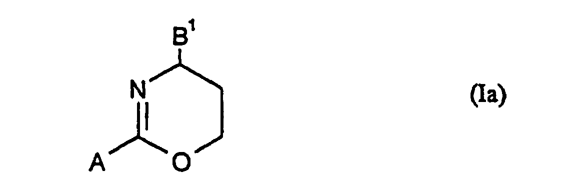 in welcher
- A: für die oben genannten allgemeinen, vorzugsweisen, besonders bevorzugten und ganz besonders bevorzugten Bedeutungen steht und
- B¹: für einfach bis fünffach, gleich oder verschieden substituiertes Phenyl steht, wobei als Substituenten die oben unter B für den Phenylrest als bevorzugt, besonders bevorzugt und ganz besonders bevorzugt genannten Substituenten in Frage kommen.

Eine besonders bevorzugte Gruppe von erfindungsgemäß herstellbaren Stoffen sind Verbindungen der Formel (I) 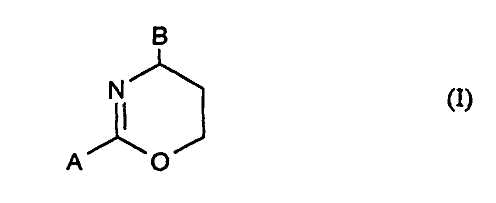 in welcher
- A: für einfach oder zweifach, gleich oder verschieden durch Fluor oder Chlor substituiertes Phenyl steht und
- B: für einfach bis dreifach, gleich oder verschieden durch Fluor, Chlor, Brom, C₁-C₈-Alkyl, C₁-C₈-Alkoxy, C₁-C₄-Halogenalkyl, C₁-C₄-Halogenalkoxy, C₁-C₈-Alkylthio oder C₃-C₆-Cycloalkyl substituiertes Phenyl oder
für einfach durch jeweils gegebenenfalls einfach oder zweifach, gleich oder verschieden im Phenylteil durch Fluor, Chlor, Brom, C₁-C₆-Alkyl, C₁-C₆-Alkoxy, C₁-C₄-Halogenalkyl oder C₁-C₄-Halogenalkoxy substituiertes Phenyl, Phenyl-C₁-C₃-alkyl, Phenyloxy oder Benzyloxy substituiertes Phenyl,
für jeweils gegebenenfalls einfach oder zweifach, gleich oder verschieden durch Fluor, Chlor, Methyl, Ethyl oder Trifluormethyl substituiertes Pyridyl, 2- oder 3-Pyrrolyl oder
für durch CHO, Hydroxy, Cyano, Carboxyl, Di-C₁-C₆-alkoxy-C₁-C₄-alkyl oder C₁-C₆-Alkoxycarbonyl substituiertes Phenyl steht.

Verwendet man beispielsweise N-[Morpholin-4-yl-(4-tert.-butylphenyl)-methyl]-2,6-difluorbenzamid und Ethylen als Ausgangsstoffe in Gegenwart von HCl-Gas und Titantetrachlorid als Katalysator, so kann der Reaktionsablauf des erfindungsgemäßen Verfahrens durch das folgende Formelschema dargestellt werden:

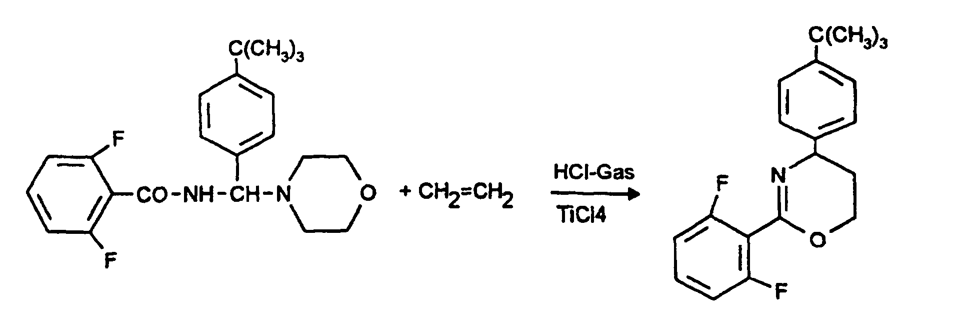

Die beim erfindungsgemäßen Verfahren als Ausgangsstoffe zu verwendenden Amid-Derivate sind durch die Formel (II) allgemein definiert. In der Formel (II) haben A und B vorzugsweise bzw. insbesondere diejenigen Bedeutungen, die bereits oben im Zusammenhang mit der Beschreibung der erfindungsgemäß hertstellbaren Verbindungen der Formel (I) als bevorzugt, besonders bevorzugt bzw. ganz besonders bevorzugt für A und B angegeben wurden.
- R¹ und R²: sind gleich oder verschieden und stehen vorzugsweise für C₁-C₄-Alkyl, insbesondere für Methyl, Ethyl, n- oder i-Propyl oder vorzugsweise gemeinsam mit dem N-Atom, an das sie gebunden sind, für einen gegebenenfalls einfach bis fünffach, bevorzugt einfach oder zweifach gleich oder verschieden substituierten 5- oder 6-gliedrigen Heterocyclus, der gegebenenfalls ein oder zwei weitere Heteroatome, bevorzugt ein weiteres Heteroatom, wie O, S oder NR enthalten kann, wobei als Substituenten insbesondere C₁-C₄-Alkyl und C₁-C₄-Alkoxy genannt seien und wobei R für Alkyl, vorzugsweise C₁-C₄-Alkyl, oder für gegebenenfalls substituiertes Aryl steht, insbesondere für gegebenenfalls einfach oder zweifach, gleich oder verschieden substituiertes Phenyl, wobei als Substituenten vorzugsweise genannt seien: Halogen, C₁-C₄-Alkyl, C₁-C₄-Halogenalkyl, C₁-C₄-Alkoxy, C₁-C₄-Halogenalkoxy, C₁-C₄-Alkylthio sowie C₁-C₄-Halogenalkylthio.

Einige Amid-Derivate der Formel (II), in welchen B für Wasserstoff steht, sind bereits bekannt (vgl. z.B. Liebigs Annalen 343, 207 (1905) und Bull. Soc. Chim. France 1979, II, 409-414).

Neu und ebenfalls Gegenstand dieser Anmeldung sind Amid-Derivate der Formel (IIa) 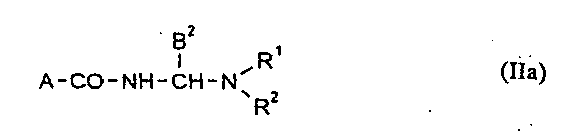 in welcher
- A, R¹ und R²: die oben angegebene Bedeutung haben und
- B²: die oben für B angegebene Bedeutung hat.

Man erhält die Amid-Derivate der Formel (IIa) beispielsweise, indem man Amide der Formel (III)

A-CO-NH₂ (III)

in welcher
- A: die oben angegebene Bedeutung hat,
mit Aldehyden der Formel (IV)

B²-CHO (IV)

in welcher
- B²: die oben angegebene Bedeutung hat,
und mit Aminen der Formel (V) 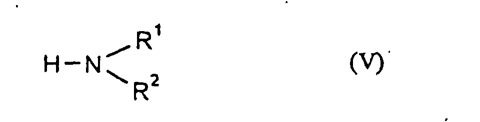 in welcher
- R¹ und R²: die oben angegebene Bedeutung haben,
gegebenenfalls in Gegenwart eines Verdünnungsmittels umsetzt.

Verwendet man beispielsweise 2,6-Difluorbenzoesäureamid, 4-tert.-Butylbenzaldehyd und Morpholin als Ausgangsstoffe, so kann der Reaktionsablauf des Verfahrens zur Herstellung der Amid-Derivate der Formel (II) durch das folgende Formelschema skizziert werden:

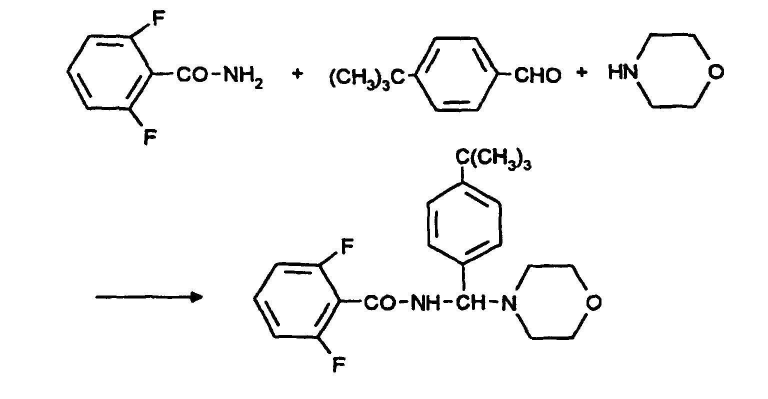

Die zur Herstellung der Amid-Derivate der Formel (II) als Ausgangsstoffe benötigten Amide der Formel (III), Aldehyde der Formel (IV) und sekundären Amine der Formel (V) sind allgemein bekannt bzw. in allgemein bekannter Art und Weise erhältlich.

Als Verdünnungsmittel kommen bei der Durchführung des Verfahrens zur Herstellung der Amid-Derivate der Formel (II) alle für derartige Umsetzungen üblichen, inerten orgenischen Solventien in Betracht. Vorzugsweise verwendbar sind Alkohole, wie Methanol oder Ethanol; Ester, wie Essigsäuremethylester oder Essisäureethylester; Ether, wie Diethylether, Methyl-tert.-butyl-ether, Ethylenglykol-dimethylether, Tetrahydrofuran oder Dioxan, ferner Nitrile, wie Acetonitril; außerdem gegebenenfalls halogenierte aliphatische, cycloaliphatische oder aromatische Kohlenwasserstoffe, wie Methylenchlorid, Chloroform, Tetrachlorkohlenstoff, Hexan, Cyclohexan, Benzol, Toluol, Xylol oder Chlorbenzol; sowie Sulfoxide, wie Dimethylsulfoxid.

Die Reaktionstemperaturen können bei der Durchführung des Verfahrens zur Herstellung der Amid-Derivate der Formel (II) innerhalb eines größeren Bereiches variiert werden. Im allgemeinen arbeitet man bei Temperaturen zwischen 0 und 120°C, vorzugsweise zwischen 10 und 100°C.

Bei der Durchführung des Verfahrens zur Herstellung der Amid-Derivate der Formel (II) setzt man pro Mol Amid der Formel (III) im allgemeinen 1,0 bis 1,5 Mol, vorzugsweise 1,0 bis 1,2 Mol an Aldehyd der Formel (IV) sowie 1,0 bis 2,0 Mol, vorzugsweise 1,0 bis 1,2 Mol an sekundärem Amin der Formel (V) ein.

Die Reaktionsdurchführung, Aufarbeitung und Isolierung der Reaktionsprodukte erfolgt nach allgemein üblichen Methoden.

Die Amid-Derivate der Formel (II) können gegebenenfalls auch direkt, d.h. ohne isoliert zu werden, gemäß dem erfindungsgemäßen Verfahren mit Ethylen zu den Endprodukten der Formel (I) umgesetzt werden (vgl. auch die Herstellungsbeispiele).

Die neuen Amid-Derivate der Formel (IIa) zeigen auch gute biologische Wirkungen bei der Verwendung zur Bekämpfung von tierischen Schädlingen.

Das erfindungsgemäße Verfahren zur Herstellung der Verbindungen der Formel (I) wird in Gegenwart von Chlorwasserstoff-Gas und eines Katalysators durchgeführt. Als Katalysatoren kommen Lewis-Säuren, vorzugsweise Übergangsmetallhalogenide in Frage, insbesondere Titantetrachlorid, Zinntetrachlorid und Zinkdichlorid.

Das erfindungsgemäße Verfahren wird vorzugsweise in Gegenwart eines Verdünnungsmittels durchgeführt.

Als Verdünnungsmittel kommen übliche organische Lösungsmittel in Betracht, z.B. gegebenenfalls halogenierte aliphatische, cycloaliphatische oder aromatische Kohlenwasserstoffe. Hierzu gehören beispielsweise Chlorbenzol, Dichlorbenzol, Petrolether, Hexan, Cyclohexan, Dichlormethan, Chloroform und Tetrachlormethan.

Die Reaktionstemperaturen können bei der Durchführung des erfindungsgemäßen Verfahrens in einem größeren Bereich variiert werden. Im allgemeinen arbeitet man bei Temperaturen zwischen -120°C und +120°C, vorzugsweise bei Temperaturen zwischen 10°C und 100°C.

Bei dieser Temperatur wird bevorzugt in einem geschlossenen Gefäß (Autoklaven) unter dem sich einstellenden Eigendruck der Reaktionsmischung gearbeitet. Es ist aber auch möglich, das Ethylen bei Normaldruck durch die Reaktionsmischung zu leiten, wobei man das nicht verbrauchte Ethylen gegebenenfalls im Kreislauf zurückführt.

Eine weitere Möglichkeit besteht darin, das Ethylen bei tiefen Temperaturen (ca. -110°C) einzukondensieren und die Reaktion bei dieser Temperatur durchzuführen.

Zur Durchführung des erfindungsgemäßen Verfahrens setzt man pro Mol Amid-Derivat der Formel (II) im allgmeinen zwischen 1 und 5 Mol, vorzugsweise zwischen 1 bis 3 Mol Ethylen und jeweils zwischen 1 und 5 Mol, vorzugsweise zwischen 1 bis 4 Mol Chlorwasserstoffgas sowie Katalysator ein.

Die Aufarbeitung kann auf übliche Weise durchgeführt werden; vorzugsweise wird das Reaktionsgemisch unter Kühlung bei Temperaturen zwischen 0°C und 10°C alkalisch gestellt und das Endprodukt in allgemein üblicher Art und Weise isoliert.

Die nach dem erfindungsgemäßen Verfahren herzustellenden 5,6-Dihydro-1,3-oxazine der Formel (I) sind weitgehend bekannt (vgl. z.B. EP-A 0 635 500 sowie WO-A 94/14783) und werden zur Bekämpfung von tierischen Schädlingen eingesetzt.

Neu und ebenfalls Gegenstand der Erfindung sind Verbindungen der Formel (Ib) 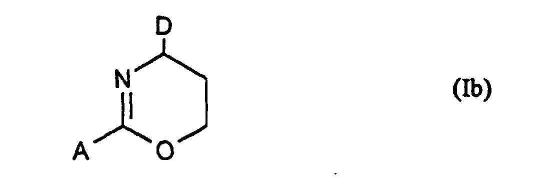 in welcher
- A: die oben angegebene Bedeutung hat und
- D: für jeweils gegebenenfalls substituiertes 2- oder 3-Pyrrolyl oder für durch CHO, Cyano, Carboxyl, Polyalkoxyalkyl oder Alkoxycarbonyl substituiertes Phenyl steht.

Die Verbindungen der Formel (Ib) können - auch in Abhängigkeit von der Art der Substituenten - als geometrische und/oder optische Isomere oder Isomerengemische unterschiedlicher Zusammensetzung vorliegen. Die Erfindung betrifft sowohl die reinen Isomeren als auch die Isomerengemische.

Weiterhin wurde gefunden, daß sich die Verbindungen der Formel (Ib) nach dem oben beschriebenen erfindungsgemäßen Verfahren herstellen lassen.

Schließlich wurde gefunden, daß die neuen Verbindungen der Formel (Ib) sehr gut zur Bekämpfung von tierischen Schädlingen, insbesondere von Arthropoden und Nematoden, geeignet sind.

Überraschenderweise zeigen die erfindungsgemäßen Verbindungen der Formel (Ib) eine erheblich bessere Wirksamkeit gegen tierische Schädlinge als die konstitutionell ähnlichsten bekannten Verbindungen.

Die erfindungsgemäßen Verbindungen sind durch die Formel (Ib) allgemein definiert.

Bevorzugte Substituenten bzw. Bereiche der in den vorstehend und nachfolgend aufgeführten Formeln erwähnten Reste werden im folgenden erläutert.
- A: steht bevorzugt für Phenyl, das einfach bis fünffach, gleich oder verschieden substituiert ist durch Halogen, C₁-C₆-Alkyl, C₁-C₆-Alkoxy, C₁-C₆-Alkylthio, C₁-C₆-Halogenalkyl, C₁-C₆-Halogenalkoxy, C₁-C₆-Halogenalkylthio, Nitro, Cyano oder durch gegebenenfalls einfach bis fünffach, gleich oder verschieden durch Halogen, C₁-C₆-Alkyl, C₁-C₆-Halogenalkyl, C₁-C₆-Alkoxy, C₁-C₆-Halogenalkoxy, C₁-C₆-Alkylthio oder C₁-C₆-Halogenalkylthio substituiertes Phenyl,
für gegebenenfalls einfach bis dreifach, gleich oder verschieden substituiertes Naphthyl, wobei als Substituenten genannt seien Halogen, C₁-C₆-Alkyl, C₁-C₆-Alkoxy und C₁-C₆-Halogenalkoxy,
für gegebenenfalls einfach bis dreifach, gleich oder verschieden substituiertes Pyridyl, wobei als Substituenten genannt seien Halogen, C₁-C₆-Alkyl, C₁-C₆-Alkoxy, C₁-C₆-Halogenalkyl, C₁-C₆-Halogenalkoxy und Cyano,
für gegebenenfalls einfach bis dreifach, gleich oder verschieden substituiertes Thienyl, wobei als Substituenten genannt seien Halogen und C₁-C₆-Alkyl,
oder für gegebenenfalls einfach bis dreifach, gleich oder verschieden substituiertes Pyrazolyl, wobei als Substituenten genannt seien Halogen oder C₁-C₃-Alkyl.
- D: steht bevorzugt für jeweils gegebenenfalls einfach oder zweifach, gleich oder verschieden durch Halogen oder C₁-C₄-Alkyl substituiertes 2-Pyrrolyl oder 3-Pyrrolyl,
oder für durch CHO, Cyano, Carboxyl, Poly-C₁-C₈-alkoxy-C₁-C₈-alkyl oder C₁-C₈-Alkoxycarbonyl substituiertes Phenyl.
- A: steht besonders bevorzugt für Phenyl, das einfach bis vierfach, gleich oder verschieden substituiert ist durch
Fluor, Chlor, Brom, C₁-C₃-Alkyl, C₁-C₃-Alkoxy, C₁-C₃-Alkylthio, einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃, SCHF₂, Nitro, Cyano, oder durch gegebenenfalls einfach bis vierfach, gleich oder verschieden durch Fluor, Chlor, Brom, C₁-C₄-Alkyl, C₁-C₄-Alkoxy, C₁-C₄-Alkylthio, einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃ oder SCHF₂ substituiertes Phenyl,
für gegebenenfalls einfach bis dreifach, gleich oder verschieden substituiertes Naphthyl, wobei als Substituenten genannt seien
Fluor, Chlor, C₁-C₆-Alkyl, C₁-C₆-Alkoxy und einfach bis dreifach, gleich oder verschieden durch Fluor oder Chlor substituiertes C₁-C₆-Alkoxy,
für gegebenenfalls einfach oder zweifach, gleich oder verschieden substituiertes Pyridyl, wobei als Substituenten genannt seien
Fluor, Chlor, C₁-C₃-Alkyl, C₁-C₃-Alkoxy, CF₃, OCF₃ und Cyano,
für gegebenenfalls einfach oder zweifach, gleich oder verschieden substituiertes Thienyl, wobei als Substituenten genannt seien
Chlor, Brom, Methyl, Ethyl oder
für gegebenenfalls einfach bis dreifach, gleich oder verschieden substituiertes Pyrazolyl, wobei als Substituenten genannt seien
Fluor, Chlor, Brom und C₁-C₃-Alkyl.
- D: steht besonders bevorzugt für jeweils gegebenenfalls einfach oder zweifach, gleich oder verschieden durch Fluor, Chlor oder C₁-C₃-Alkyl substituiertes 2-Pyrrolyl oder 3-Pyrrolyl,
oder für durch CHO, Cyano, Carboxyl, Poly-C₁-C₈-alkoxy-C₁-C₆-alkyl oder C₁-C₈-Alkoxycarbonyl substituiertes Phenyl.
- A: steht ganz besonders bevorzugt für Phenyl, das einfach bis dreifach, gleich oder verschieden substituiert ist durch
Fluor, Chlor, Brom, C₁-C₃-Alkyl, C₁-C₃-Alkoxy, C₁-C₃-Alkylthio, einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃, SCHF₂, Nitro, Cyano, oder durch gegebenenfalls einfach bis vierfach, gleich oder verschieden durch Fluor, Chlor, Brom, C₁-C₃-Alkyl, C₁-C₃-Alkoxy, C₁-C₃-Alkylthio, einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃ oder SCHF₂ substituiertes Phenyl.
- D: steht ganz besonders bevorzugt für jeweils gegebenenfalls einfach oder zweifach, gleich oder verschieden durch Fluor, Chlor, CH₃ oder C₂H₅ substituiertes 2-Pyrrolyl oder 3-Pyrrolyl,
oder für durch CHO, Cyano, Carboxyl, Di-C₁-C₆-alkoxy-C₁-C₄-alkyl oder C₁-C₆-Alkoxycarbonyl substituiertes Phenyl.
- A: steht besonders hervorgehoben für einfach oder zweifach, gleich oder verschieden durch Fluor oder Chlor substituiertes Phenyl.
- D: steht besonders hervorgehoben für jeweils gegebenenfalls einfach oder zweifach, gleich oder verschieden durch Fluor, Chlor, Methyl oder Ethyl substituiertes 2- oder 3-Pyrrolyl,
oder für durch CHO, Cyano, Carboxyl, Di-C₁-C₆-alkoxy-C₁-C₄-alkyl oder C₁-C₆-Alkoxycarbonyl substituiertes Phenyl.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) gegen die Raupen der Kohlschabe (Plutella maculipennis).

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

### Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin, Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Primiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

### Herbizide:

beispielsweise Anilide, wie z.B. Diflufenican und Propanil, Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuronmethyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die Herstellung und die Verwendung der erfindungsgemäßen Wirkstoffe geht aus den nachfolgenden Beispielen hervor.

### Herstellungsbeispiele

### Beispiel 1

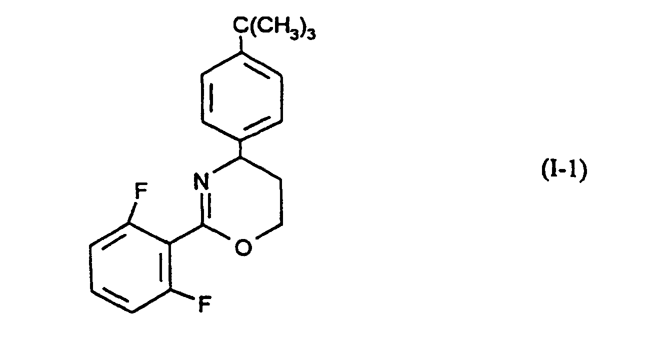

Eine Lösung aus 3 g (7,7 mMol) N-[Morpholin-4-yl-(4-tert.-butylphenyl)-methyl]-2,6-difluorbenzamid (vgl. Bsp. IIa-1) in 20 ml wasserfreiem Dichlormethan und 25 ml (25 mMol) Titan-IV-chlorid (1-molare Lösung in Dichlormethan) wird bei Raumtemperatur im Autoklaven mit trockenem Chlorwasserstoff-Gas gesättigt. Danach wird bei ca. 0°C etwa 20 bis 30 Minuten lang Ethylen eingeleitet und anschließend 24 Stunden bei 70°C und ca. 14 bar Eigendruck gerührt. Das Reaktionsgemisch wird anschließend mit ca. 100 ml Eiswasser verrührt, bei 0°C mit Natronlauge alkalisch gestellt und mehrmals mit Dichlormethan extrahiert. Die vereinigten organischen Phasen werden über Magnesiumsulfat getrocknet und eingeengt. Der ölige Rückstand wird chromatographisch gereinigt.

Man erhält 0,83 g (32,5 % der Theorie) 4-(4-tert.-Butylphenyl)-2-(2,6-difluorphenyl)-5,6-dihydro-4H-1,3-oxazin.
¹H-NMR (ppm in CDCl₃): 7.42-6.90 (m, 7H); 4.78 (m, 1H); 4.41-4.30 (m, 2H, 1H); 1.31 (s, 9H).

### Beispiel 2

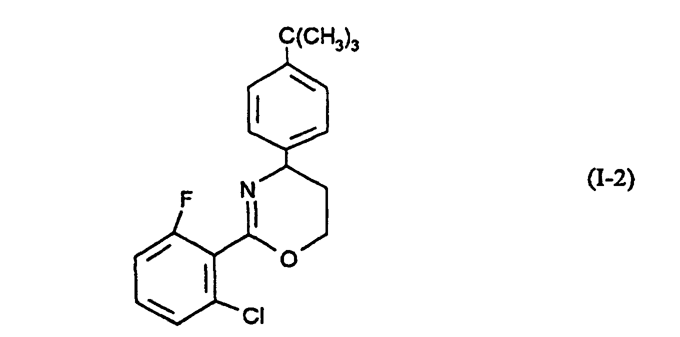

(ohne Zwischenisolierung der Verbindung (II))

3,5 g (20 mMol) 2-Chlor-6-fluorbenzoesäureamid werden in 40 ml Dichlormethan mit 3,4 g (21 mMol) 4-tert.-Butylbenzaldehyd und 2,1 g (24 mMol) Morpholin 18 Stunden bei 50°C gerührt.

Nach dem Abkühlen wird das Reaktionsgemisch mit 45 ml Titan-IV-chlorid (1-molare Lösung in Dichlormethan) versetzt und bei Raumtemperatur im Autoklaven mit trockenem Chlorwasserstoffgas gesättigt. Danach wird bei ca. 0°C etwa 20 bis 30 Minuten lang Ethylen eingeleitet und anschließend 24 Stunden bie 70°C und ca. 14 bar Eigendruck gerührt. Das Reaktionsgemisch wird mit ca. 150 ml Eiswasser verrührt, bei 0°C mit Natronlauge alkalisch gestellt und mehrmals mit Dichlormethan extrahiert. Die vereinigten organischen Phasen werden über Magnesiumsulfat getrocknet und eingeengt.

Man erhält 1,38 g (30 % der Theorie) 4-(4-tert.-Butylphenyl)-2-(2-chlor-6-fluorphenyl)-5,6-dihydro-4H-1,3-oxazin mit einem logP-Wert von 4.71.

Gemäß den Beispielen 1 und 2 sowie entsprechend den allgemeinen Verfahrensangaben können die folgenden Verbindungen der allgemeinen Formel (I) erhalten werden:

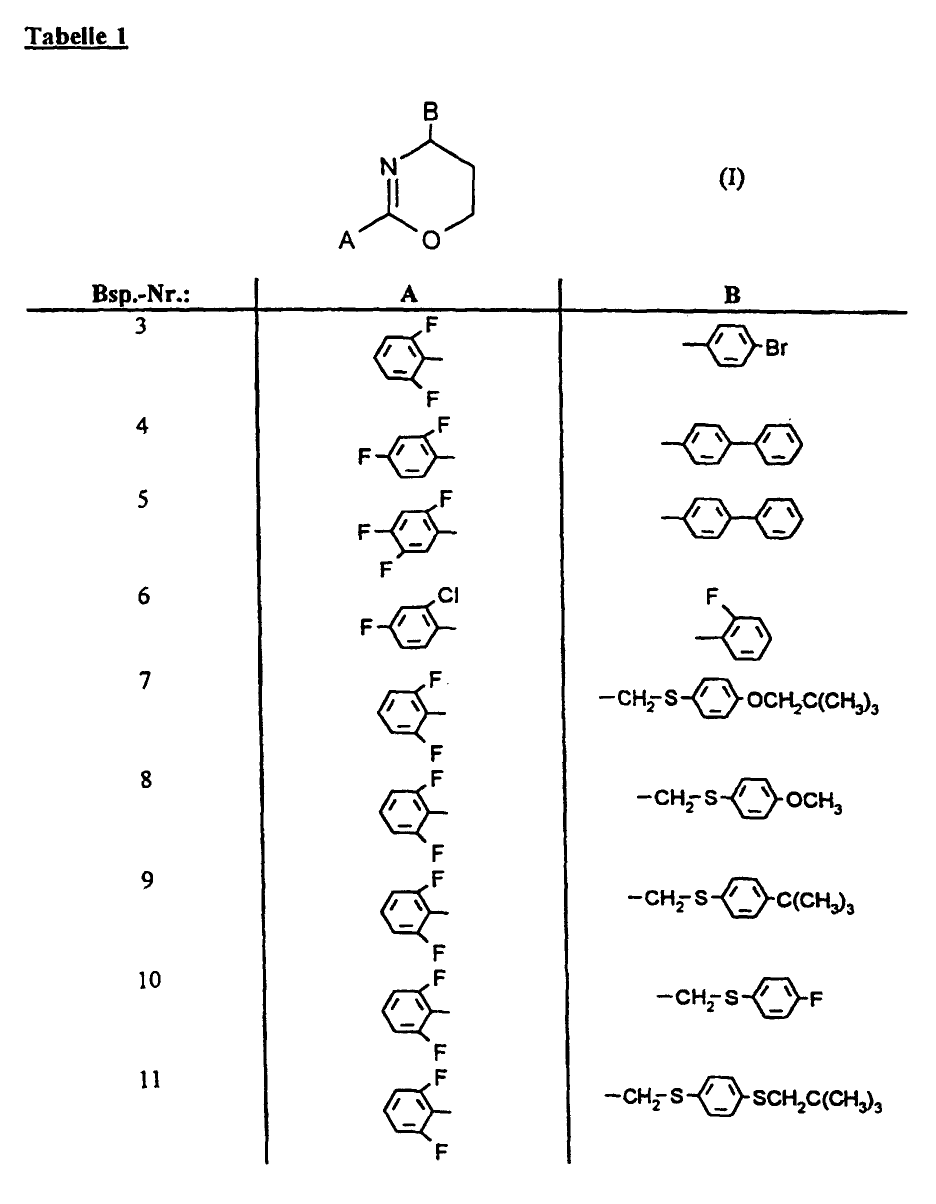

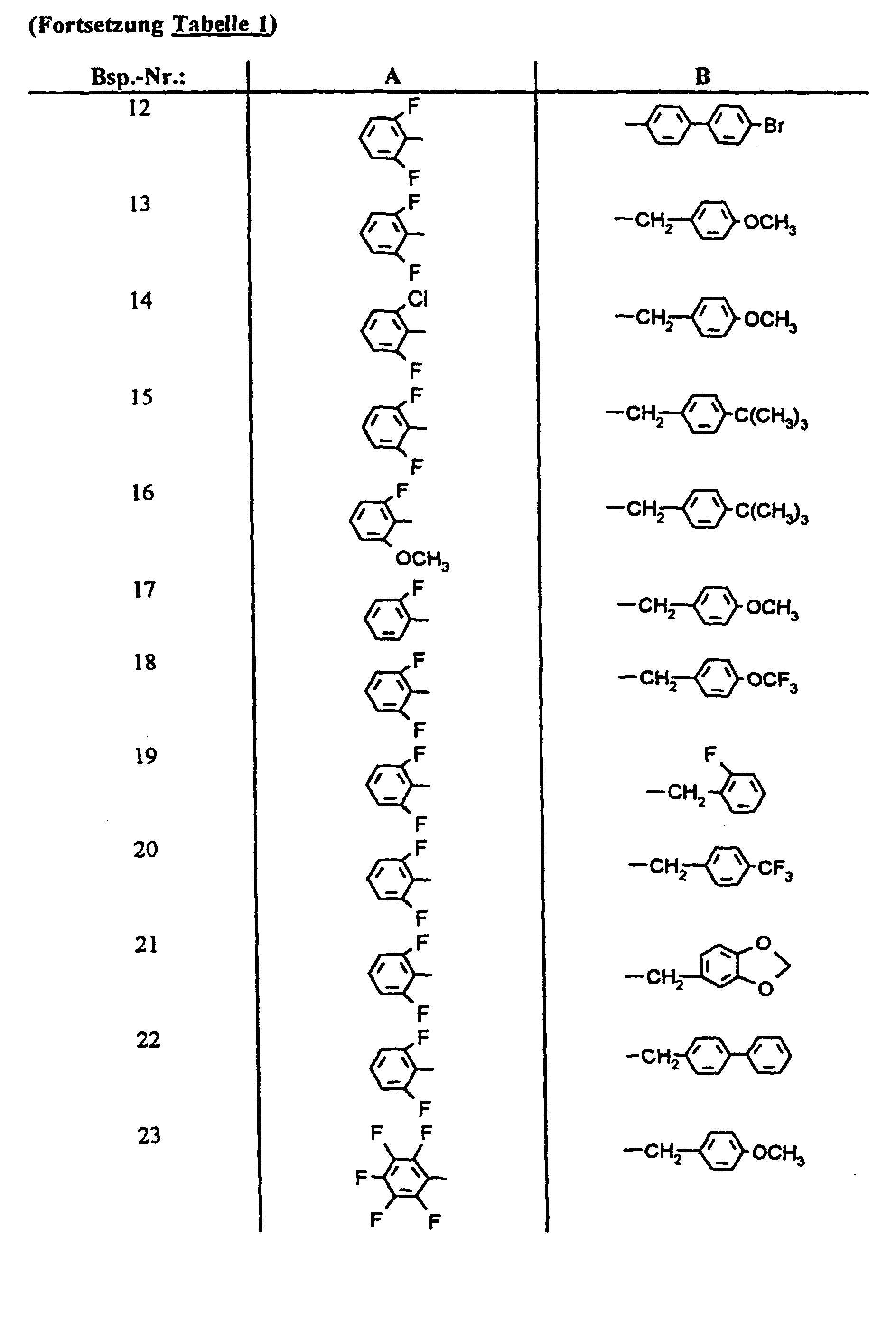

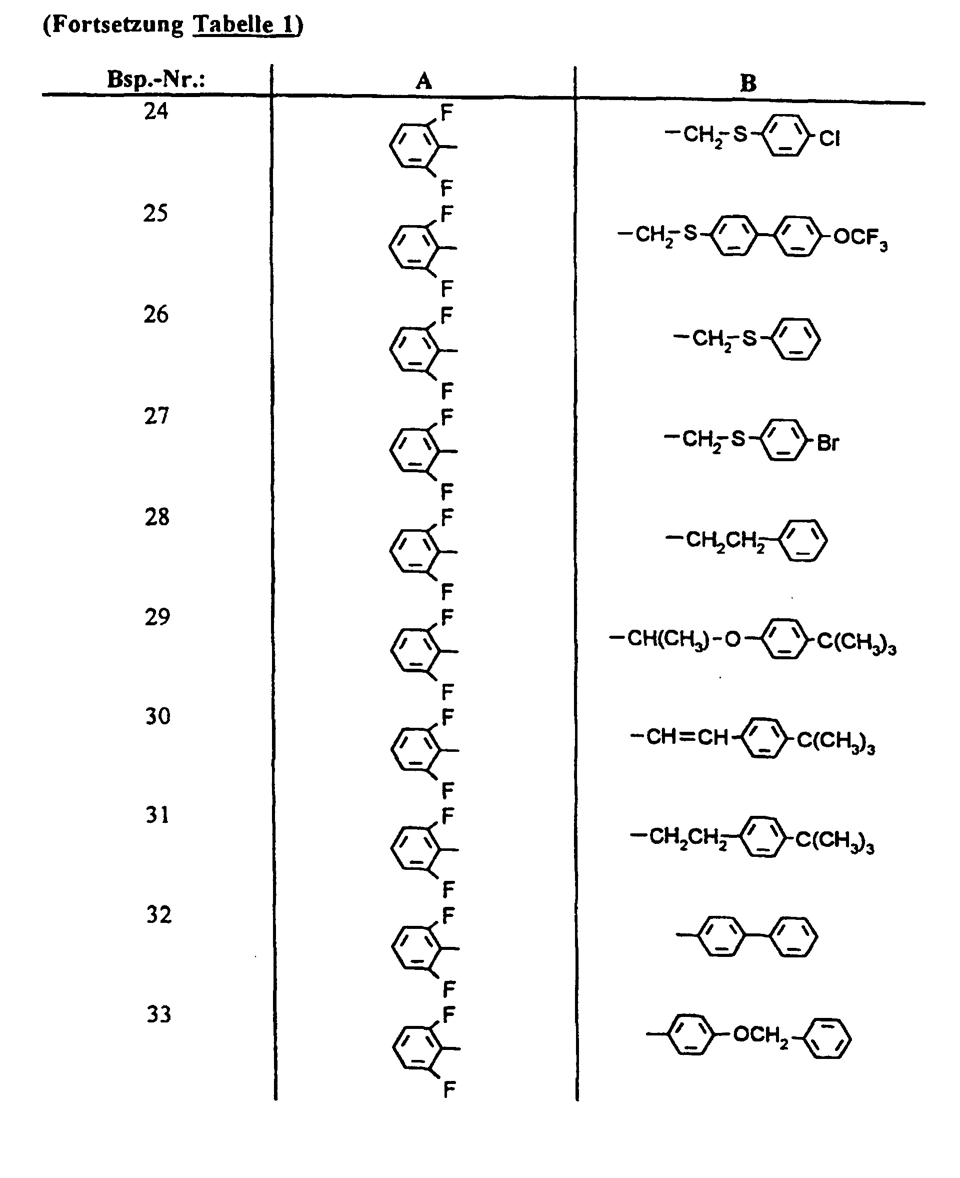

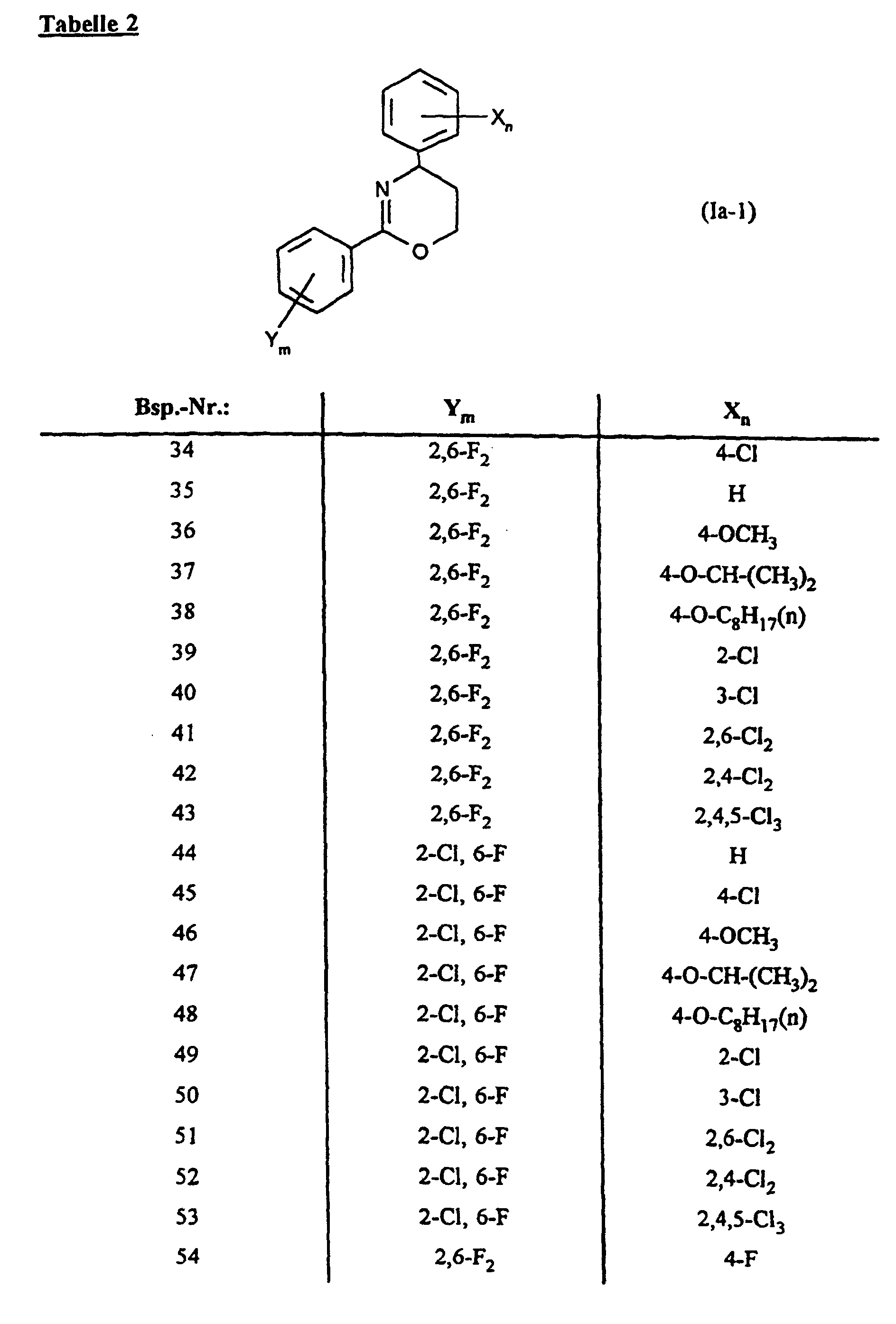

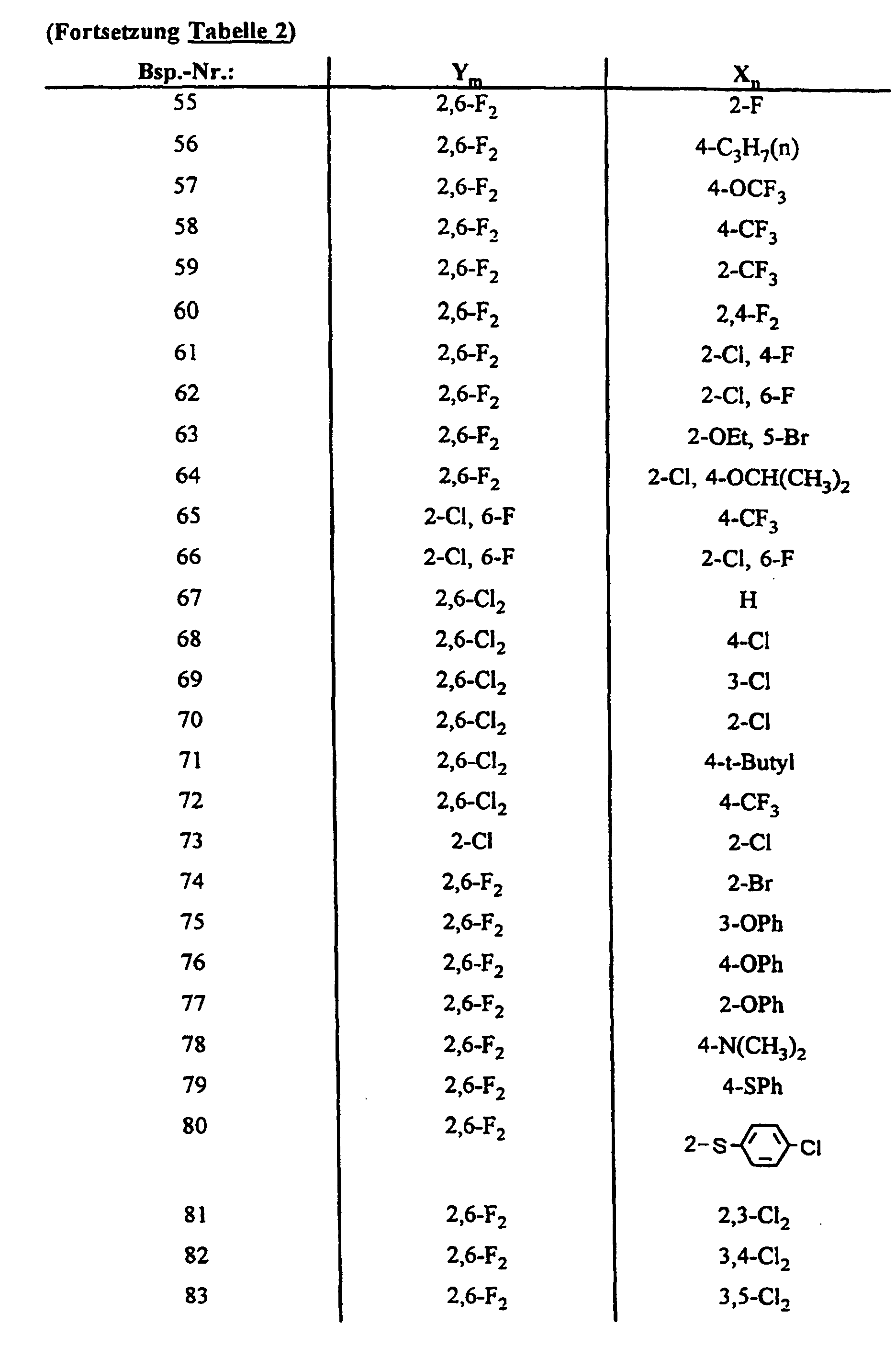

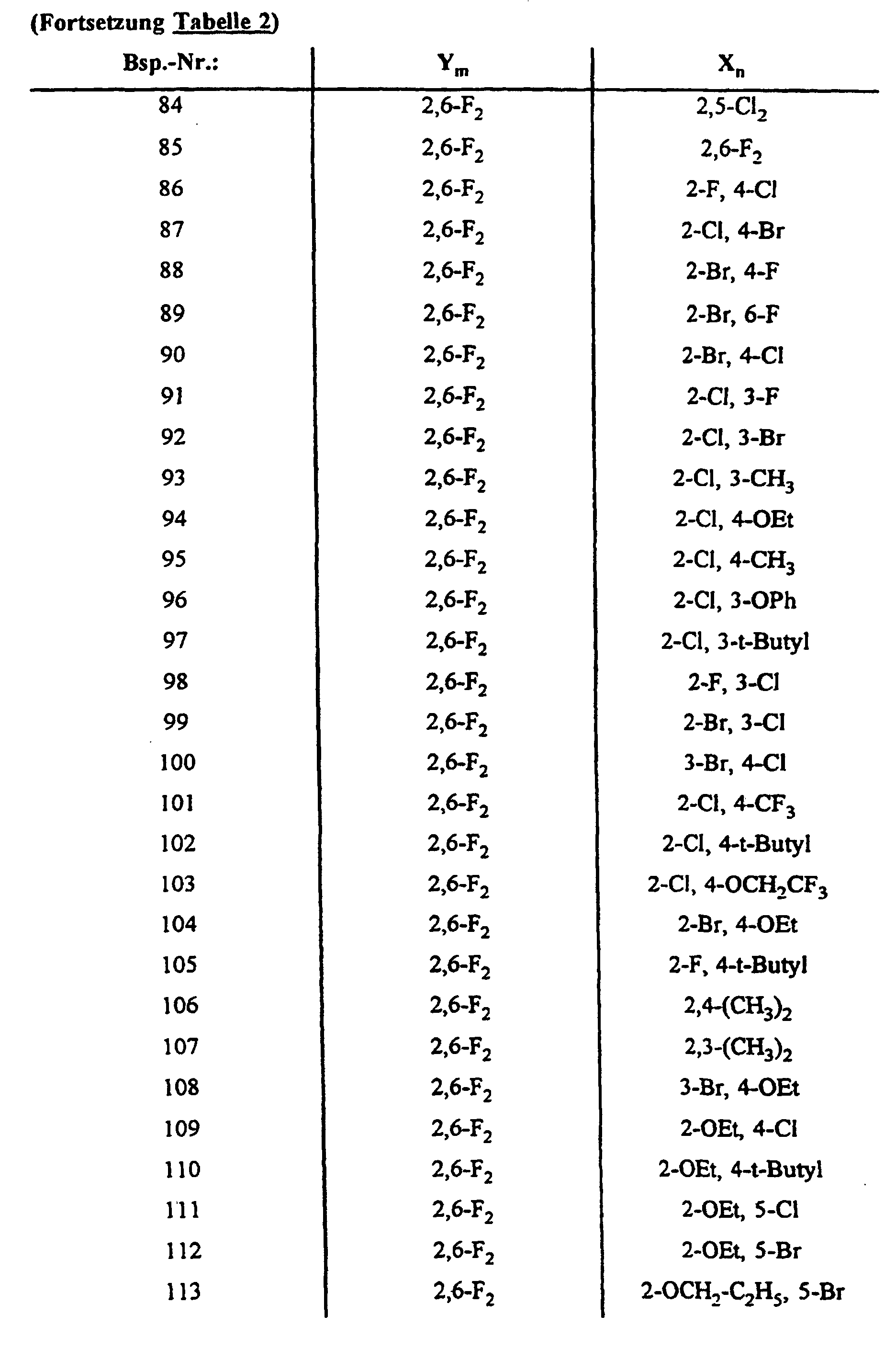

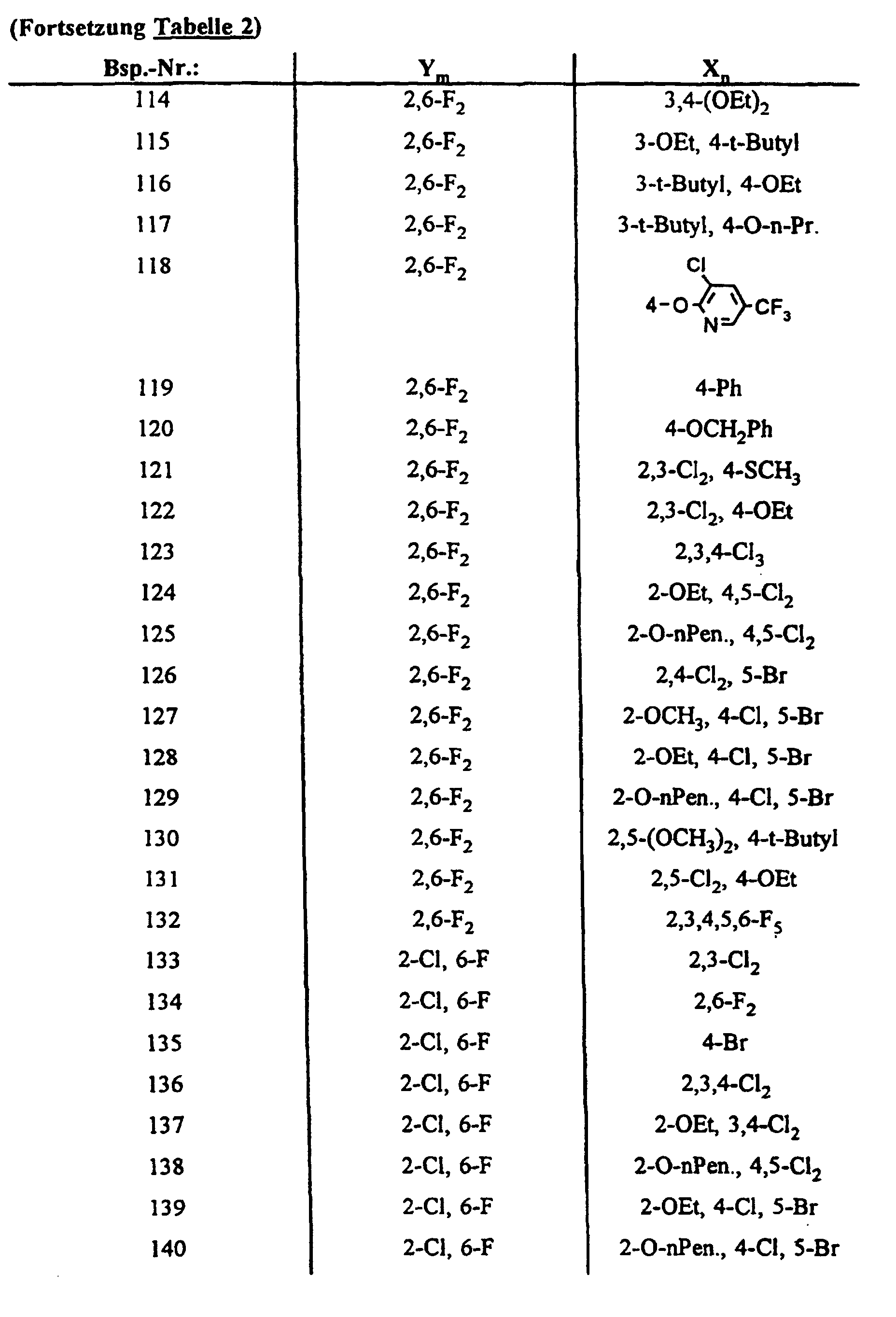

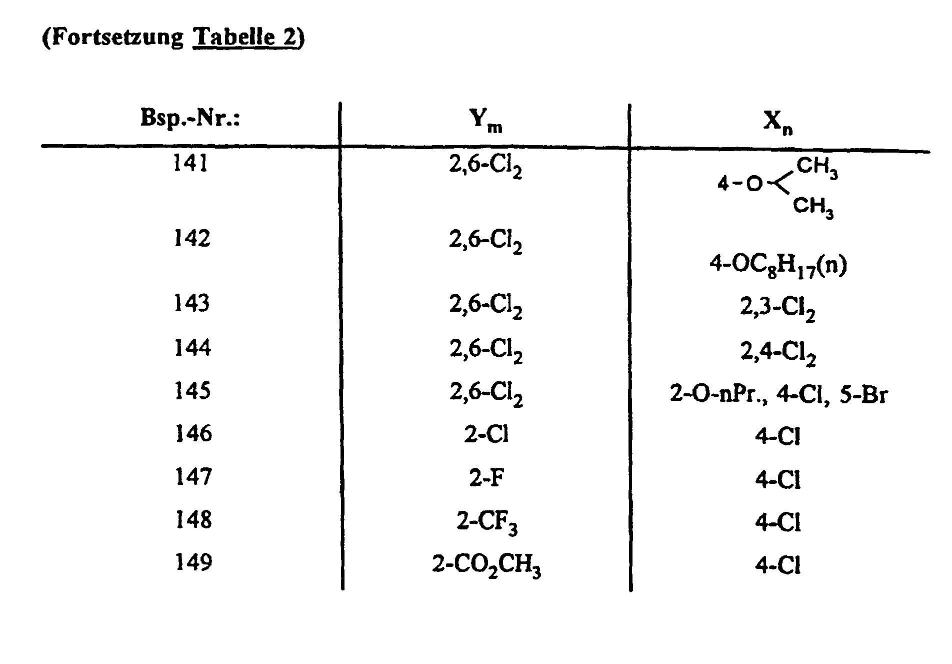

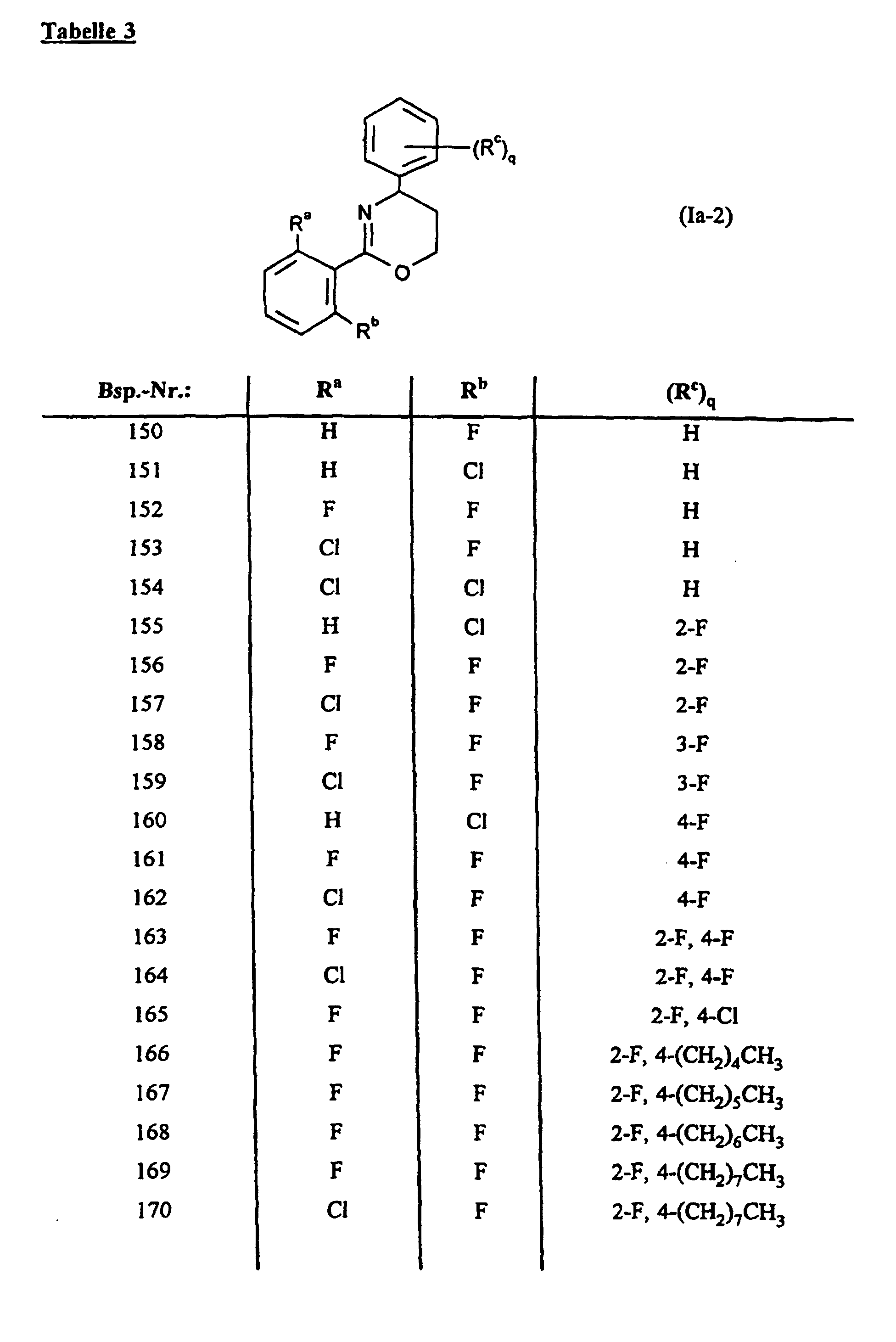

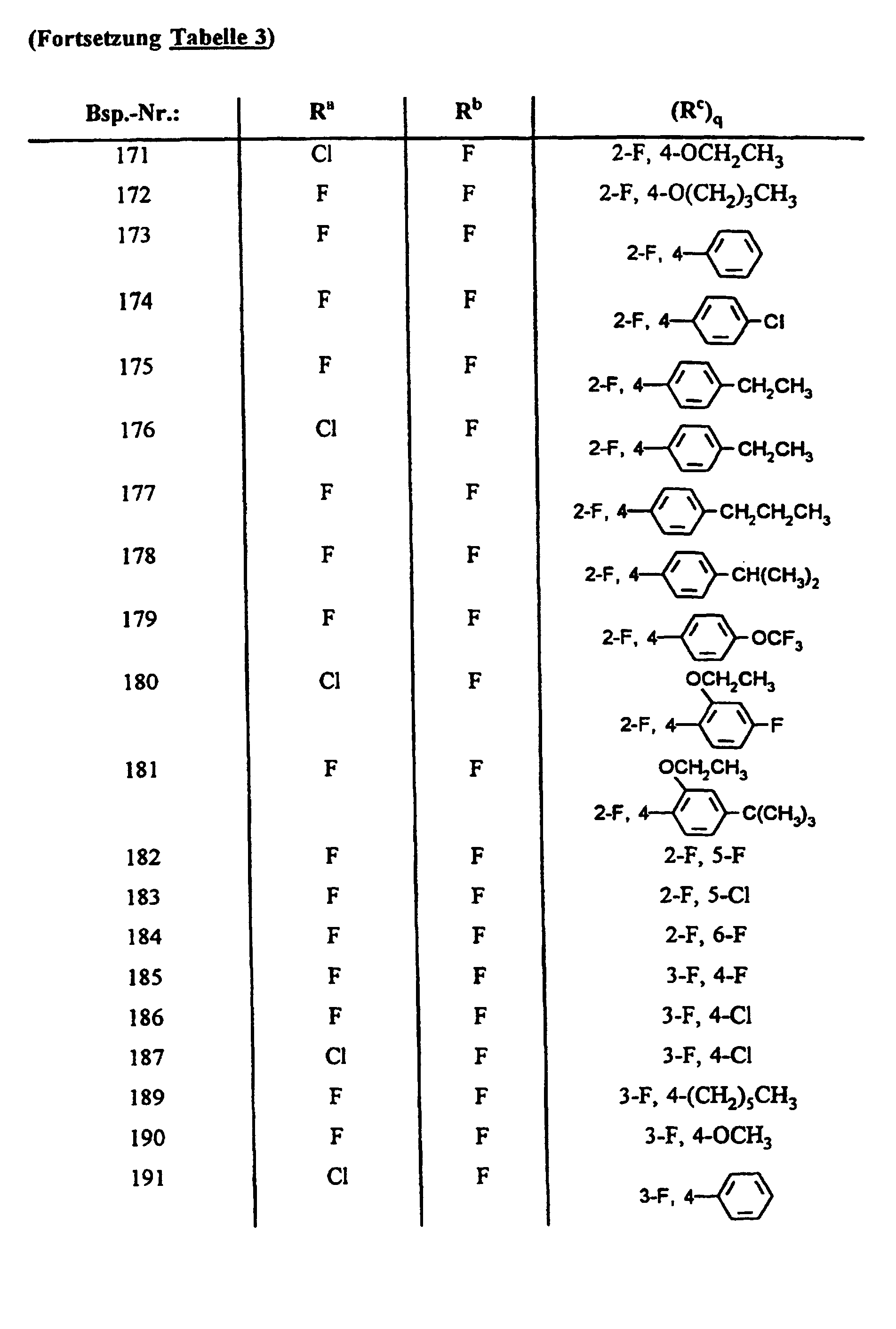

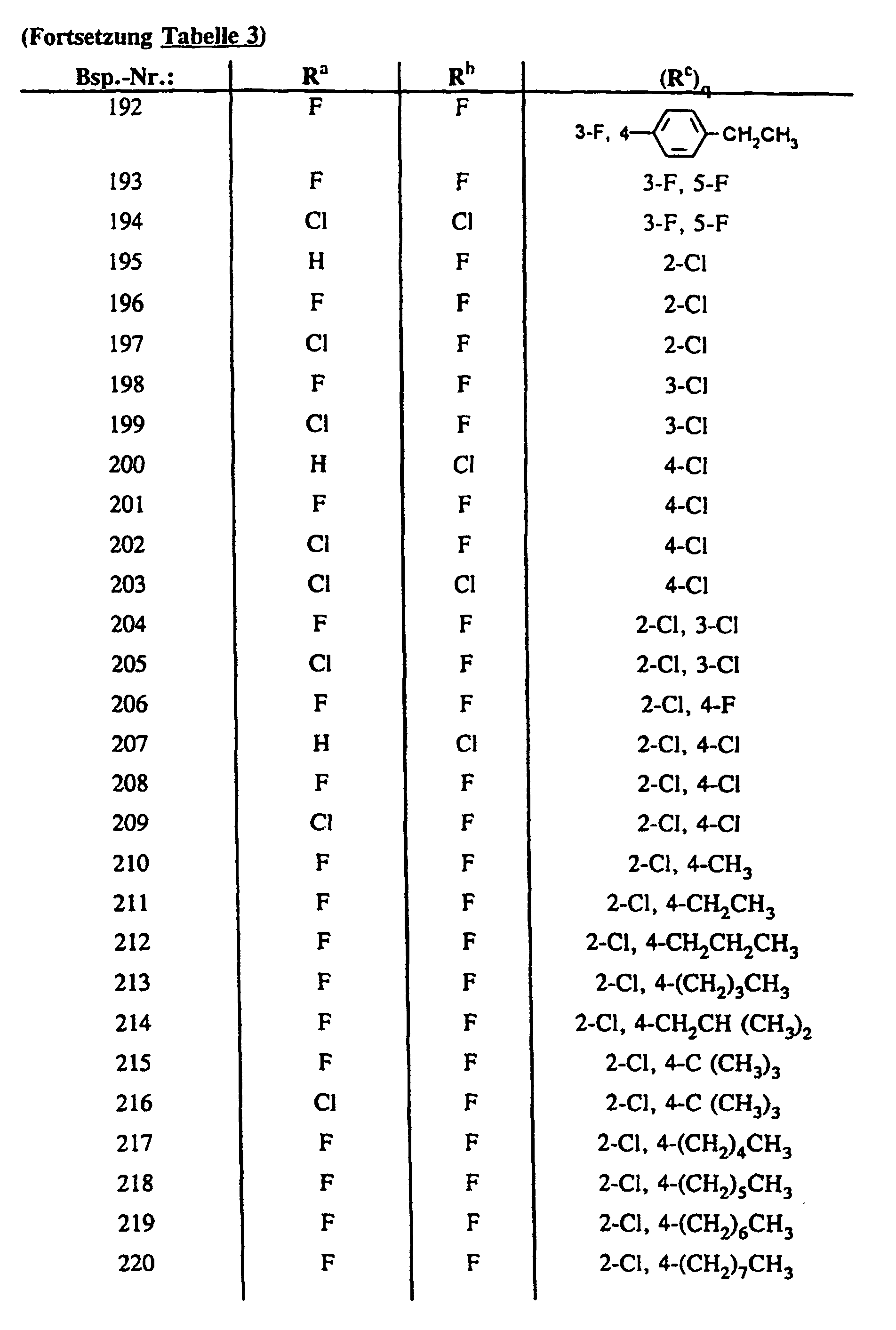

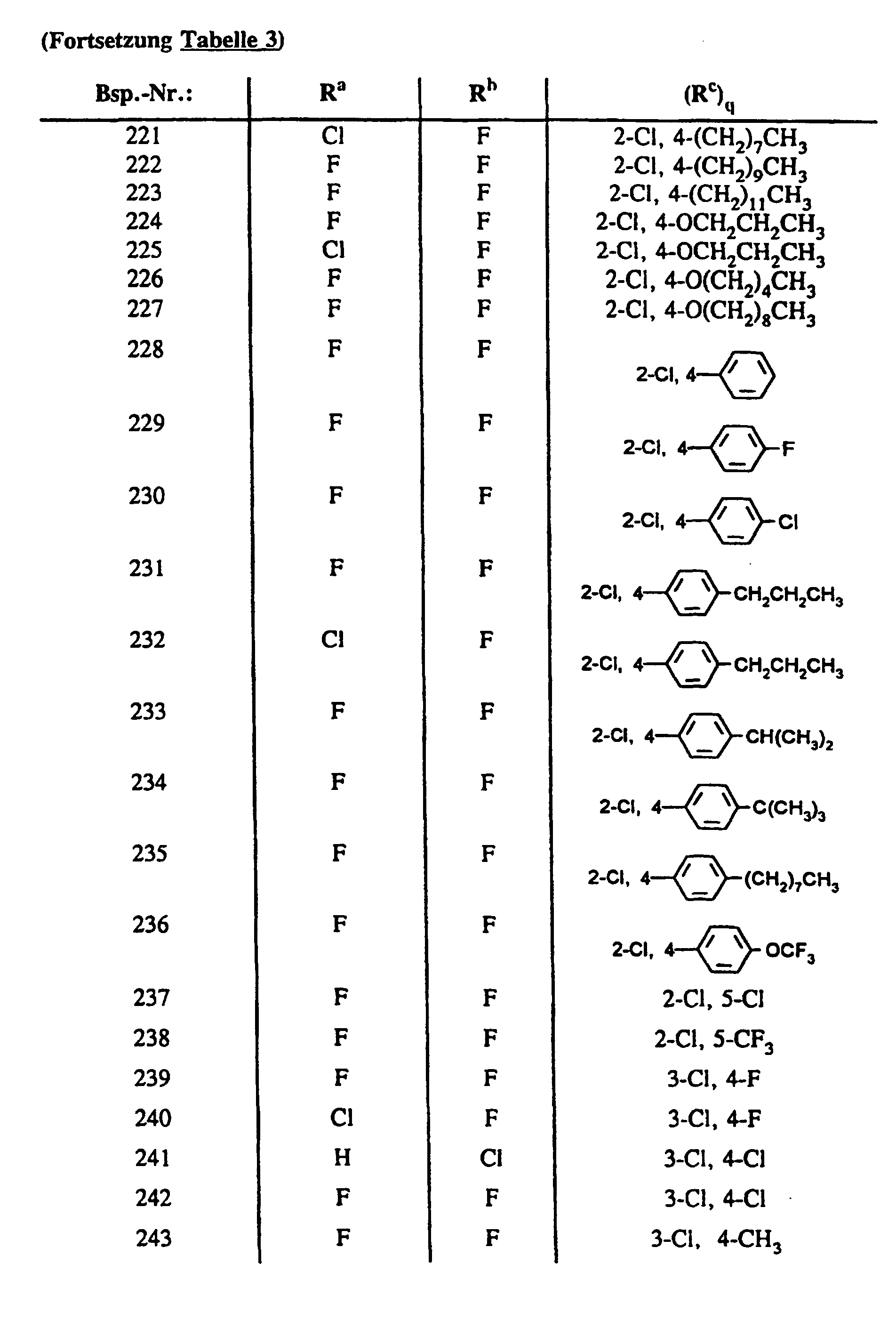

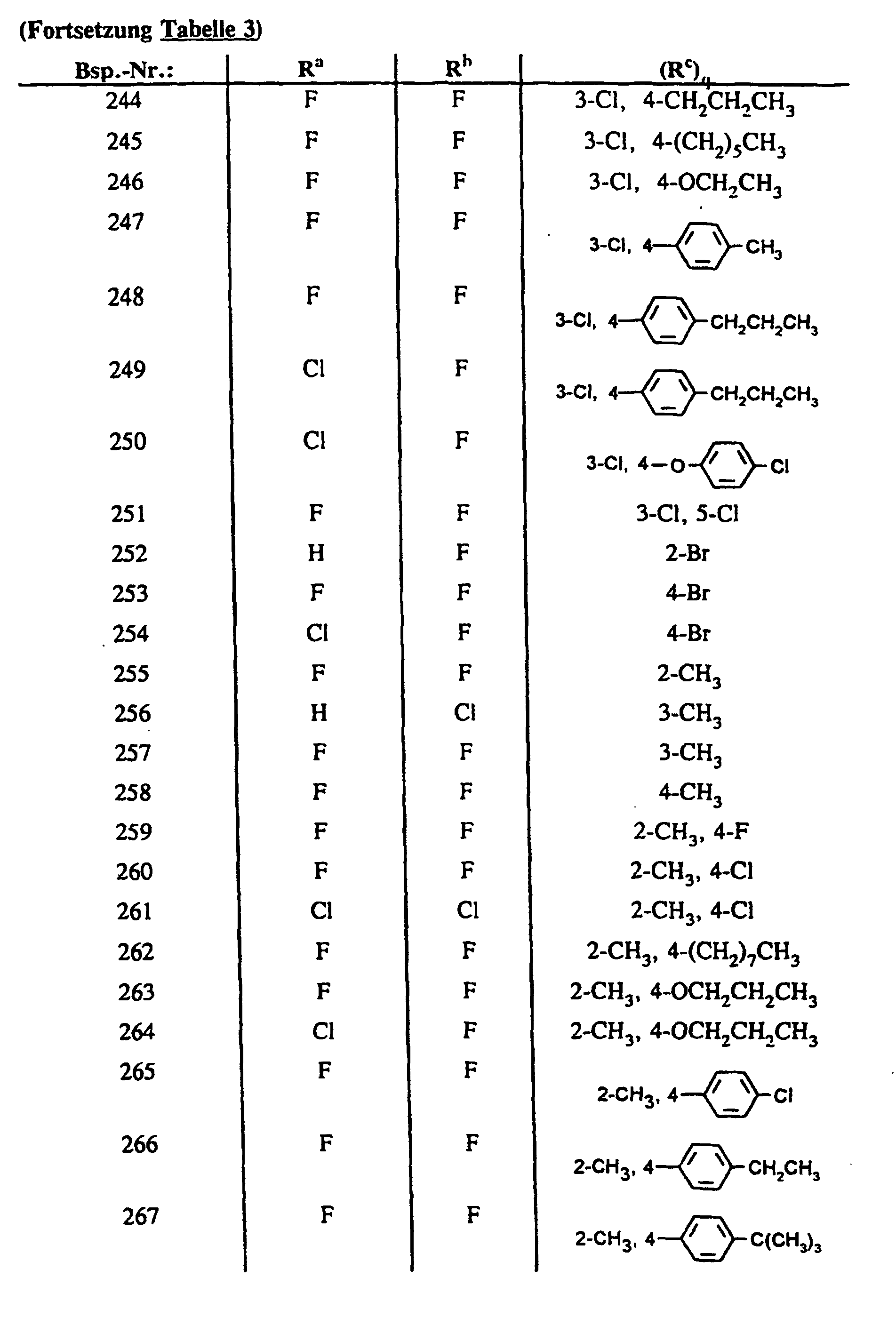

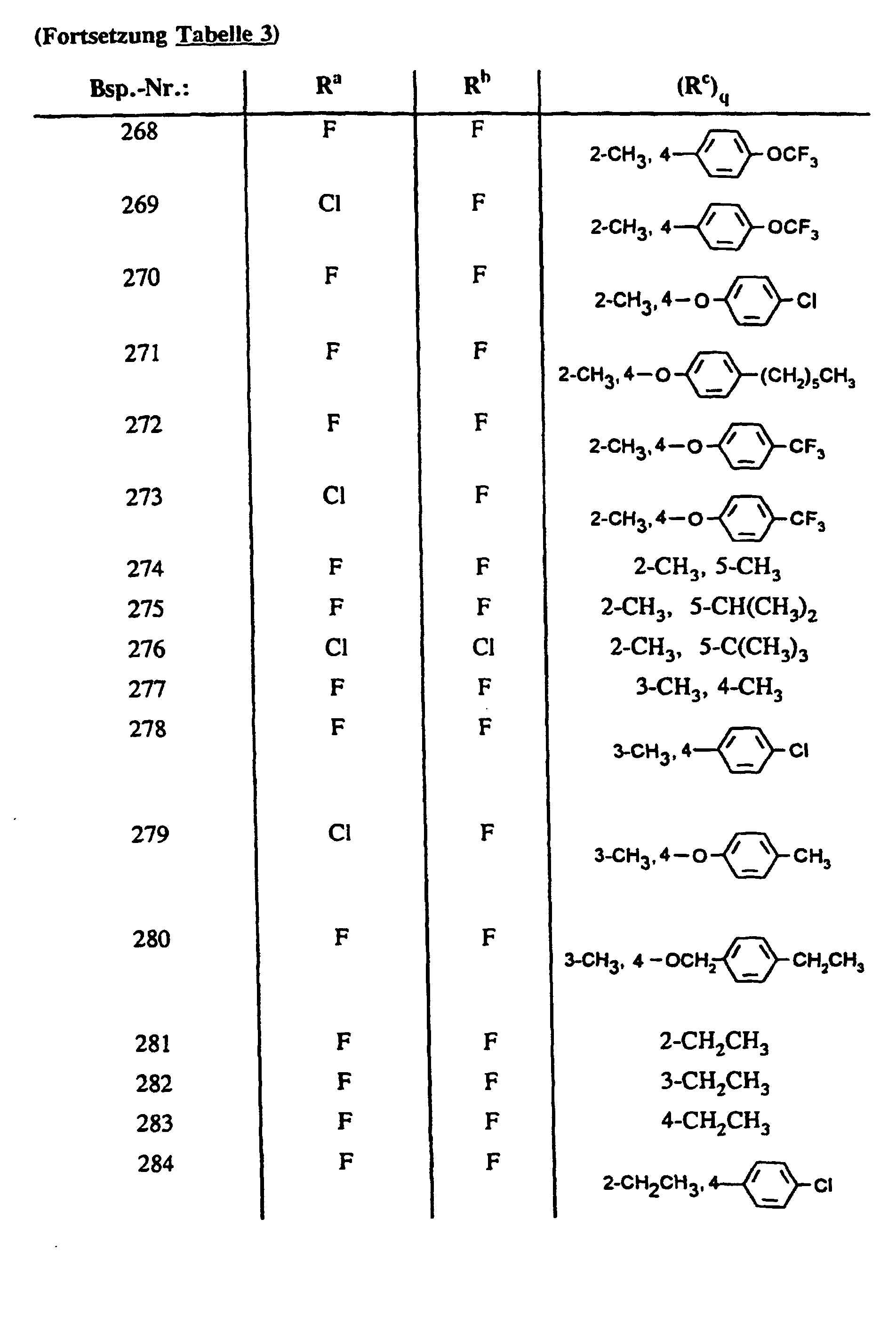

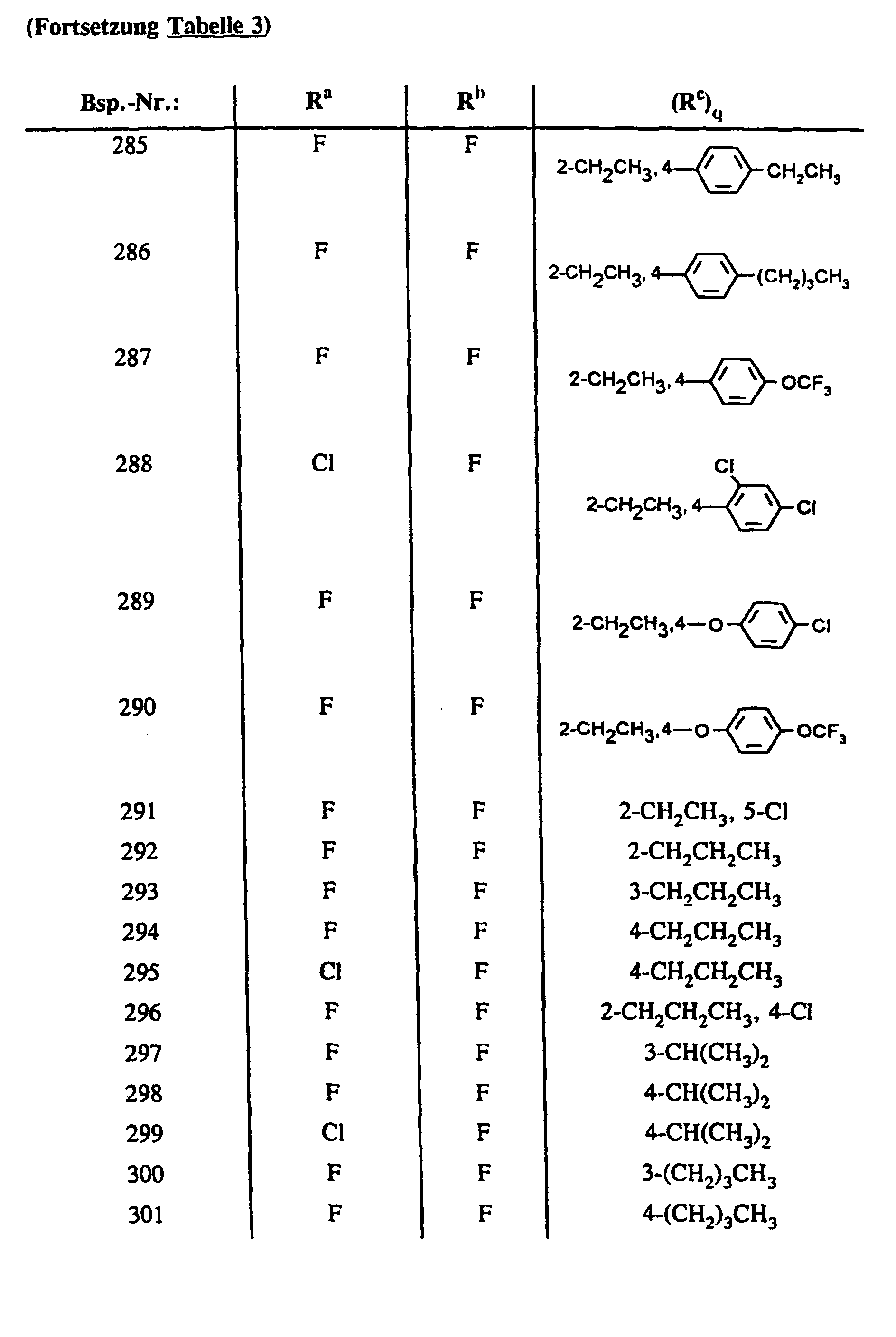

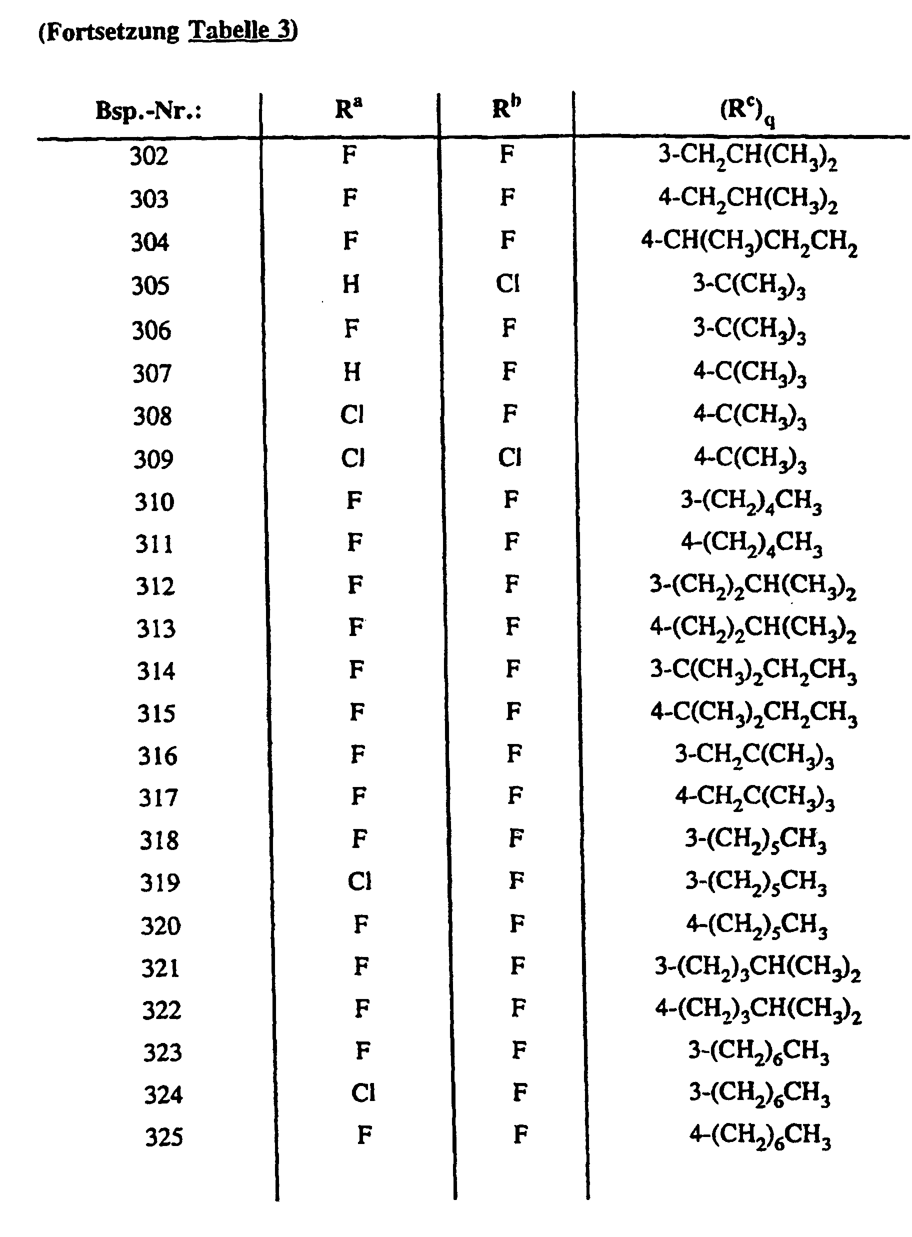

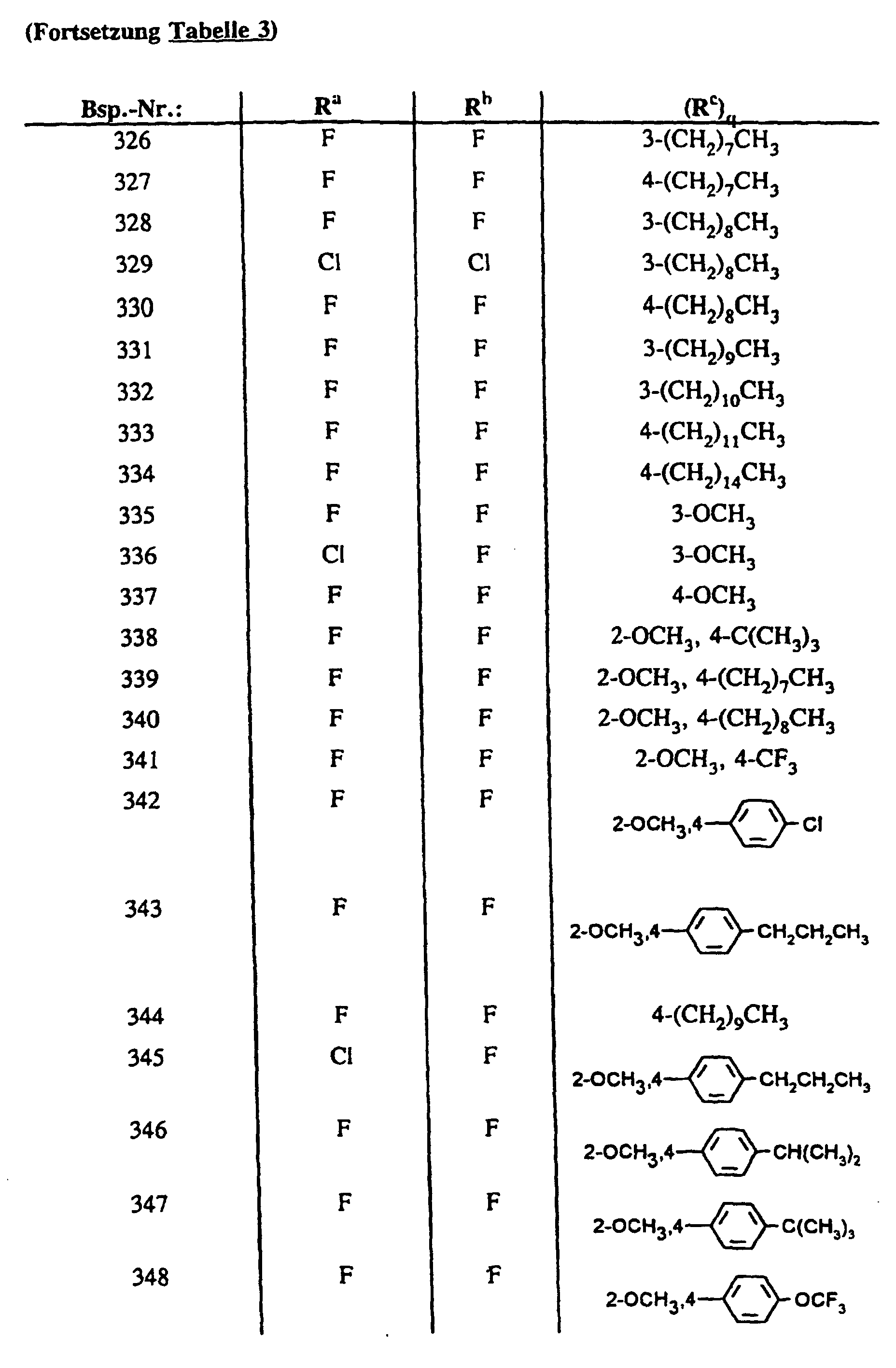

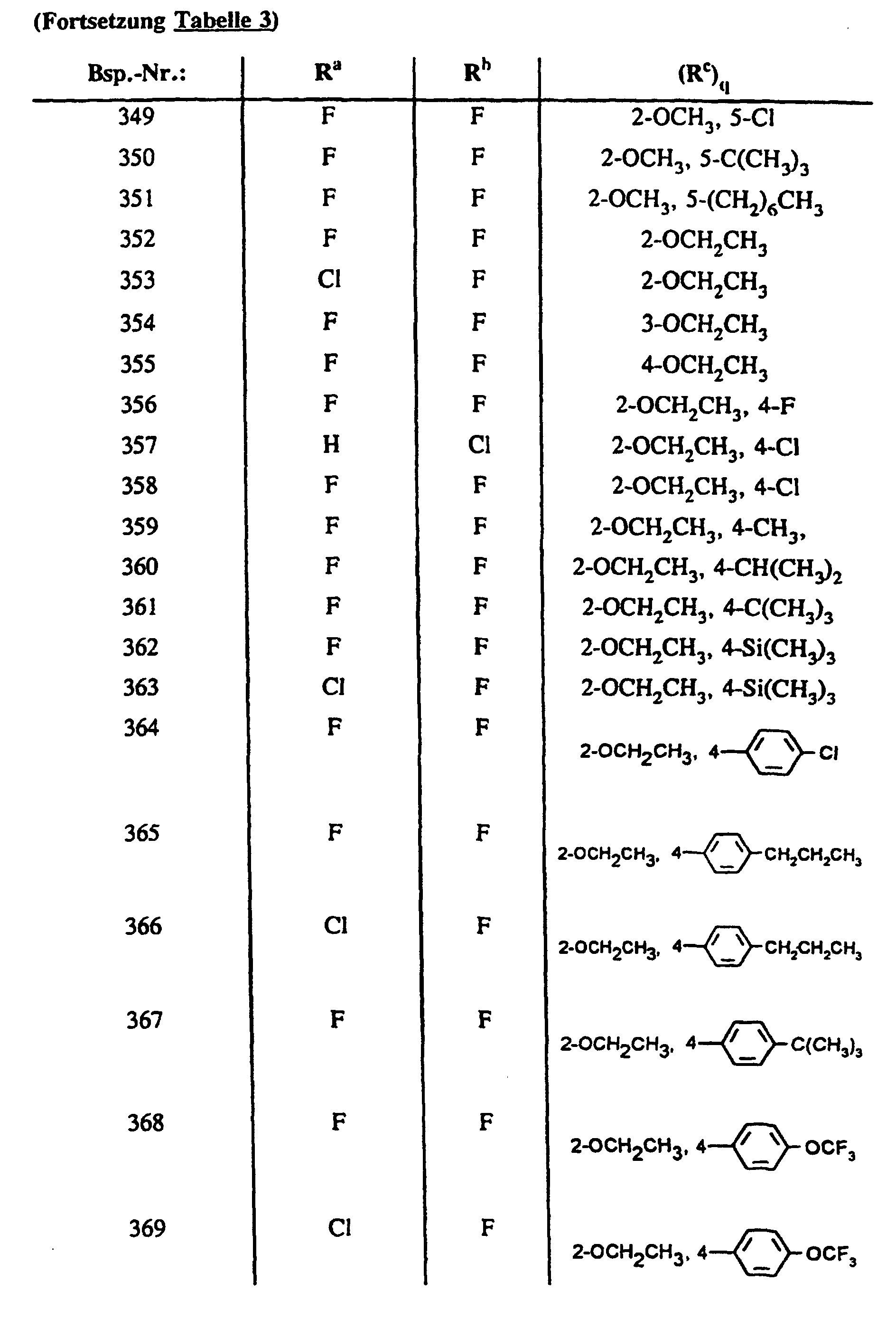

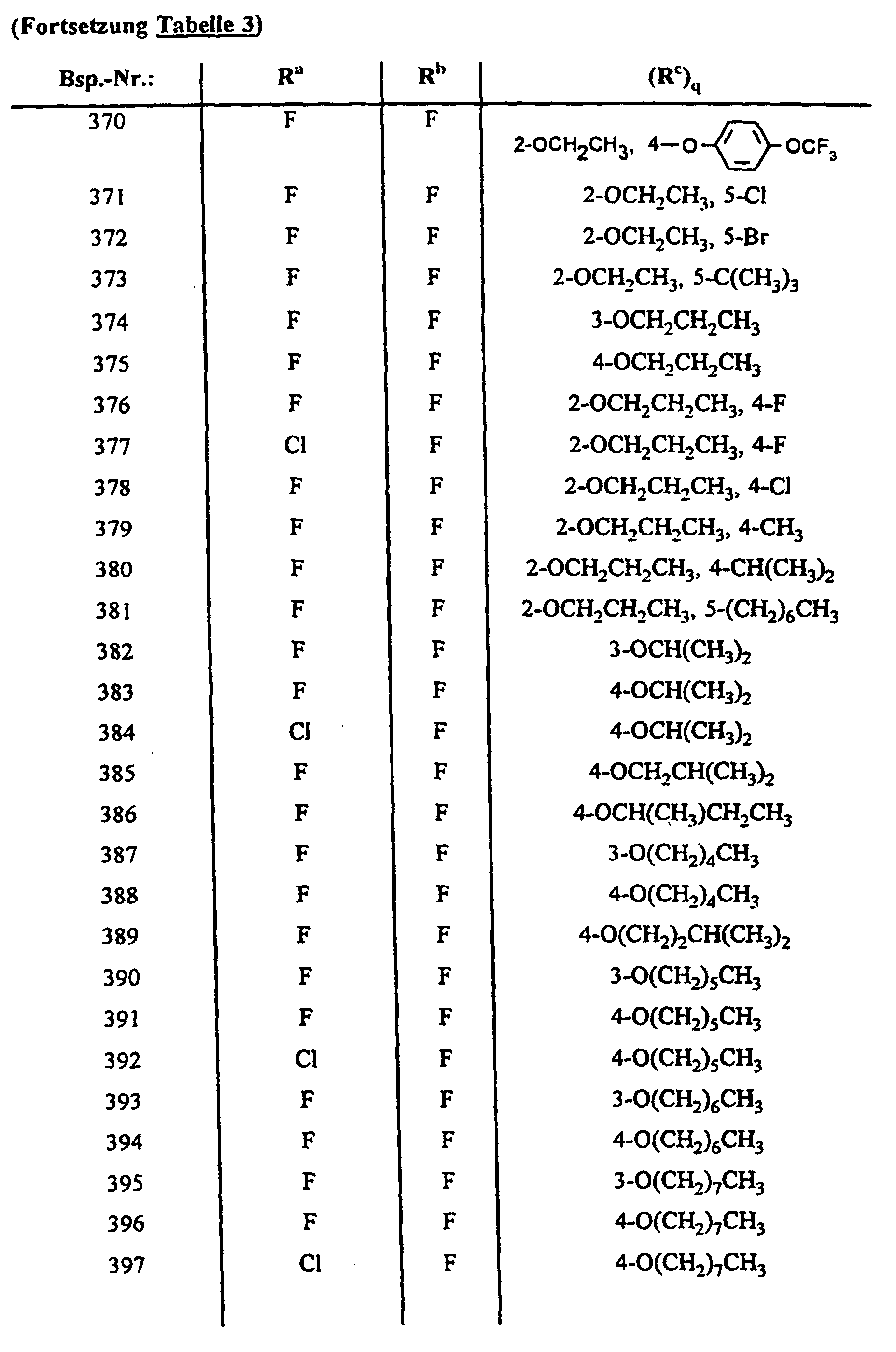

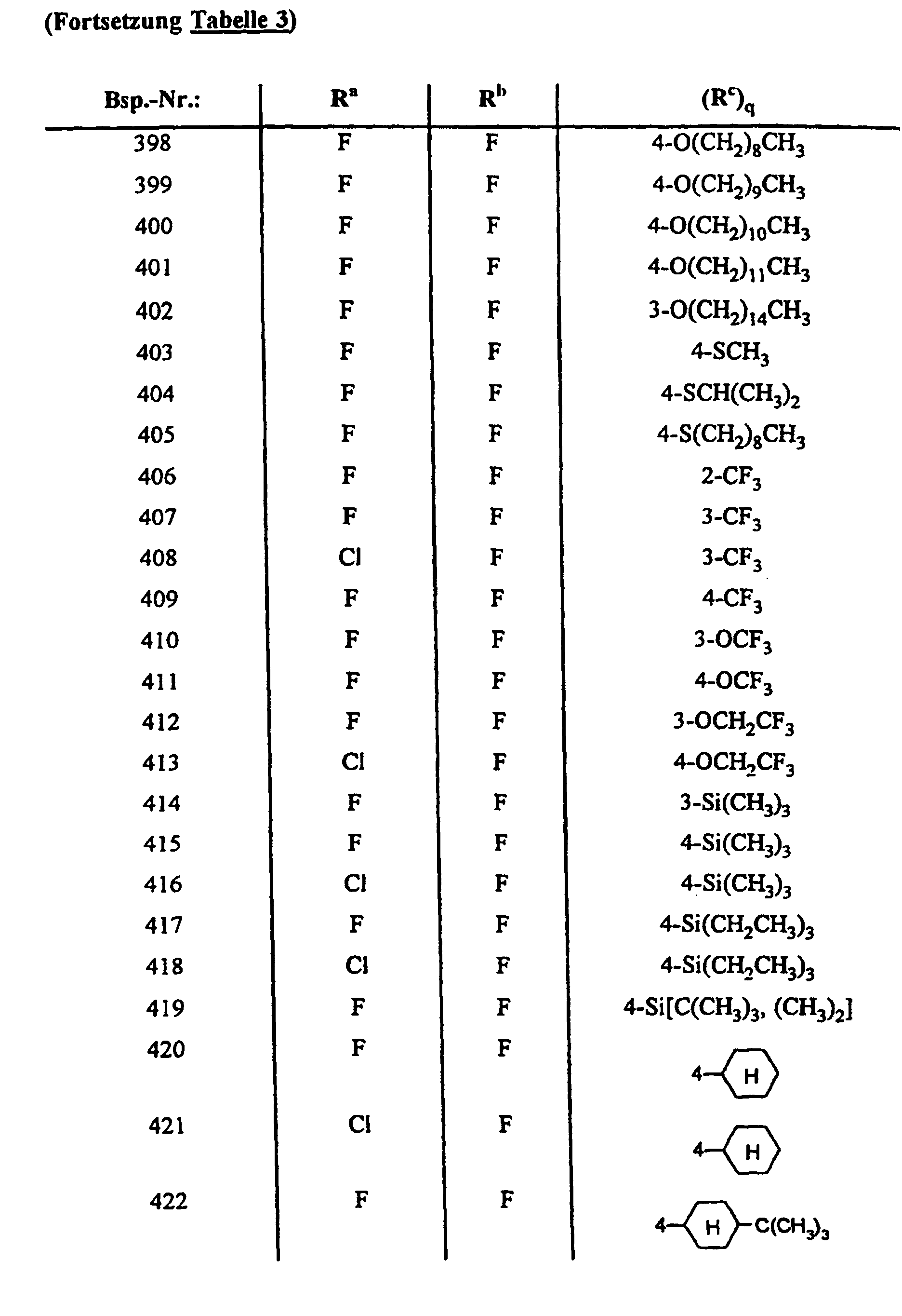

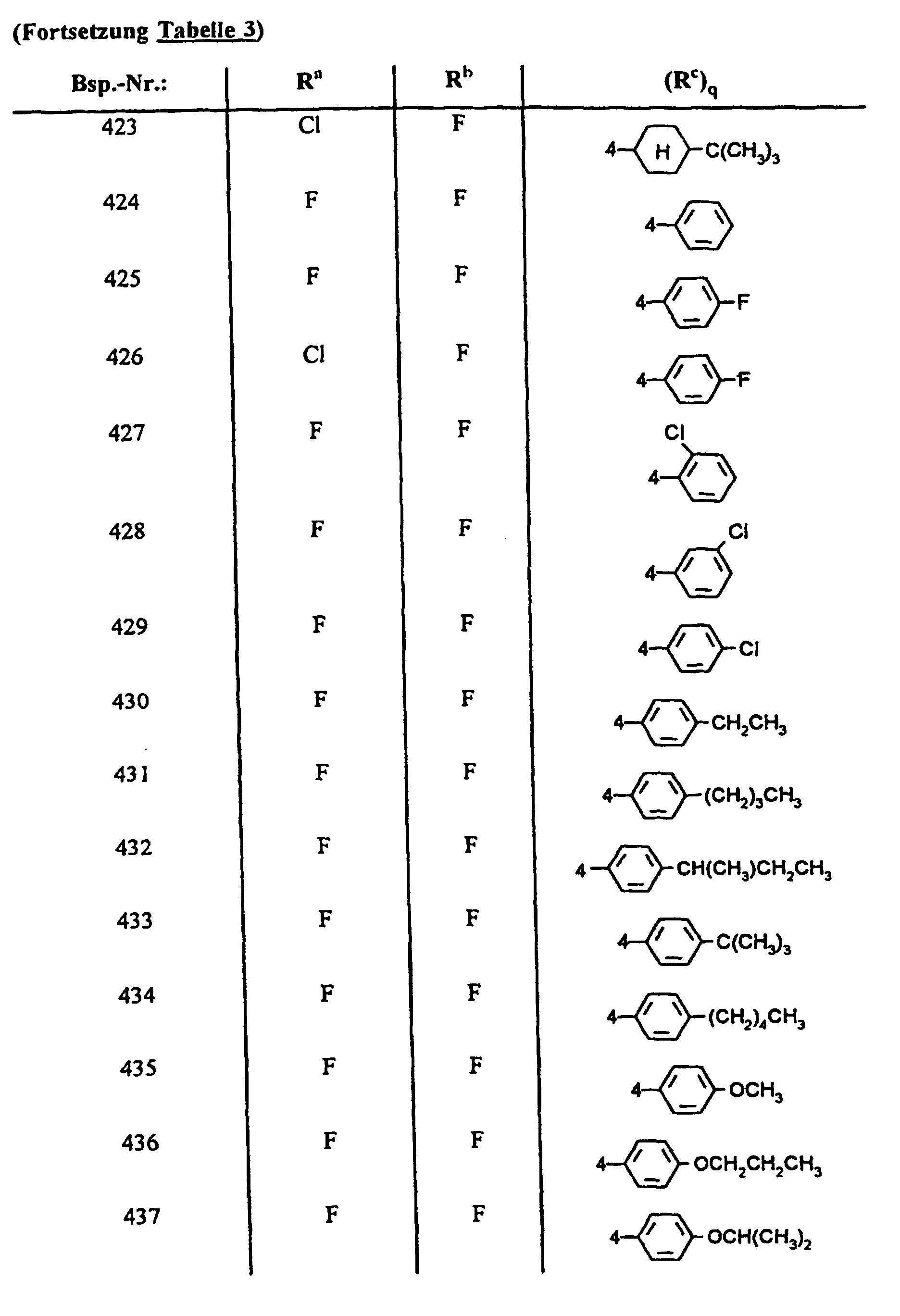

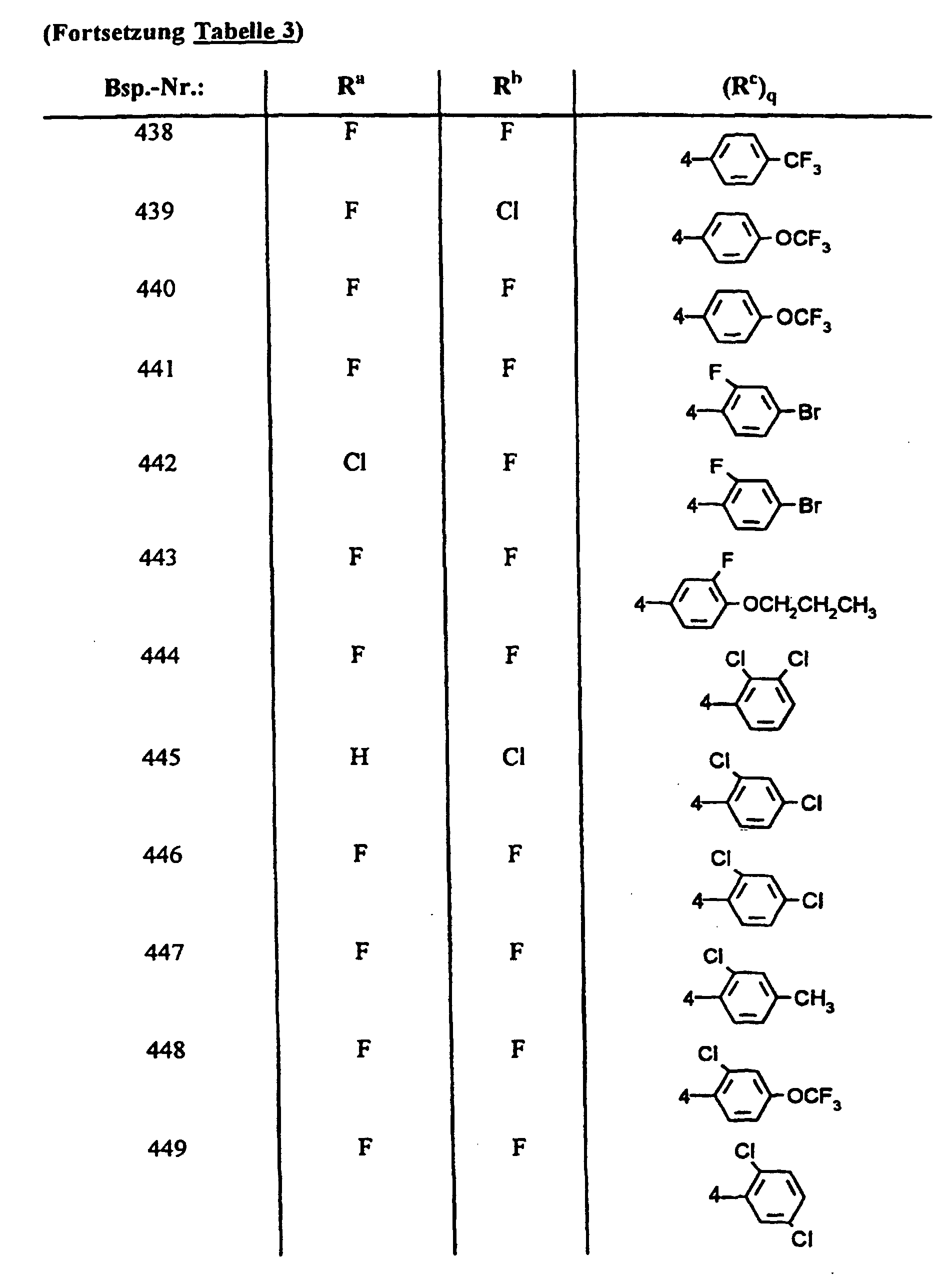

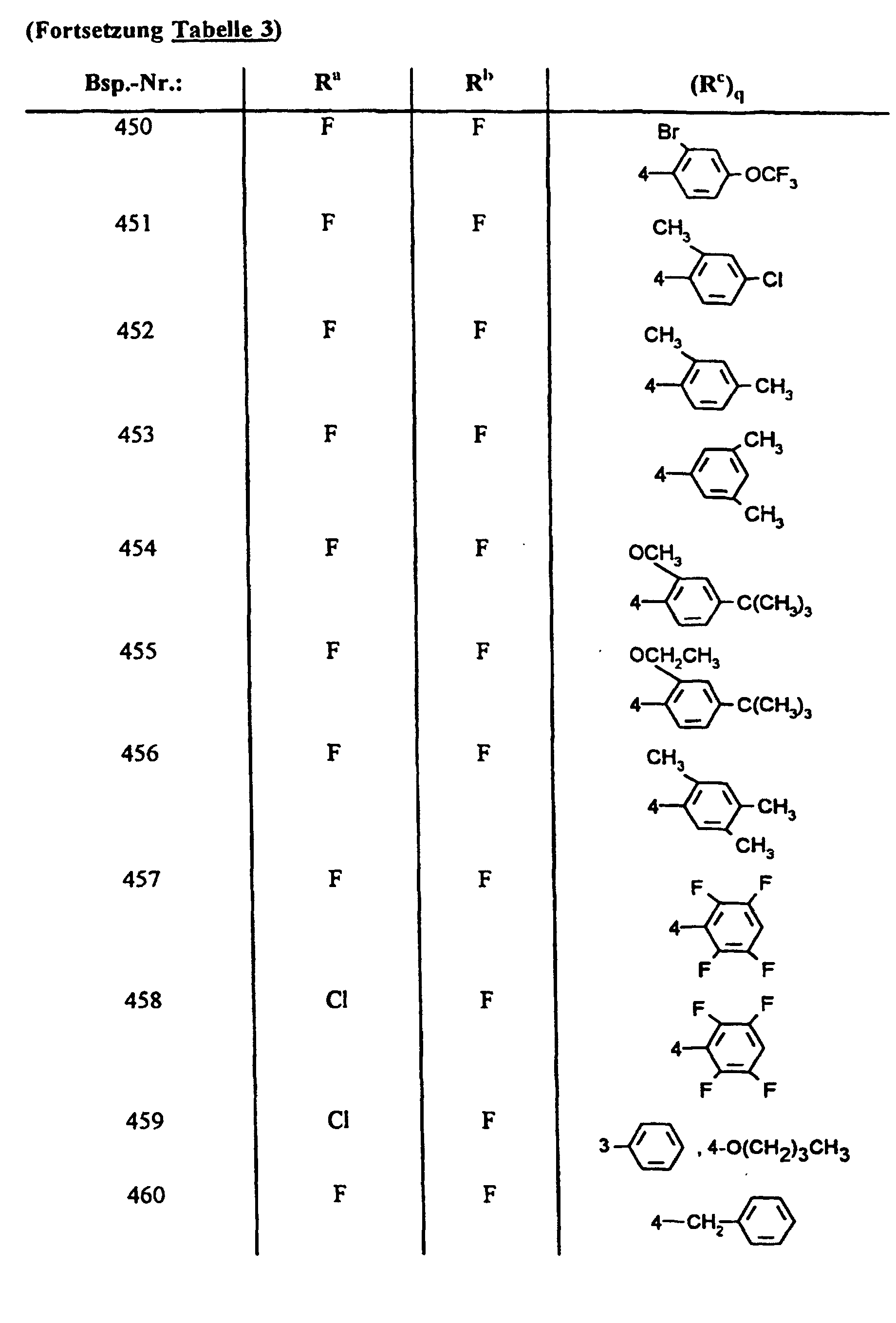

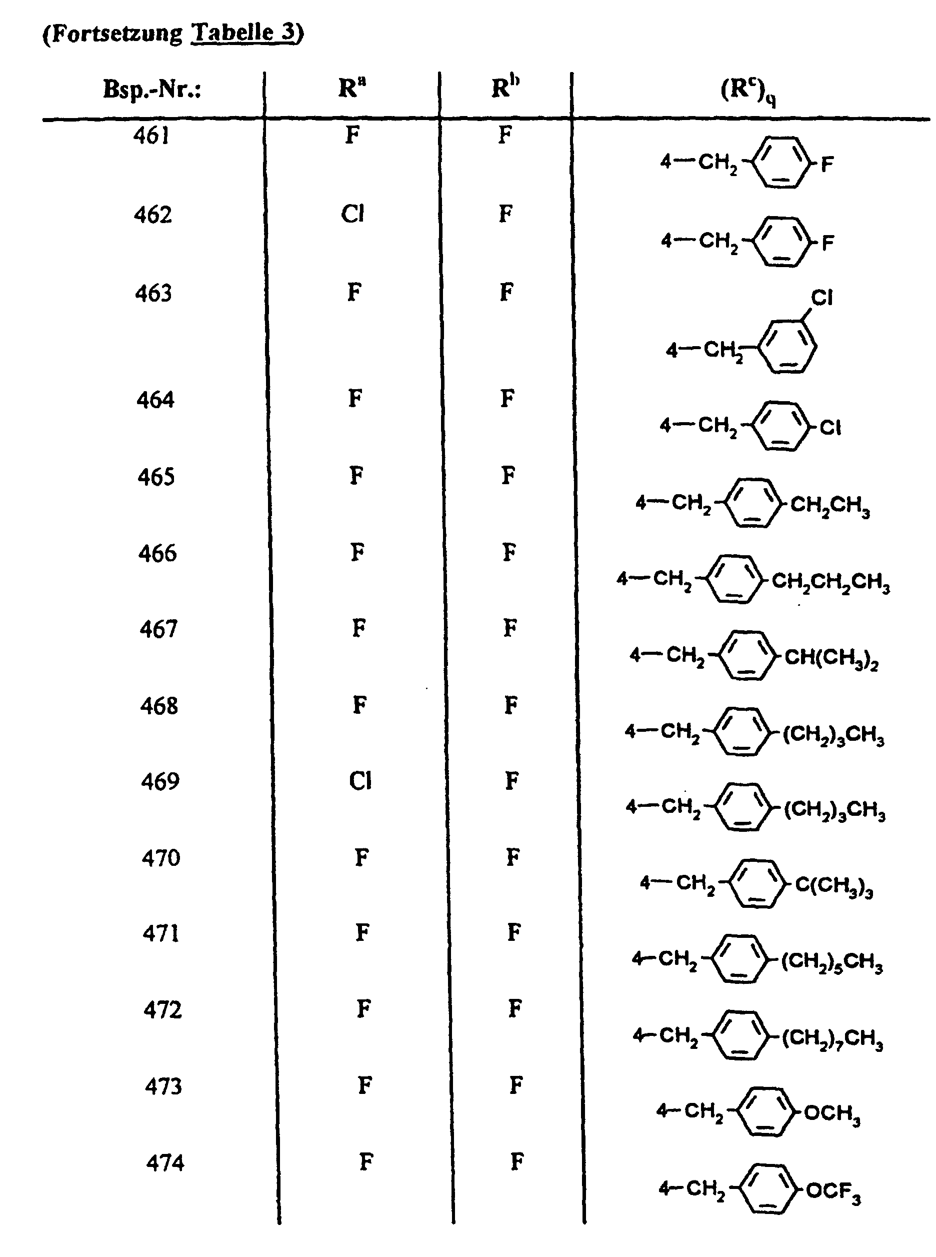

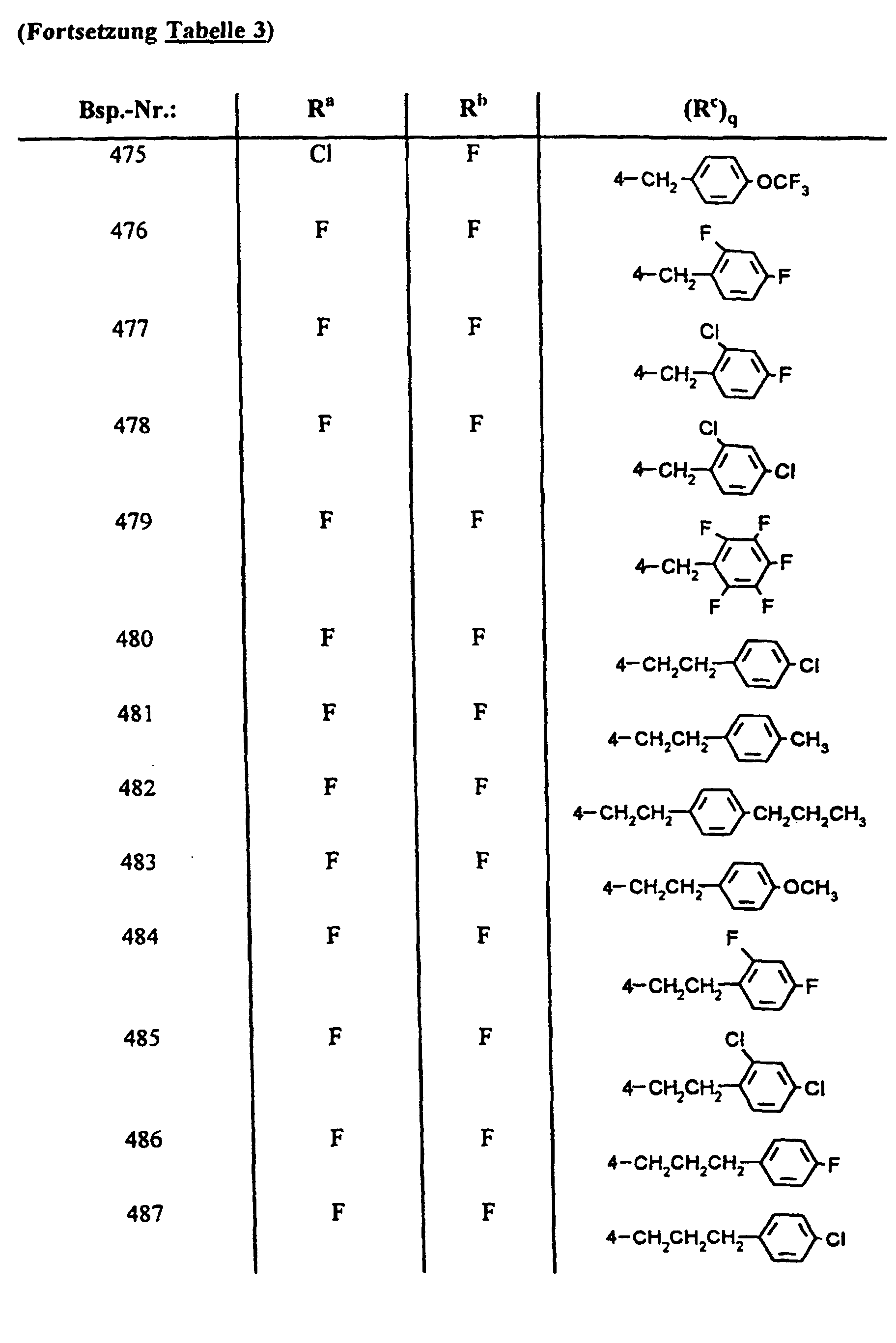

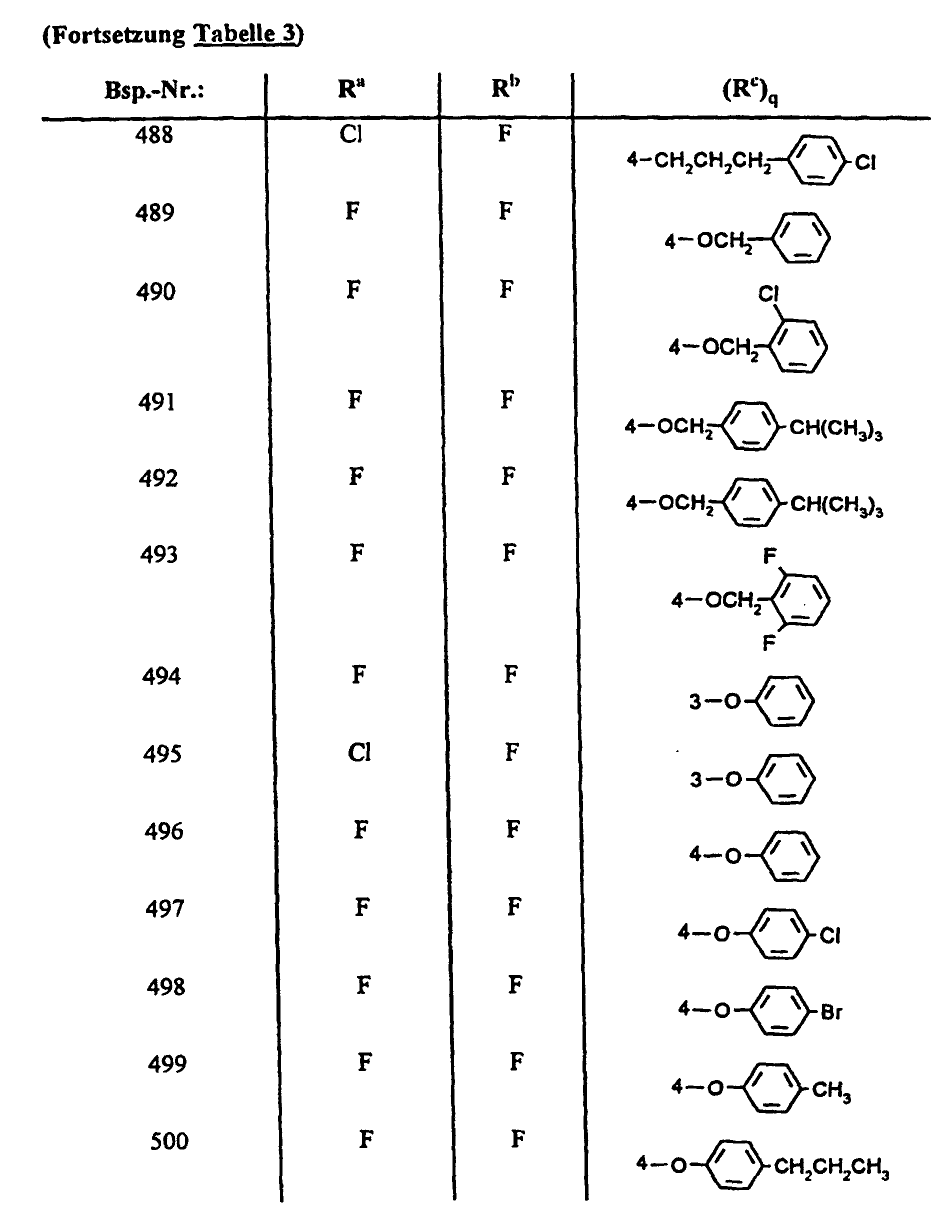

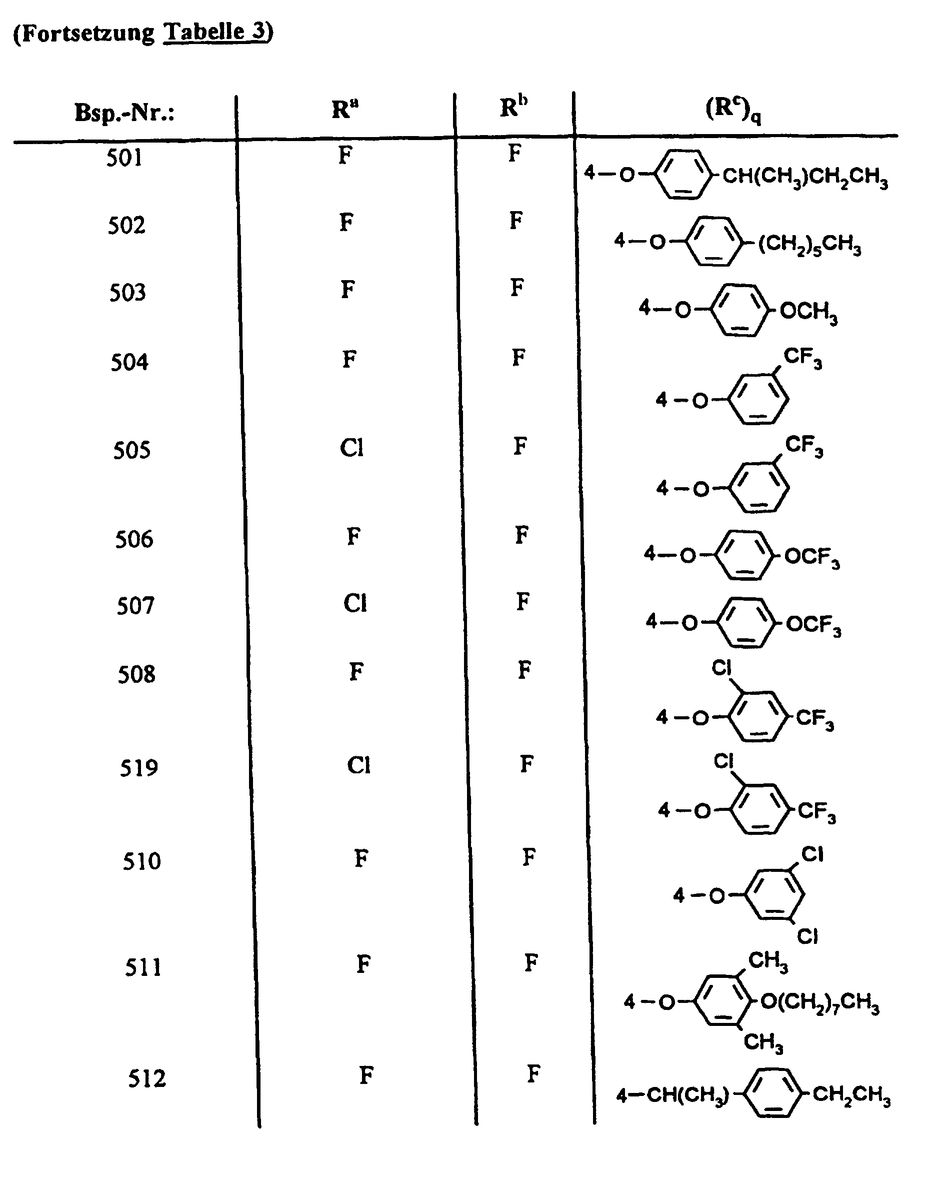

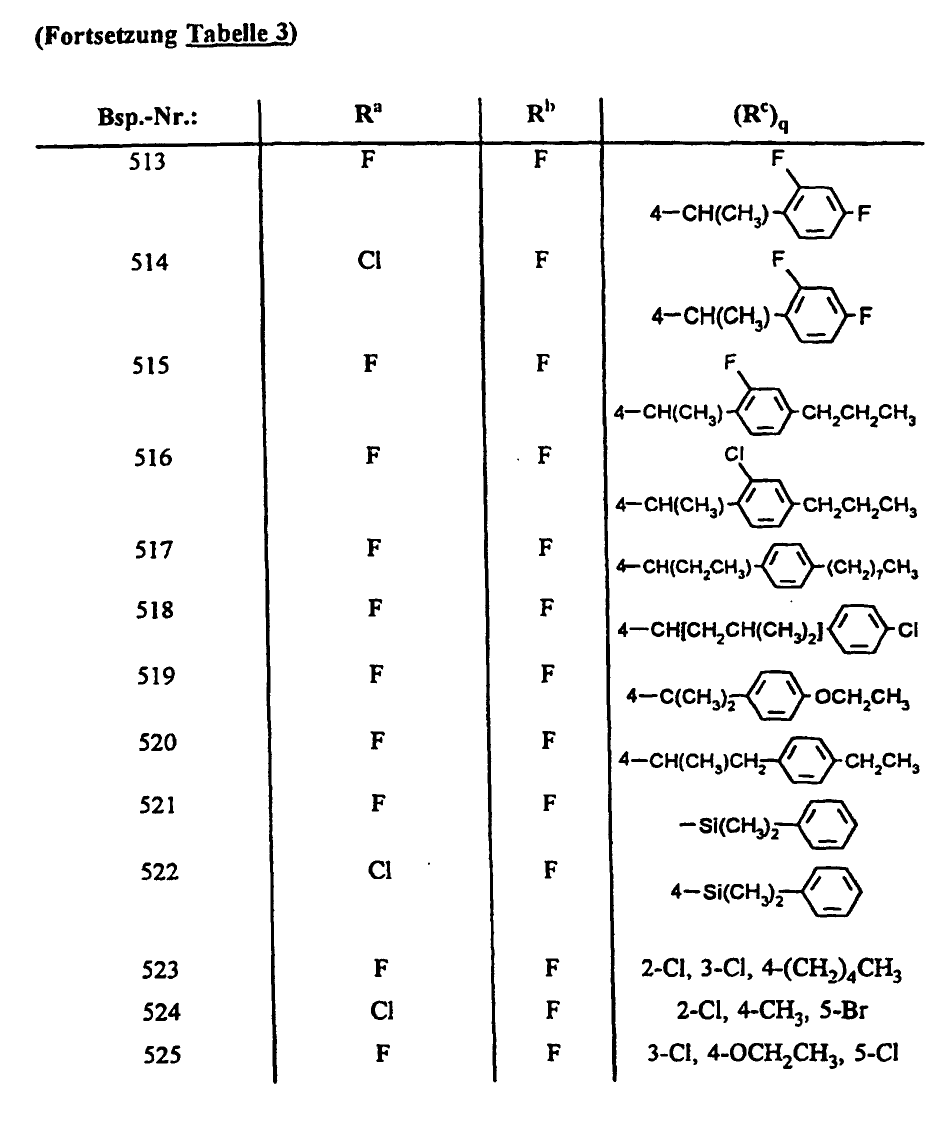

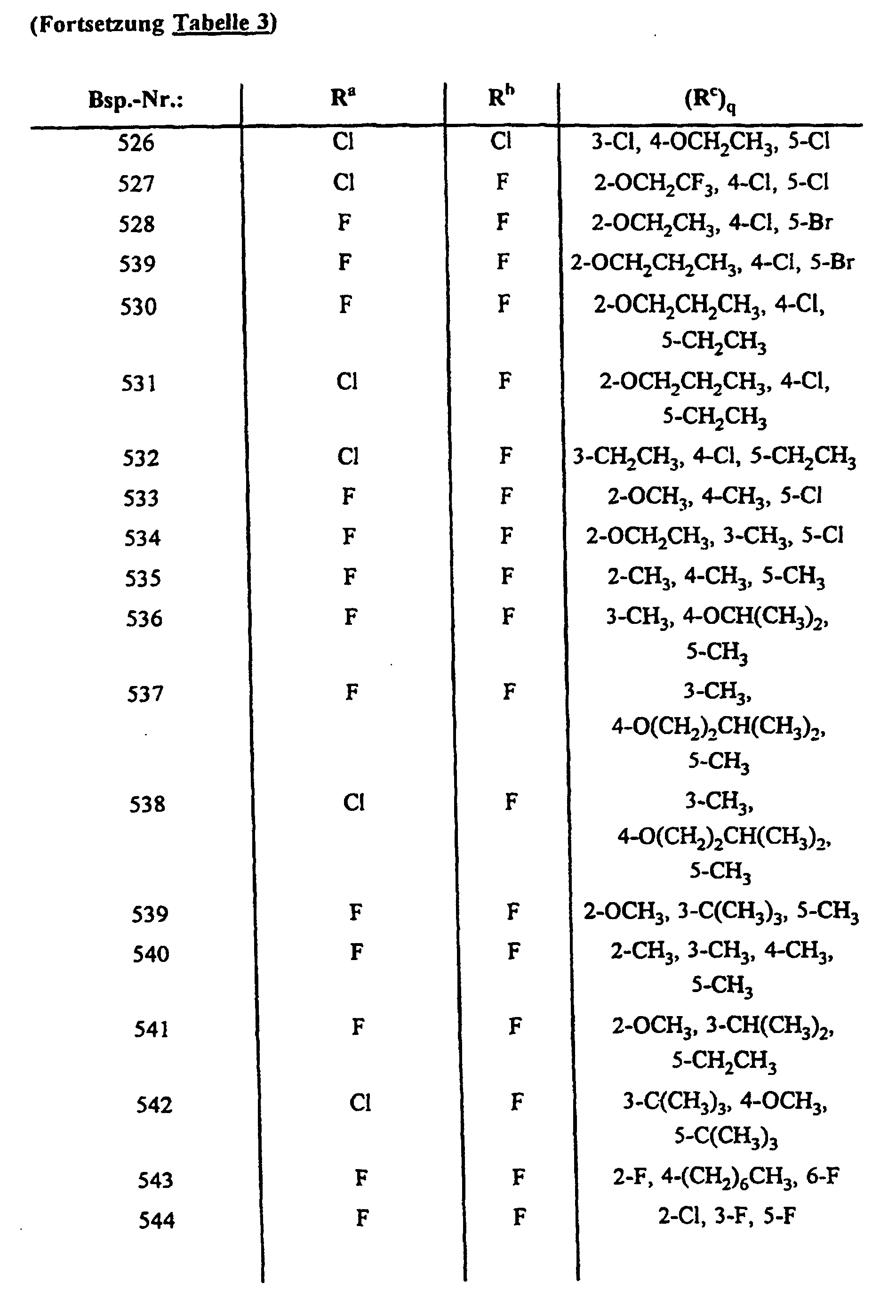

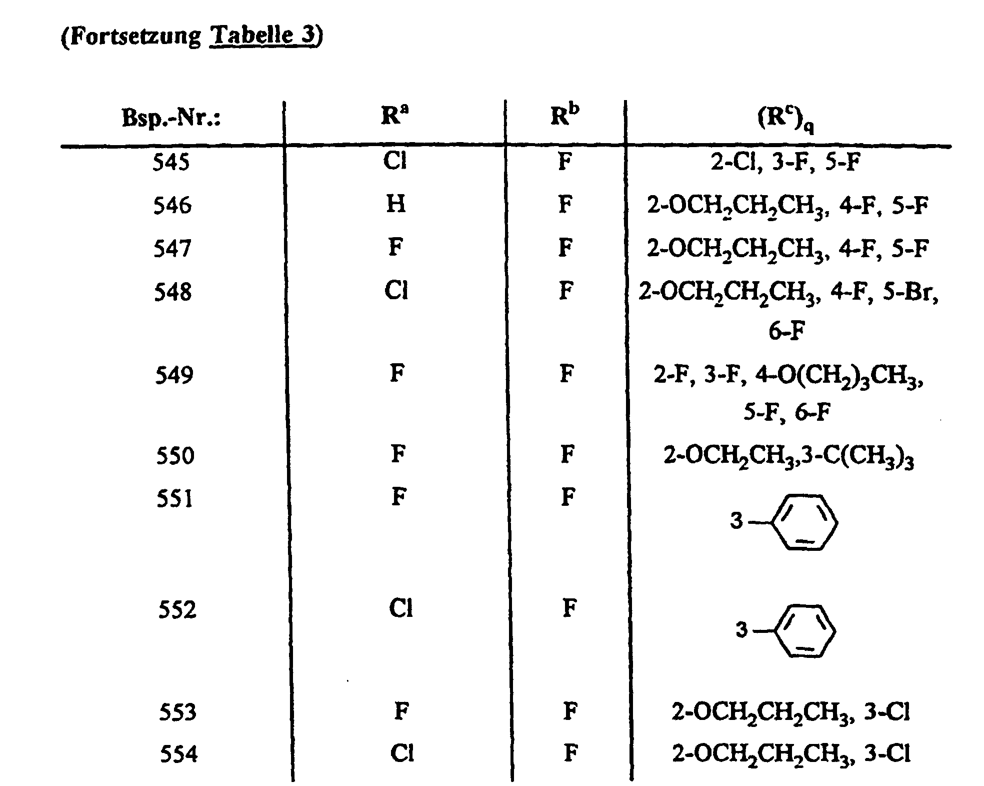

### Beispiel Nr. 555

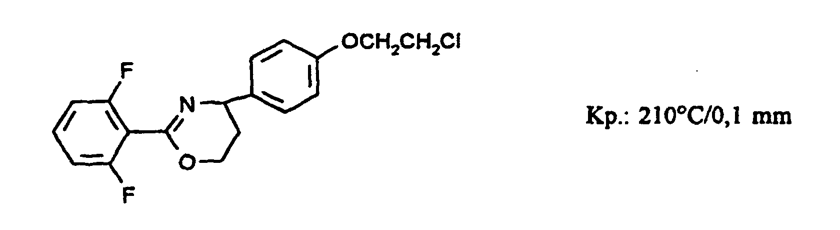

In den Tabellen werden folgende Abkürzungen verwendet:
- Me =: Methyl
- Et =: Ethyl
- Pr =: Propyl
- Bu =: Butyl
- Pen =: Pentyl
- Ph =: Phenyl

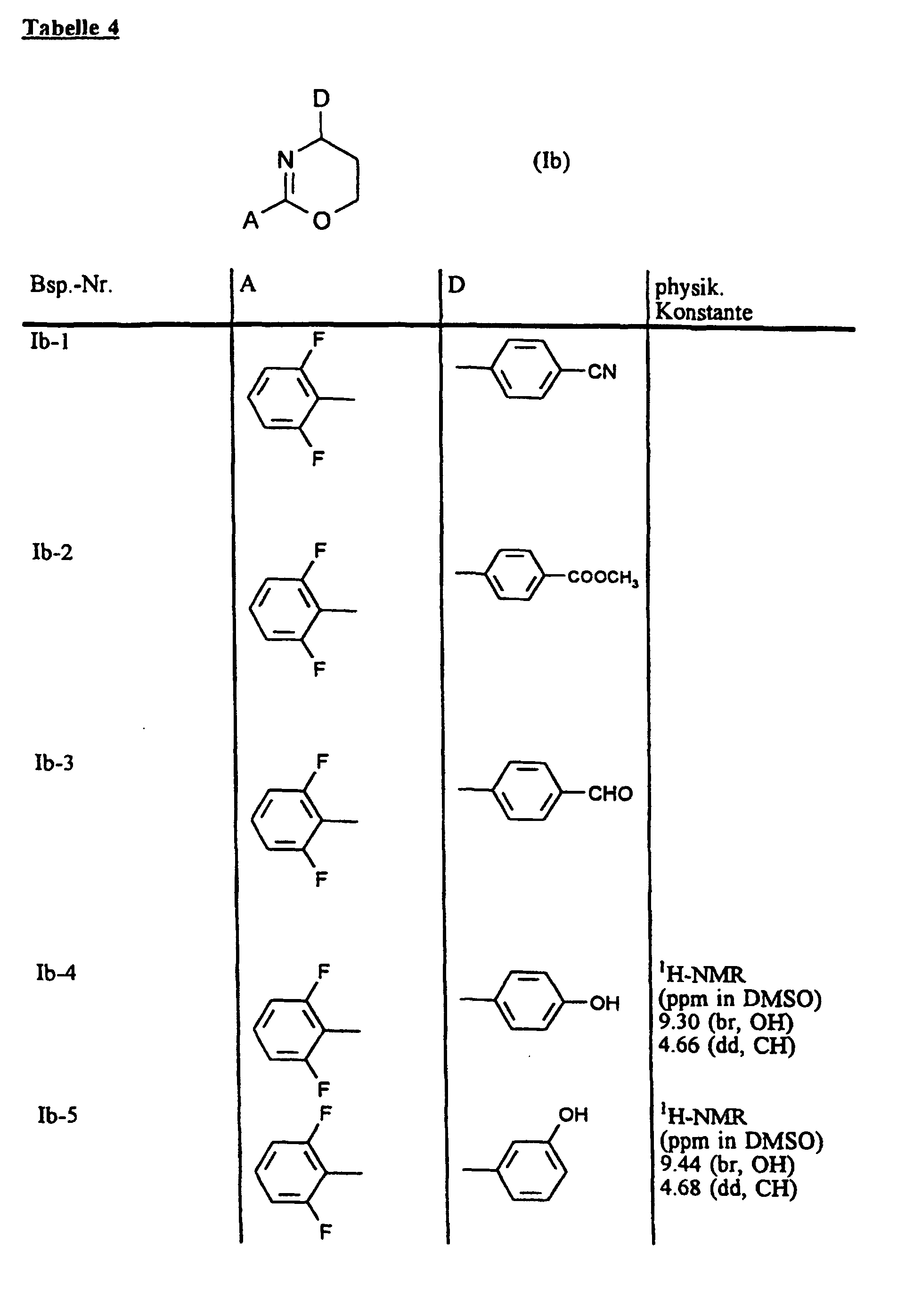

### Herstellung der Ausgangsprodukte der Formel (IIa)

### Beispiel (IIa-1)

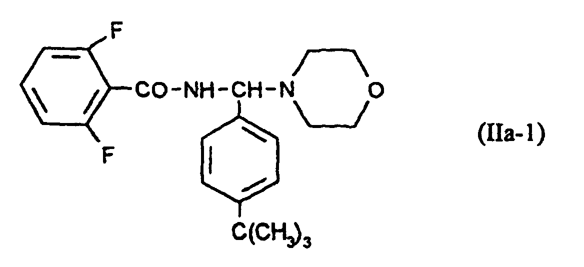

Zu einer Lösung von 12,8 g (81,5 m Mol) 2,6-Difluorbenzoesäureamid in 100 ml wasserfreiem Methanol gibt man 13,6 g (84 m Mol) 4-tert.-Butylbenzaldehyd und 8,5 g (97,7 m Mol) Morpholin und rührt das Reaktionsgemisch 24 Stunden bei ca. 40 bis 50°C. Anschließend wird mit ca. 100 ml Eiswasser verrührt, der Niederschlag abgesaugt, mit Wasser gewaschen und getrocknet.

Man erhält 21 g (67 % der Theorie) N-[Morpholin-4-yl-(4-tert.-butylphenyl)-methyl]-2,6-difluorbenzamid vom Schmelzpunkt 199-200°C.

### Beispiel (IIa-2)

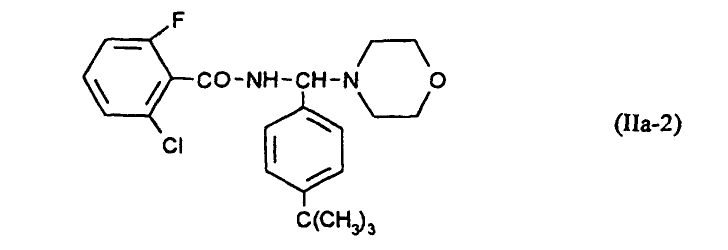

3,5 g (20 m Mol) 2-Chlor-6-fluorbenzoesäureamid werden in 30 ml Methanol mit 3,4 g (21 m Mol) 4-tert.-Butyl-benzaldehyd und 2,1 g (24,1 m Mol) Morpholin 18 Stunden bei 50°C gerührt. Nach dem Abkühlen wird das Reaktionsgemisch mit 100 ml Eiswasser verrührt, der Niederschlag abgesaugt, mit Wasser gewaschen und getrocknet.

Man erhält 6,3 g (77,4 % der Theorie) N-[Morpholin-4-yl-(4-tert.-butylphenyl)-methyl]-2-chlor-6-fluor-benzamid vom Schmelzpunkt 180-181°C.

Gemäß den Beispielen (IIa-1) und (IIa-2) sowie entsprechend den allgemeinen Verfahrensangaben werden die folgenden Ausgangsprodukte der Formel (IIa) erhalten:

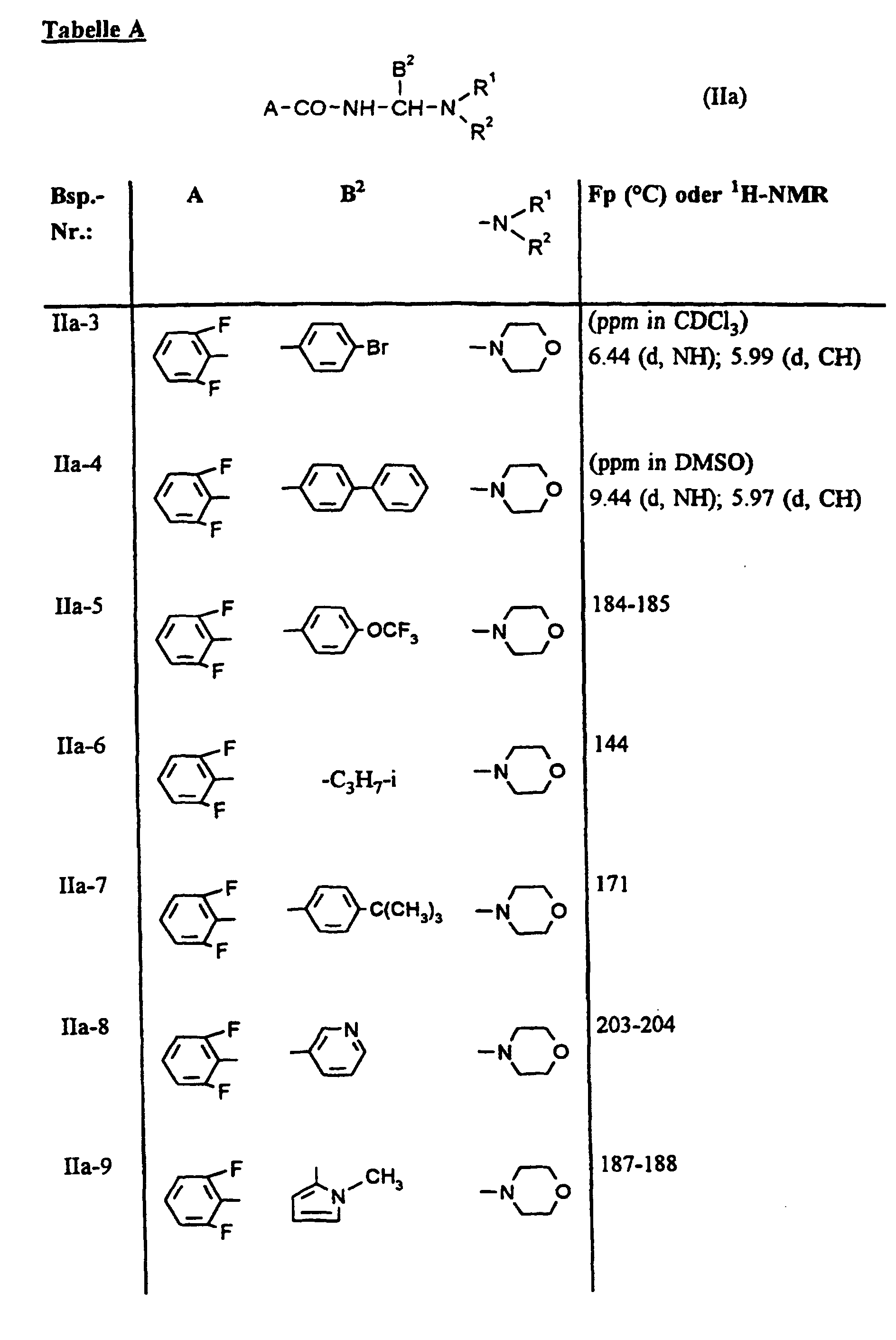

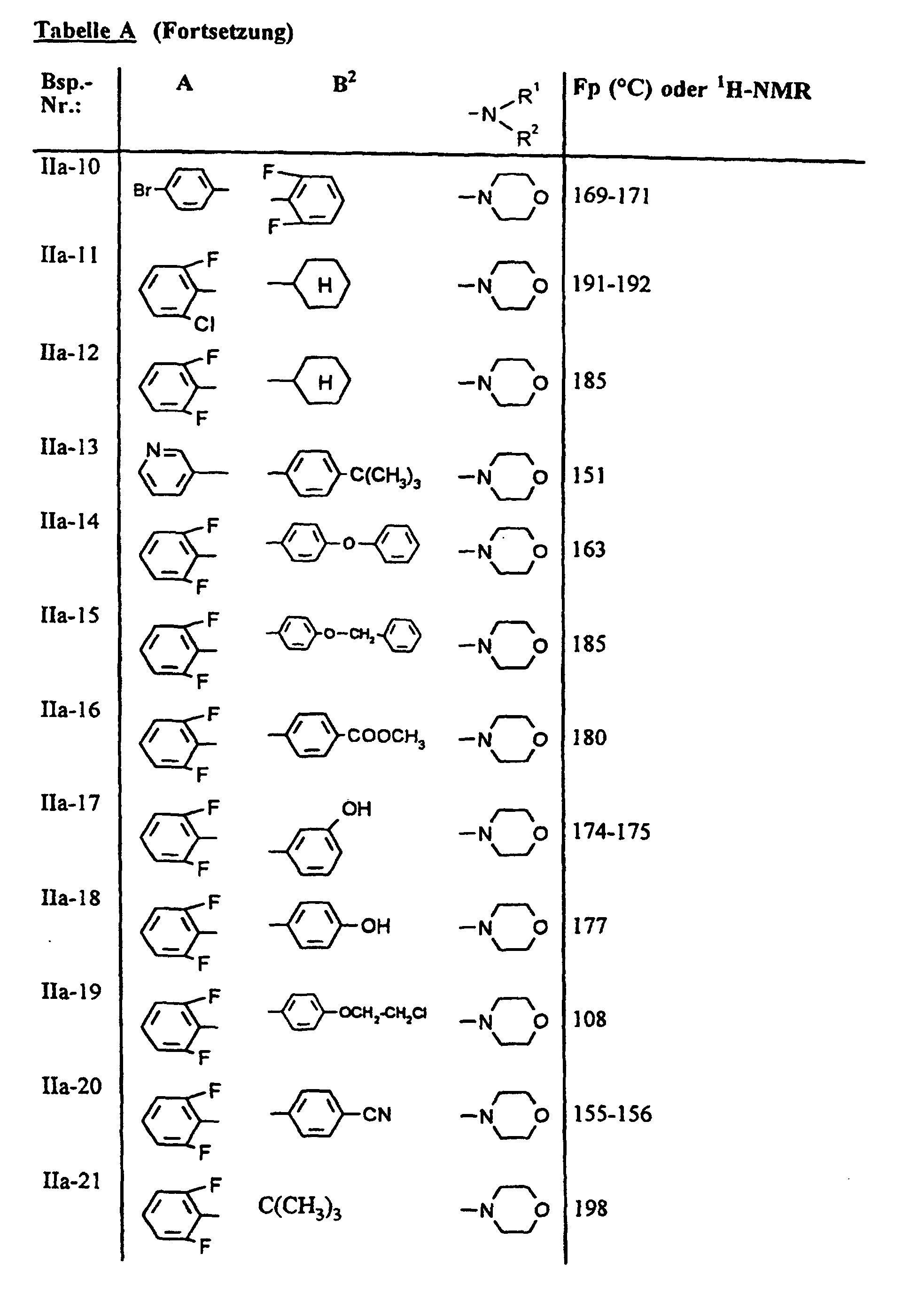

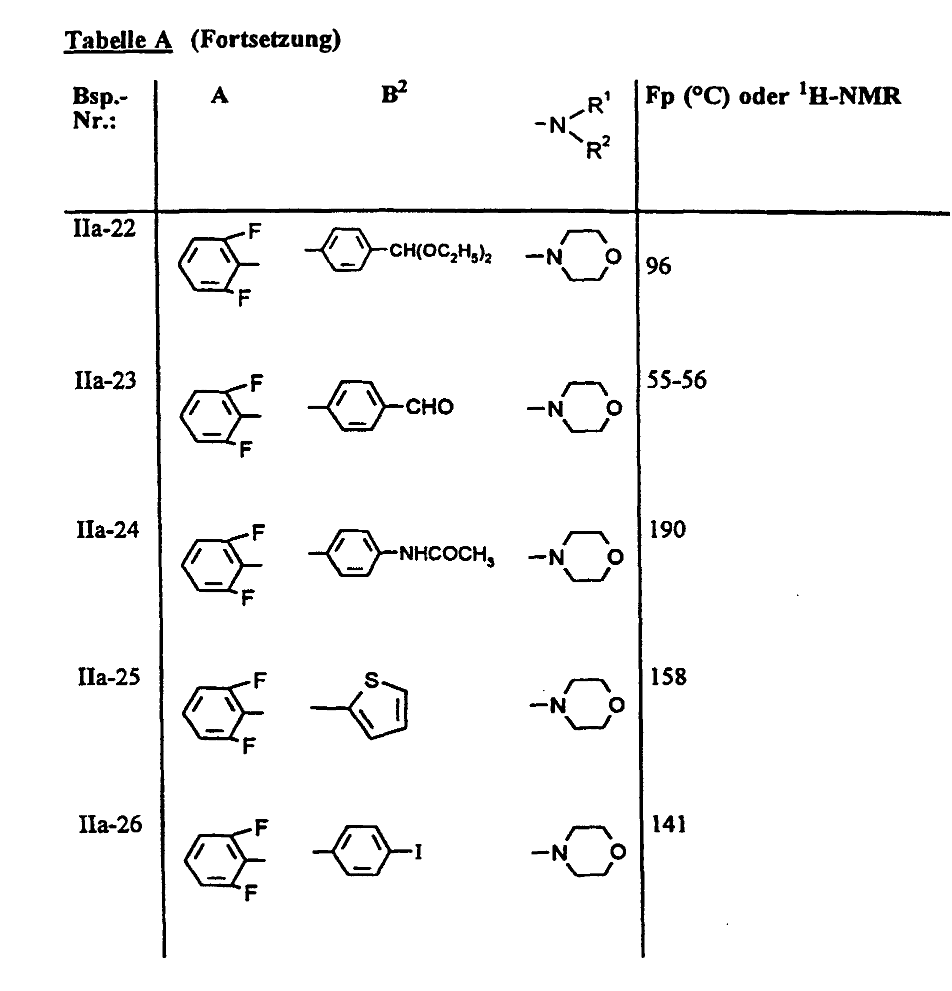

### Anwendungsbeispiele

### Beispiel A

### Plutella-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet Wurden.

In diesem Test bewirkte die Verbindung gemäß Beispiel IIa-5 bei einer beispielhaften Wirkstoffkonzentration von 0,1 % nach 7 Tagen eine Abtötung von 100 %.

## Patentansprüche

1. Verfahren zur Herstellung von Verbindungen der Formel (I) 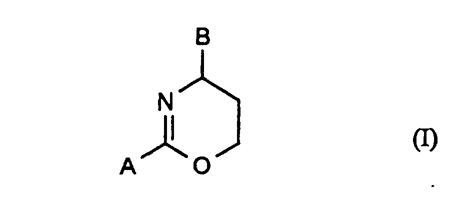 in welcher
A für substituiertes Phenyl oder für jeweils gegebenenfalls substituiertes Naphthyl, Pyridyl, Thienyl oder Pyrazolyl steht und
B für Alkyl, Alkoxyalkyl, Alkylthioalkyl, Halogenalkyl, Alkenyl, gegebenenfalls substituiertes Cycloalkyl oder jeweils gegebenenfalls substituiertes Phenyl, Phenylalkyl, Phenylalkenyl, Phenoxyalkyl, Phenylthioalkyl, Pyridyl, 2- oder 3-Pyrrolyl steht,
erhältlich durch Umsetzung eines Amid-Derivates der Formel (II) 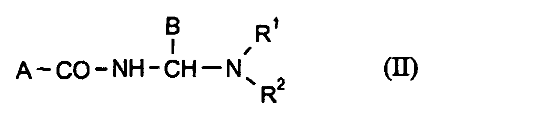 in welcher
A und B die oben angegebene Bedeutung haben und
R¹ und R² gleich oder verschieden sind und jeweils für Alkyl stehen oder
gemeinsam mit dem N-Atom, an das sie gebunden sind, für einen gegebenenfalls substituierten Heterocyclus stehen,
mit Ethylen in Gegenwart von Chlorwasserstoff-Gas und eines Katalysators, gegebenenfalls in Gegenwart eines Verdünnungsmittels.

2. Verfahren nach Anspruch 1, in welchem
A für Phenyl, das einfach bis fünffach, gleich oder verschieden substituiert ist durch Halogen, C₁-C₆-Alkyl, C₁-C₆-Alkoxy, C₁-C₆-Alkylthio, C₁-C₆-Halogenalkyl, C₁-C₆-Halogenalkoxy, C₁-C₆-Halogenalkylthio, Nitro, Cyano oder durch gegebenenfalls einfach bis fünffach, gleich oder verschieden durch Halogen, C₁-C₆-Alkyl, C₁-C₆-Halogenalkyl, C₁-C₆-Alkoxy, C₁-C₆-Halogenalkoxy, C₁-C₆-Alkylthio oder C₁-C₆-Halogenalkylthio substituiertes Phenyl,
für gegebenenfalls einfach bis dreifach, gleich oder verschieden durch Halogen, C₁-C₆-Alkyl, C₁-C₆-Alkoxy oder C₁-C₆-Halogenalkoxy substituiertes Naphthyl,
für gegebenenfalls einfach bis dreifach, gleich oder verschieden durch Halogen, C₁-C₆-Alkyl, C₁-C₆-Alkoxy, C₁-C₆-Halogenalkyl, C₁-C₆-Halogenalkoxy oder Cyano substituiertes Pyridyl,
für gegebenenfalls einfach bis dreifach, gleich oder verschieden durch Halogen oder C₁-C₆-Alkyl substituiertes Thienyl,
oder für gegebenenfalls einfach bis dreifach, gleich oder verschieden durch Halogen oder C₁-C₃-Alkyl substituiertes Pyrazolyl steht und
B für C₁-C₆-Alkyl, C₁-C₆-Alkoxy-C₁-C₆-alkyl, C₁-C₆-Alkylthio-C₁-C₆-alkyl, C₁-C₆-Halogenalkyl oder C₂-C₆-Alkenyl,
für gegebenenfalls einfach bis fünffach, gleich oder verschieden durch Halogen, C₁-C₆-Alkyl, C₁-C₆-Alkoxy, C₁-C₆-Halogenalkyl oder C₁-C₆-Halogenalkoxy substituiertes C₃-C₇-Cycloalkyl,
für gegebenenfalls einfach bis dreifach, gleich oder verschieden durch Halogen, C₁-C₆-Alkyl, C₁-C₆-Alkoxy, C₁-C₆-Halogenalkyl, C₁-C₆-Halogenalkoxy oder Cyano substituiertes Pyridyl,
für jeweils gegebenenfalls einfach oder zweifach, gleich oder verschieden durch Halogen oder C₁-C₄-Alkyl substituiertes α-Pyrrolyl oder β-Pyrrolyl,
für durch Hydroxy, CHO, Cyano, Carboxyl, Poly-C₁-C₈-alkoxy-C₁-C₈-alkyl oder C₁-C₈-Alkoxycarbonyl substituiertes Phenyl oder
für jeweils gegebenenfalls einfach bis fünffach, gleich oder verschieden im Phenylring substituiertes Phenyl, Benzyl, Pheneth-1-yl, Pheneth-2-yl, Phenoxymethyl, Phenylthiomethyl, Phenylthioeth-1-yl, Phenoxyeth-1-yl, Phenoxyeth-2-yl oder Styryl, wobei jeweils als Substituenten in Frage kommen
Halogen,
C₁-C₁₈-Alkyl,
C₁-C₈-Alkoxy-C₁-C₈-alkyl,
C₁-C₈-Halogenalkoxy,
C₁-C₄-Halogenalkyl,
C₁-C₁₈-Alkoxy, das gegebenenfalls durch weitere 1 bis 3 Sauerstoffatome unterbrochen ist,
C₁-C₁₈-Alkylthio,
C₁-C₈-Halogenalkylthio,
Tri-(C₁-C₈)-alkylsilyl,
Phenyl-di-(C₁-C₈)-alkylsilyl,
3,4-Difluormethylendioxo,
3,4-Tetrafluorethylendioxo,
eine anellierte Benzogruppe,
eine anellierte C₃-C₄-Alkandiylgruppe,
gegebenenfalls durch Halogen, C₁-C₄-Alkyl oder C₃-C₆-Cycloalkyl substituiertes Benzyliminooxymethyl,
jeweils gegebenenfalls durch C₁-C₆-Alkyl, C₁-C₆-Alkoxy, Cyclohexyl oder Phenyl substituiertes Cyclohexyl oder Cyclohexyloxy,
gegebenenfalls einfach oder zweifach, gleich oder verschieden durch Halogen, C₁-C₄-Alkyl oder C₁-C₄-Halogenalkyl substituiertes Pyridyloxy,
jeweils gegebenenfalls einfach bis fünffach, gleich oder verschieden durch Halogen, C₁-C₁₂-Alkyl, C₁-C₄-Halogenalkyl, C₁-C₁₂-Alkoxy, C₁-C₆-Halogenalkoxy, C₁-C₆-Alkoxy-C₁-C₆-alkyl, C₁-C₆-Alkoxyethylenoxy, C₁-C₆-Alkylthio oder C₁-C₆-Halogenalkylthio substituiertes Phenyl, Phenyl-C₁-C₆-alkyl, Phenoxy, Phenylthio, Phenyl-C₁-C₆-alkyloxy oder Benzylthio steht.

3. Verfahren nach Anspruch 1, in welchem
A für Phenyl, das einfach bis vierfach, gleich oder verschieden substituiert ist durch Fluor, Chlor, Brom, C₁-C₃-Alkyl, C₁-C₃-Alkoxy, C₁-C₃-Alkylthio, einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃, SCHF₂, Nitro, Cyano, oder durch gegebenenfalls einfach bis vierfach, gleich oder verschieden durch Fluor, Chlor, Brom, C₁-C₄-Alkyl, C₁-C₄-Alkoxy, C₁-C₄-Alkylthio, einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃ oder SCHF₂ substituiertes Phenyl,
für gegebenenfalls einfach bis dreifach, gleich oder verschieden durch Fluor, Chlor, C₁-C₆-Alkyl, C₁-C₆-Alkoxy oder einfach bis dreifach, gleich oder verschieden durch Fluor oder Chlor substituiertes C₁-C₆-Alkoxy substituiertes Naphthyl,
für gegebenenfalls einfach oder zweifach, gleich oder verschieden durch Fluor, Chlor, C₁-C₃-Alkyl, C₁-C₃-Alkoxy, CF₃, OCF₃ oder Cyano substituiertes Pyridyl,
für gegebenenfalls einfach oder zweifach, gleich oder verschieden durch Chlor, Brom, Methyl oder Ethyl substituiertes Thienyl oder
für gegebenenfalls einfach bis dreifach, gleich oder verschieden durch Fluor, Chlor, Brom oder C₁-C₃-Alkyl substituiertes Pyrazolyl steht und
B für C₁-C₄-Alkyl, C₁-C₄-Alkoxy-C₁-C₄-alkyl, C₁-C₄-Alkylthio-C₁-C₄-alkyl, C₁-C₄-Halogenalkyl oder C₂-C₆-Alkenyl,
für jeweils gegebenenfalls einfach bis dreifach, gleich oder verschieden durch Fluor, Chlor, Brom, C₁-C₃-Alkyl, C₁-C₃-Alkoxy, CF₃ oder OCF₃ substituiertes Cyclopropyl, Cyclopentyl oder Cyclohexyl,
für gegebenenfalls einfach oder zweifach, gleich oder verschieden durch Fluor, Chlor, C₁-C₃-Alkyl, C₁-C₃-Alkoxy, CF₃, OCF₃ oder Cyano substituiertes Pyridyl,
für jeweils gegebenenfalls einfach oder zweifach, gleich oder verschieden durch Fluor, Chlor oder C₁-C₃-Alkyl substituiertes α-Pyrrolyl oder β-Pyrrolyl,
für durch CHO, Cyano, Hydroxy, Carboxyl, Poly-C₁-C₈-alkoxy-C₁-C₆-alkyl oder C₁-C₈-Alkoxycarbonyl substituiertes Phenyl oder
für jeweils gegebenenfalls einfach bis vierfach, gleich oder verschieden im Phenylring substituiertes Phenyl, Benzyl, Pheneth-1-yl, Pheneth-2-yl, Phenoxymethyl, Phenylthiomethyl, Phenylthioeth-1-yl, Phenoxyeth-1-yl, Phenoxyeth-2-yl, oder Styryl, wobei jeweils als Substituenten in Frage kommen
Fluor, Chlor, Brom,
C₁-C₁₈-Alkyl,
C₁-C₆-Alkoxy-C₁-C₈-alkyl,
einfach bis sechsfach, gleich oder verschieden durch Fluor oder Chlor substituiertes C₁-C₈-Alkoxy,
einfach bis fünffach, gleich oder verschieden durch Fluor oder Chlor substituiertes C₁-C₂-Alkyl,
C₁-C₁₈-Alkoxy oder -(OC₂H₄)₁₋₃-O-C₁-C₆-alkyl,
C₁-C₁₅-Alkylthio,
einfach bis sechsfach, gleich oder verschieden durch Fluor oder Chlor substituiertes C₁-C₈-Alkylthio,
Tri-(C₁-C₆)-alkylsilyl,
Phenyl-di-(C₁-C₆)-alkylsilyl,
3,4-Difluormethylendioxo,
3,4-Tetrafluorethylendioxo,
eine anellierte Benzogruppe,
eine anellierte C₄-Alkandiylgruppe,
die Gruppen 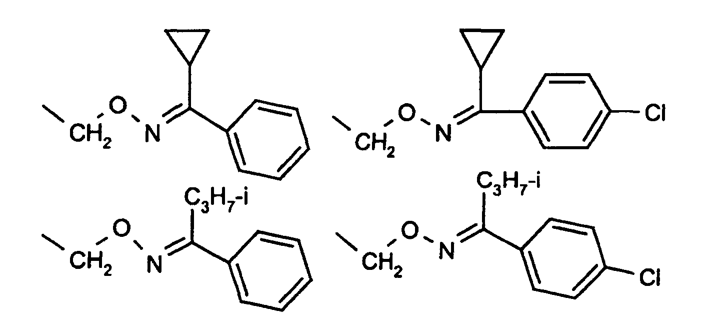 jeweils gegebenenfalls durch C₁-C₄-Alkyl, C₁-C₄-Alkoxy, Cyclohexyl oder
Phenyl substituiertes Cyclohexyl oder Cyclohexyloxy,
gegebenenfalls einfach oder zweifach, gleich oder verschieden durch Fluor, Chlor oder CF₃ substituiertes Pyridyloxy,
jeweils gegebenenfalls einfach bis vierfach, gleich oder verschieden durch
Fluor, Chlor, Brom, C₁-C₁₂-Alkyl, einfach bis sechsfach, gleich oder verschieden durch Fluor oder Chlor substituiertes C₁-C₄-Alkyl, C₁-C₁₂-Alkoxy, einfach bis sechsfach, gleich oder verschieden durch Fluor oder Chlor substituiertes C₁-C₄-Alkoxy, C₁-C₄-Alkoxy-C₁-C₄-alkyl, C₁-C₄-Alkoxyethylenoxy, C₁-C₄-Alkylthio oder einfach bis sechsfach, gleich oder verschieden durch Fluor oder Chlor substituiertes C₁-C₄-Alkylthio substituiertes Phenyl, Phenyl-C₁-C₆-alkyl, Phenoxy, Phenylthio, Phenyl-C₁-C₆-alkyloxy oder Benzylthio steht.

4. Verfahren nach Anspruch 1, in welchem
A für Phenyl, das einfach bis dreifach, gleich oder verschieden substituiert ist durch Fluor, Chlor, Brom, C₁-C₃-Alkyl, C₁-C₃-Alkoxy, C₁-C₃-Alkylthio, einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃, SCHF₂, Nitro, Cyano, oder durch gegebenenfalls einfach bis vierfach, gleich oder verschieden durch Fluor, Chlor, Brom, C₁-C₃-Alkyl, C₁-C₃-Alkoxy, C₁-C₃-Alkylthio, einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃ oder SCHF₂ substituiertes Phenyl steht und
B für C₁-C₄-Alkyl, C₁-C₂-Alkoxy-C₁-C₄-alkyl, C₁-C₂-Alkylthio-C₁-C₄-alkyl, C₁-C₂-Halogenalkyl oder C₂-C₄-Alkenyl,
für jeweils gegebenenfalls einfach bis dreifach, gleich oder verschieden durch Fluor, Chlor, CH₃, C₂H₅, OCH₃, CF₃ oder OCF₃ substituiertes Cyclopropyl, Cyclopentyl oder Cyclohexyl,
für gegebenenfalls einfach oder zweifach, gleich oder verschieden durch Fluor, Chlor, CH₃, C₂H₅, OCH₃, CF₃, OCF₃ oder Cyano substituiertes Pyridyl,
für jeweils gegebenenfalls einfach oder zweifach, gleich oder verschieden durch Fluor, Chlor, CH₃ oder C₂H₅ substituiertes α-Pyrrolyl oder β-Pyrrolyl,
für durch CHO, Hydroxy, Cyano, Carboxyl, Di-C₁-C₆-alkoxy-C₁-C₄-alkyl oder C₁-C₆-Alkoxycarbonyl substituiertes Phenyl oder
für jeweils gegebenenfalls einfach bis dreifach, gleich oder verschieden im Phenylring substituiertes Phenyl, Benzyl, Pheneth-1-yl, Pheneth-2-yl, Phenoxymethyl, Phenylthiomethyl, Phenylthioeth-1-yl, Phenoxyeth-1-yl, Phenoxyeth-2-yl, oder Styryl, wobei jeweils als Substituenten in Frage kommen
Fluor, Chlor, Brom,
C₁-C₁₈-Alkyl,
C₁-C₆-Alkoxy-C₁-C₈-alkyl,
einfach bis sechsfach, gleich oder verschieden durch Fluor oder Chlor substituiertes C₁-C₈-Alkoxy,
einfach bis fünffach, gleich oder verschieden durch Fluor oder Chlor substituiertes C₁-C₂-Alkyl,
C₁-C₁₈-Alkoxy oder -(OC₂H₄)₁₋₃-O-C₁-C₆-alkyl,
C₁-C₁₅-Alkylthio,
einfach bis sechsfach, gleich oder verschieden durch Fluor oder Chlor substituiertes C₁-C₈-Alkylthio,
Tri-(C₁-C₆)-alkylsilyl,
Phenyl-di-(C₁-C₆)-alkylsilyl,
3,4-Difluormethylendioxo,
3,4-Tetrafluorethylendioxo,
eine anellierte Benzogruppe,
eine anellierte C₄-Alkandiylgruppe,
die Gruppen 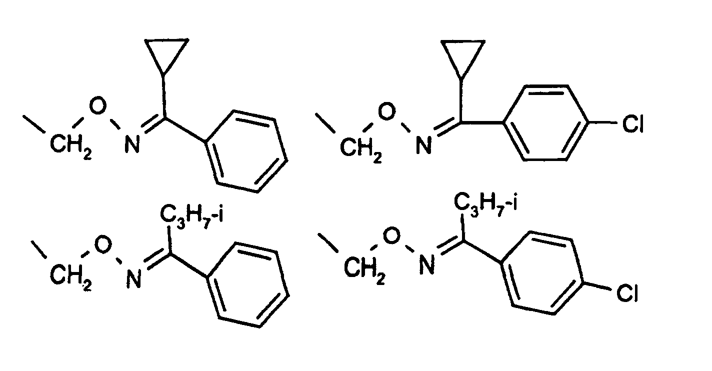 jeweils gegebenenfalls durch C₁-C₄-Alkyl, C₁-C₄-Alkoxy, Cyclohexyl oder
Phenyl substituiertes Cyclohexyl oder Cyclohexyloxy,
gegebenenfalls einfach oder zweifach, gleich oder verschieden durch Fluor, Chlor oder CF₃ substituiertes Pyridyloxy,
jeweils gegebenenfalls einfach bis vierfach, gleich oder verschieden durch Fluor, Chlor, Brom, C₁-C₁₂-Alkyl, einfach bis sechsfach durch Fluor oder Chlor substituiertes C₁-C₄-Alkyl, C₁-C₁₂-Alkoxy, einfach bis sechsfach, gleich oder verschieden durch Fluor oder Chlor substituiertes C₁-C₄-Alkoxy, C₁-C₄-Alkoxy-C₁-C₄-alkyl, C₁-C₄-Alkoxyethylenoxy, C₁-C₄-Alkylthio oder einfach bis sechsfach, gleich oder verschieden durch Fluor oder Chlor substituiertes C₁-C₄-Alkylthio substituiertes Phenyl, Phenyl-C₁-C₆-alkyl, Phenoxy, Phenylthio, Phenyl-C₁-C₆-alkyloxy oder Benzylthio steht.

5. Verfahren nach Anspruch 1, in welchem
A für einfach oder zweifach, gleich oder verschieden durch Fluor oder Chlor substituiertes Phenyl steht und
B für einfach bis dreifach, gleich oder verschieden durch Fluor, Chlor, Brom, C₁-C₈-Alkyl, C₁-C₈-Alkoxy, C₁-C₄-Halogenalkyl, C₁-C₄-Halogenalkoxy, C₁-C₈-Alkylthio oder C₃-C₆-Cycloalkyl substituiertes Phenyl oder
für einfach durch jeweils gegebenenfalls einfach oder zweifach, gleich oder verschieden im Phenylteil durch Fluor, Chlor, Brom, C₁-C₆-Alkyl, C₁-C₆-Alkoxy, C₁-C₄-Halogenalkyl oder C₁-C₄-Halogenalkoxy substituiertes Phenyl, Phenyl-C₁-C₃-alkyl, Phenyloxy oder Benzyloxy substituiertes Phenyl,
für jeweils gegebenenfalls einfach oder zweifach, gleich oder verschieden durch Fluor, Chlor, Methyl, Ethyl oder Trifluormethyl substituiertes Pyridyl, 2- oder 3-Pyrrolyl oder
für durch CHO, Hydroxy, Cyano, Carboxyl, Di-C₁-C₆-alkoxy-C₁-C₄-alkyl oder C₁-C₆-Alkoxycarbonyl substituiertes Phenyl steht.

6. Verfahren nach einem der Ansprüche 1 bis 5 in welchem
R¹ und R² gleich oder verschieden sind und für C₁-C₄-Alkyl stehen oder gemeinsam mit dem N-Atom, an das sie gebunden sind, für einen gegebenenfalls einfach bis fünffach, gleich oder verschieden substituierten 5- oder 6-gliedrigen Heterocyclus, der gegebenenfalls ein oder zwei weitere Heteroatome, wie O, S oder NR enthalten kann, steht, wobei als Substituenten insbesondere C₁-C₄-Alkyl und C₁-C₄-Alkoxy genannt seien und wobei R für Alkyl, oder für gegebenenfalls substituiertes Aryl steht.

7. Verfahren nach einem der Ansprüche 1 bis 5 in welchem
R¹ und R² gleich oder verschieden sind und für Methyl, Ethyl, n- oder i-Propyl stehen oder
gemeinsam mit dem N-Atom, an das sie gebunden sind, für einen gegebenenfalls einfach oder zweifach gleich oder verschieden substituierten 5- oder 6-gliedrigen Heterocyclus, der gegebenenfalls ein weiteres Heteroatom, wie O, S oder NR enthalten kann, steht; wobei als Substituenten C₁-C₄-Alkyl und C₁-C₄-Alkoxy genannt seien und wobei R für C₁-C₄-Alkyl, oder für gegebenenfalls einfach oder zweifach, gleich oder verschieden substituiertes Phenyl, steht; wobei als Substituenten Halogen, C₁-C₄-Alkyl, C₁-C₄-Halogenalkyl, C₁-C₄-Alkoxy, C₁-C₄-Halogenalkoxy, C₁-C₄-Alkylthio sowie C₁-C₄-Halogenalkylthio genannt seien.

8. Verbindungen der Formel (IIa) 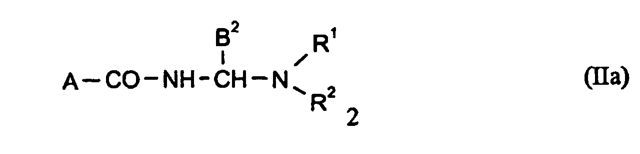 in welcher
A, R¹ und R² die in Anspruch 1 angegebene Bedeutung haben und
B² die in Anspruch 1 für B angegebene Bedeutung hat.

## Claims

1. Process for preparing compounds of the formula (I) 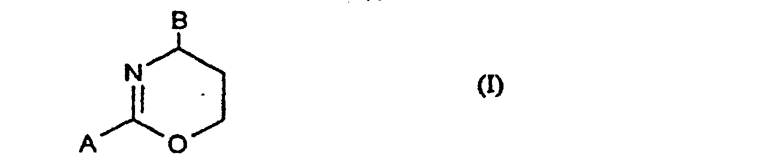 in which
A represents substituted phenyl, or represents in each case optionally substituted naphthyl, pyridyl, thienyl or pyrazolyl, and
B represents alkyl, alkoxyalkyl, alkylthioalkyl, halogenoalkyl, alkenyl, optionally substituted cycloalkyl, or in each case optionally substituted phenyl, phenylalkyl, phenylalkenyl, phenoxyalkyl, phenylthioalkyl, pyridyl or 2- or 3-pyrrolyl
are obtainable by reaction of an amide derivative of the formula (II) 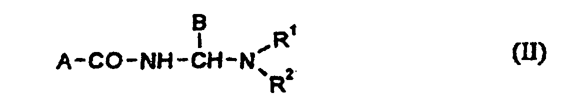 in which
A and B have the abovementioned meanings and
R¹ and R² are identical or different and in each case represent alkyl, or together with the N atom to which they are bonded represent an optionally substituted heterocycle
with ethylene in the presence of hydrogen chloride gas and of a catalyst, if appropriate in the presence of a diluent.

2. Process according to Claim 1 wherein
A represents phenyl which is monosubstituted to pentasubstituted by identical or different substituents from the series consisting of halogen, C₁-C₆-alkyl, C₁-C₆-alkoxy, C₁-C₆-alkylthio, C₁-C₆-halogenoalkyl, C₁-C₆-halogenoalkoxy, C₁-C₆-halogenoalkylthio, nitro, cyano or by phenyl which is optionally monosubstituted to pentasubstituted by identical or different substituents from the series consisting of halogen, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio,
or represents naphthyl which is optionally monosubstituted to trisubstituted by identical or different substituents from the series consisting of halogen, C₁-C₆-alkyl, C₁-C₆-alkoxy and C₁-C₆-halogenoalkoxy,
or represents pyridyl which is optionally monosubstituted to trisubstituted by identical or different substituents from the series consisting of halogen, C₁-C₆-alkyl, C₁-C₆-alkoxy, C₁-C₆-halogenoalkyl, C₁-C₆-halogenoalkoxy and cyano,
or represents thienyl which is optionally monosubstituted to trisubstituted by identical or different substituents from the series consisting of halogen and C₁-C₆-alkyl,
or represents pyrazolyl which is optionally monosubstituted to trisubstituted by identical or different substituents from the series consisting of halogen and C₁-C₃-alkyl,
B represents C₁-C₆-alkyl, C₁-C₆-alkoxy-C₁-C₆-alkyl, C₁-C₆-alkylthio-C₁-C₆-alkyl, C₁-C₆-halogenoalkyl and C₂-C₆-alkenyl,
or represents C₃-C₇-cycloalkyl which is optionally monosubstituted to pentasubstituted by identical or different substituents from the series consisting of halogen, C₁-C₆-alkyl, C₁-C₆-alkoxy, C₁-C₆-halogenoalkyl and C₁-C₆-halogenoalkoxy,
or represents pyridyl which is optionally monosubstituted to trisubstituted by identical or different substituents from the series consisting of halogen, C₁-C₆-alkyl, C₁-C₆-alkoxy, C₁-C₆-halogenoalkyl, C₁-C₆-halogenoalkoxy and cyano,
or represents α-pyrrolyl or β-pyrrolyl, each of which is optionally monosubstituted or disubstituted by identical or different substituents from the series consisting of halogen and C₁-C₄-alkyl,
or represents phenyl which is substituted by hydroxyl, CHO, cyano, carboxyl, poly-C₁-C₈-alkoxy-C₁-C₈-alkyl or C₁-C₈-alkoxycarbonyl
or represents phenyl, benzyl, pheneth-1-yl, pheneth-2-yl, phenoxymethyl, phenylthiomethyl, phenylthioeth-1-yl, phenoxyeth-1-yl, phenoxyeth-2-yl or styryl, each of which is optionally monosubstituted to pentasubstituted in the phenyl ring by identical or different substituents, suitable substituents in each case being
halogen
C₁-C₁₈-alkyl,
C₁-C₈-alkoxy-C₁-C₈-alkyl,
C₁-C₈-halogenoalkoxy,
C₁-C₄-halogenoalkyl,
C₁-C₁₈-alkoxy which is optionally interrupted by a further 1 to 3 oxygen atoms,
C₁-C₁₈-alkylthio,
C₁-C₈-halogenoalkylthio,
tri-(C₁-C₈)-alkylsilyl,
phenyl-di-(C₁-C₈)-alkylsilyl,
3,4-difluoromethylenedioxo,
3,4-tetrafluoroethylenedioxo,
a fused benzo group,
a fused C₃-C₄-alkanediyl group,
benzyliminooxymethyl which is optionally substituted by halogen, C₁-C₄-alkyl or C₃-C₆-cycloalkyl,
cyclohexyl or cyclohexyloxy, each of which is optionally substituted by C₁-C₆-alkyl, C₁-C₆-alkoxy, cyclohexyl or phenyl,
pyridyloxy which is optionally monosubstituted or disubstituted by identical or different substituents from the series consisting of halogen, C₁-C₄-alkyl and C₁-C₄-halogenoalkyl,
phenyl, phenyl-C₁-C₆-alkyl, phenoxy, phenylthio, phenyl-C₁-C₆-alkoxy or benzylthio, each of which is optionally monosubstituted to pentasubstituted by identical or different substituents from the series consisting of halogen, C₁-C₁₂-alkyl, C₁-C₄-halogenoalkyl, C₁-C₁₂-alkoxy, C₁-C₆-halogenoalkoxy, C₁-C₆-alkoxy-C₁-C₆-alkyl, C₁-C₆-alkoxy-ethyleneoxy, C₁-C₆-alkylthio and C₁-C₆-halogenoalkylthio.

3. Process according to Claim 1 wherein
A represents phenyl which is monosubstituted to tetrasubstituted by identical or different substituents from the series consisting of fluorine, chlorine, bromine, C₁-C₃-alkyl, C₁-C₃-alkoxy, C₁-C₃-alkylthio, by C₁-C₂-alkyl which is monosubstituted to pentasubstituted by identical or different substituents from the series consisting of fluorine and chlorine, by C₁-C₄-alkoxy which is monosubstituted to pentasubstituted by identical or different substituents from the series consisting of fluorine and chlorine, by SCF₃, SCHF₂, nitro, cyano, or by phenyl which is optionally monosubstituted to tetrasubstituted by identical or different substituents from the series consisting of fluorine, chlorine, bromine, C₁-C₄-alkyl, C₁-C₄-alkoxy, C₁-C₄-alkylthio, C₁-C₂-alkyl which is monosubstituted to pentasubstituted by identical or different substituents from the series consisting of fluorine and chlorine, by C₁-C₄-alkoxy which is monosubstituted to pentasubstituted by identical or different substituents from the series consisting of fluorine and chlorine, or by SCF₃ or SCHF₂,
or represents naphthyl which is optionally monosubstituted to trisubstituted by identical or different substituents from the series consisting of fluorine, chlorine, C₁-C₆-alkyl, C₁-C₆-alkoxy and C₁-C₆-alkoxy which is monosubstituted to trisubstituted by identical or different substituents from the series consisting of fluorine and chlorine,
or represents pyridyl which is optionally monosubstituted or disubstituted by identical or different substituents from the series consisting of fluorine, chlorine, C₁-C₃-alkyl, C₁-C₃-alkoxy, CF₃, OCF₃ and cyano,
or represents thienyl which is optionally monosubstituted or disubstituted by identical or different substituents from the series consisting of chlorine, bromine, methyl and ethyl,
or represents pyrazolyl which is optionally monosubstituted to trisubstituted by identical or different substituents from the series consisting of fluorine, chlorine, bromine and C₁-C₃-alkyl,
B represents C₁-C₄-alkyl, C₁-C₄-alkoxy-C₁-C₄-alkyl, C₁-C₄-alkylthio-C₁-C₄-alkyl, C₁-C₄-halogenoalkyl or C₂-C₆-alkenyl,
or represents cyclopropyl, cyclopentyl or cyclohexyl, each of which is optionally monosubstituted to trisubstituted by identical or different substituents from the series consisting of fluorine, chlorine, bromine, C₁-C₃-alkyl, C₁-C₃-alkoxy, CF₃ and OCF₃,
or represents pyridyl which is optionally monosubstituted or disubstituted by identical or different substituents from the series consisting of fluorine, chlorine, C₁-C₃-alkyl, C₁-C₃-alkoxy, CF₃, OCF₃ and cyano,
or represents α-pyrrolyl or β-pyrrolyl, each of which is optionally monosubstituted or disubstituted by identical or different substituents from the series consisting of fluorine, chlorine and C₁-C₃-alkyl,
or represents phenyl which is substituted by CHO, cyano, hydroxyl, carboxyl, poly-C₁-C₈-alkoxy-C₁-C₆-alkyl and C₁-C₈-alkoxycarbonyl or represents phenyl, benzyl, pheneth-1-yl, pheneth-2-yl, phenoxymethyl, phenylthiomethyl, phenylthioeth-1-yl, phenoxyeth-1-yl, phenoxyeth-2-yl or styryl, each of which is optionally monosubstituted to tetrasubstituted in the phenyl ring by identical or different substituents, suitable substituents in each case being fluorine, chlorine, bromine,
C₁-C₁₈-alkyl,
C₁-C₆-alkoxy-C₁-C₈-alkyl,
C₁-C₈-alkoxy which is monosubstituted to hexasubstituted by identical or different substituents from the series consisting of fluorine and chlorine,
C₁-C₂-alkyl which is monosubstituted to pentasubstituted by identical or different substituents from the series consisting of fluorine and chlorine,
C₁-C₁₈-alkoxy or-(OC₂H₄)₁₋₃-O-C₁-C₆-alkyl,
C₁-C₁₅-alkylthio,
C₁-C₈-alkylthio, which is monosubstituted to hexasubstituted by identical or different substituents from the series consisting of fluorine and chlorine,
tri-(C₁-C₆)-alkylsilyl,
phenyl-di-(C₁-C₆)-alkylsilyl,
3,4-difluoromethylenedioxo,
3,4-tetrafluoroethylenedioxo,
a fused benzo group,
a fused C₄-alkanediyl group,
the groups 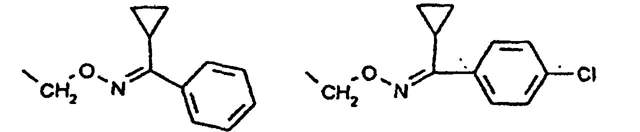 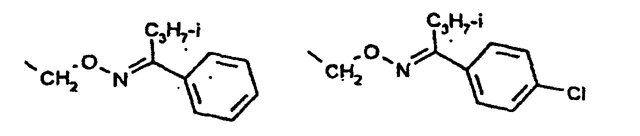 cyclohexyl or cyclohexyloxy, each of which is optionally substituted by C₁-C₄-alkyl, C₁-C₄-alkoxy, cyclohexyl or phenyl, pyridyloxy which is optionally monosubstituted or disubstituted by identical or different substituents from the series consisting of fluorine, chlorine and CF₃,
phenyl, phenyl-C₁-C₆-alkyl, phenoxy, phenylthio, phenyl-C₁-C₆-alkyloxy or benzylthio, each of which is optionally monosubstituted to tetrasubstituted by identical or different substituents from the series consisting of fluorine, chlorine, bromine, C₁-C₁₂-alkyl, C₁-C₄-alkyl which is monosubstituted to hexasubstituted by identical or different substituents from the series consisting of fluorine and chlorine, or by C₁-C₁₂-alkoxy, C₁-C₄-alkoxy, which is monosubstituted to hexasubstituted by identical or different substituents from the series consisting of fluorine and chlorine, or by C₁-C₄-alkoxy-C₁-C₄-alkyl, C₁-C₄-alkoxyethyleneoxy, C₁-C₄-alkylthio or C₁-C₄-alkylthio which is monosubstituted to hexasubstituted by identical or different substituents from the series consisting of fluorine and chlorine.

4. Process according to Claim 1 wherein
A represents phenyl which is monosubstituted to trisubstituted by identical or different substituents from the series consisting of fluorine, chlorine, bromine, C₁-C₃-alkyl, C₁-C₃-alkoxy, C₁-C₃-alkylthio, C₁-C₂-alkyl which is monosubstituted to pentasubstituted by identical or different substituents from the series consisting of fluorine and chlorine, by C₁-C₄-alkoxy which is monosubstituted to pentasubstituted by identical or different substituents from the series consisting of fluorine and chlorine, by SCF₃, SCHF₂, nitro, cyano, or by phenyl which is optionally monosubstituted to tetrasubstituted by identical or different substituents from the series consisting of fluorine, chlorine, bromine, C₁-C₃-alkyl, C₁-C₃-alkoxy, C₁-C₃-alkylthio, C₁-C₂-alkyl which is monosubstituted to pentasubstituted by identical or different substituents from the series consisting of fluorine and chlorine, by C₁-C₄-alkoxy which is monosubstituted to pentasubstituted by identical or different substituents from the series consisting of fluorine and chlorine, or by SCF₃ or SCHF₂,
B represents C₁-C₄-alkyl, C₁-C₂-alkoxy-C₁-C₄-alkyl, C₁-C₂-alkylthio- C₁-C₄-alkyl, C₁-C₂-halogenoalkyl or C₂-C₄-alkenyl,
or represents cyclopropyl, cyclopentyl or cyclohexyl, each of which is optionally monosubstituted to trisubstituted by identical or different substituents from the series consisting of fluorine, chlorine, CH₃, C₂ H₅, OCH₃, CF₃ or OCF₃,
or represents pyridyl which is optionally monosubstituted or disubstituted by identical or different substituents from the series consisting of fluorine, chlorine, CH₃, C₂H₅, OCH₃, CF₃, OCF₃ and cyano,
or represents α-pyrrolyl or β-pyrrolyl, each of which is optionally monosubstituted or disubstituted by identical or different substituents from the series consisting of fluorine, chlorine, CH₃ and C₂H₅,
or represents phenyl which is substituted by CHO, hydroxyl, cyano, carboxyl, di-C₁-C₆-alkoxy-C₁-C₄-alkyl or C₁-C₆-alkoxycarbonyl,
or represents phenyl, benzyl, pheneth-1-yl, pheneth-2-yl, phenoxymethyl, phenylthiomethyl, phenylthioeth-1-yl, phenoxyeth-1-yl, phenoxyeth-2-yl or styryl, each of which is optionally monosubstituted to trisubstituted in the phenyl ring by identical or different substituents, suitable substituents in each case being
fluorine, chlorine, bromine,
C₁-C₁₈-alkyl,
C₁-C₆-alkoxy-C₁-C₈-alkyl,
C₁-C₈-alkoxy which is monosubstituted to hexasubstituted by identical or different substituents from the series consisting of fluorine and chlorine,
C₁-C₂-alkyl which is monosubstituted to pentasubstituted by identical or different substituents from the series consisting of fluorine and chlorine,
C₁-C₁₈-alkoxy or -(OC₂H₄)₁₋₃-O-C₁-C₆-alkyl,
C₁-C₁₅-alkylthio,
C₁-C₈-alkylthio, which is monosubstituted to hexasubstituted by identical or different substituents from the series consisting of fluorine and chlorine,
tri-(C₁-C₆)-alkylsilyl,
phenyl-di-(C₁-C₆)-alkylsilyl,
3,4-difluoromethylenedioxo,
3,4-tetrafluoroethylenedioxo,
a fused benzo group,
a fused C₄-alkanediyl group,
the groups 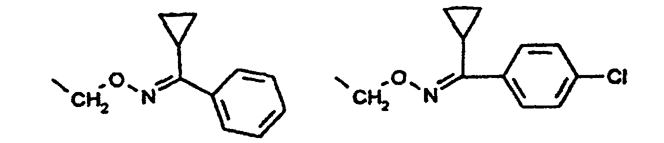 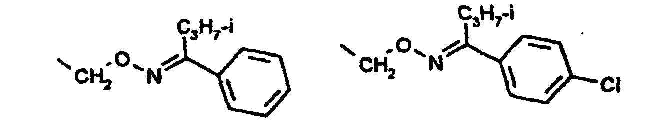 cyclohexyl or cyclohexyloxy, each of which is optionally substituted by C₁-C₄-alkyl, C₁-C₄-alkoxy, cyclohexyl or phenyl,
pyridyloxy which is optionally monosubstituted or disubstituted by identical or different substituents from the series consisting of fluorine, chlorine and CF₃,
phenyl, phenyl-C₁-C₆-alkyl, phenoxy, phenylthio, phenyl-C₁-C₆-alkyloxy or benzylthio, each of which is optionally monosubstituted to tetrasubstituted by identical or different substituents from the series consisting of fluorine, chlorine, bromine, C₁-C₁₂-alkyl, C₁-C₄-alkyl which is monosubstituted to hexasubstituted by fluorine or chlorine, or C₁-C₁₂-alkoxy, C₁-C₄-alkoxy which is monosubstituted to hexasubstituted by identical or different substituents from the series consisting of fluorine and chlorine, or by C₁-C₄-alkoxy-C₁-C₄-alkyl, C₁-C₄-alkoxy-ethyleneoxy, C₁-C₄-alkylthio or C₁-C₄-alkylthio which is monosubstituted to hexasubstituted by identical or different substituents from the series consisting of fluorine and chlorine.

5. Process according to Claim 1 wherein
A represents phenyl which is monosubstituted or disubstituted by identical or different substituents from the series consisting of fluorine and chlorine and
B represents phenyl which is monosubstituted to trisubstituted by identical or different substituents from the series consisting of fluorine, chlorine, bromine, C₁-C₈-alkyl, C₁-C₈-alkoxy, C₁-C₄-halogenoalkyl, C₁-C₄-halogenoalkoxy, C₁-C₈-alkylthio and C₃-C₆-cycloalkyl
or represents phenyl which is substituted by phenyl, phenyl-C₁-C₃-alkyl, phenyloxy or benzyloxy, each of which is optionally monosubstituted or disubstituted in the phenyl moiety by identical or different substituents from the series consisting of fluorine, chlorine, bromine, C₁-C₆-alkyl, C₁-C₆-alkoxy, C₁-C₄-halogenoalkyl and C₁-C₄-halogenoalkoxy,
or represents pyridyl or 2- or 3-pyrrolyl, each of which is optionally monosubstituted or disubstituted by identical or different substituents from the series consisting of fluorine, chlorine, methyl, ethyl and trifluoromethyl,
or represents phenyl which is substituted by CHO, hydroxyl, cyano, carboxyl, di-C₁-C₆-alkoxy-C₁-C₄-alkyl or C₁-C₆-alkoxycarbonyl.

6. Process according to any one of Claims 1 to 5 wherein
R¹ and R² are identical or different and represent C₁-C₄-alkyl or together with the N atom to which they are bonded represent a 5- or 6-membered heterocycle which is optionally monosubstituted to pentasubstituted by identical or different substituents and which can optionally contain one or two further hetero atoms, such as O, S or NR, substituents which may be mentioned being, in particular, C₁-C₄-alkyl and C₁-C₄-alkoxy, and R representing alkyl or optionally substituted aryl.

7. Process according to any one of Claims 1 to 5 wherein
R¹ and R² are identical or different and represent methyl, ethyl, n- or i-propyl or together with the N atom to which they are bonded represent a 5- or 6-membered heterocycle which is optionally monosubstituted or disubstituted by identical or different substituents and which can optionally contain one further hetero atom, such as O, S or NR, substituents which may be mentioned being C₁-C₄-alkyl and C₁-C₄-alkoxy and R representing C₁-C₄-alkyl or phenyl which is optionally monosubstituted or disubstituted by identical or different substituents, substituents which may be mentioned being halogen, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio.

8. Compounds of the formula (IIa) 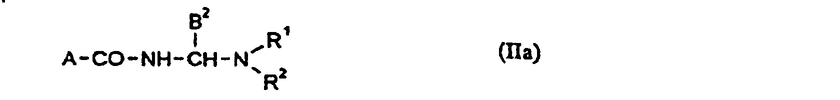 in which
A, R¹ and R² have the meanings indicated in Claim 1 and
B² has the meaning indicated for B in Claim 1.

## Revendications

1. Procédé de production de composés de formule (I) 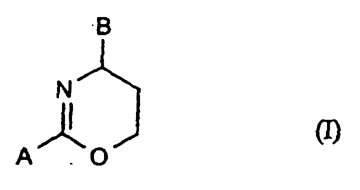 dans laquelle
A est un reste phényle substitué ou un reste naphtyle, pyridyle, thiényle ou pirazolyle dont chacun est éventuellement substitué et
B est un reste alkyle, alkoxyalkyle, alkylthioalkyle, halogénalkyle, alcényle, un reste cycloalkyle éventuellement substitué ou un reste phényle, phénylalkyle, phénylalcényle, phénoxyalkyle, phénylthioalkyle, pyridyle, 2- ou 3-pyrrolyle dont chacun est éventuellement substitué,
obtenus par réduction d'un dérivé d'amide de formule (II) 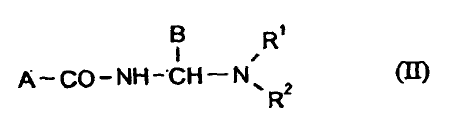 dans laquelle
A et B ont la définition indiquée ci-dessus et
R¹ et R² sont identiques ou différents et représentent chacun un reste alkyle, ou
forment un hétérocycle éventuellement substitué conjointement avec l'atome d'azote auquel ils sont liés,
avec l'éthylène en présence de chlorure d'hydrogène gazeux et d'un catalyseur, éventuellement en présence d'un diluant.

2. Procédé suivant la revendication 1, dans lequel :
A est un reste phényle qui est substitué une à cinq fois identiques ou différentes par un radical halogéno, alkyle en C₁ à C₆, alkoxy en C₁ à C₆, alkylthio en C₁ à C₆, halogénalkyle en C₁ à C₆, halogénalkoxy en C₁ à C₆, halogénalkylthio en C₁ à C₆, nitro, cyano ou par un radical phényle éventuellement substitué une à cinq fois identiques ou différentes par un radical halogéno, alkyle en C₁ à C₆, halogénalkyle en C₁ à C₆, alkoxy en C₁ à C₆, halogénalkoxy en C₁ à C₆, alkylthio en C₁ à C₆ ou halogénalkylthio en C₁ à C₆,
un reste naphtyle éventuellement substitué une à cinq fois identiques ou différentes par un radical halogéno, alkyle en C₁ à C₆, alkoxy en C₁ à C₆ ou halogénalkoxy en C₁ à C₆,
un reste pyridyle éventuellement substitué une à trois fois identiques ou différentes par un radical halogéno, alkyle en C₁ à C₆, alkoxy en C₁ à C₆, halogénalkyle en C₁ à C₆, halogénalkoxy en C₁ à C₆ ou cyano,
un reste thiényle éventuellement substitué une à trois identiques ou différentes par un radical halogéno ou alkyle en C₁ à C₆,
ou bien un reste pyrazolyle éventuellement substitué une à trois fois identiques ou différentes par un radical halogéno ou alkyle en C₁ à C₃ et
B est un reste alkyle en C₁ à C₆, (alkoxy en C₁ à C₆)-(alkyle en C₁ à C₆), (alkylthio en C₁ à C₆)-(alkyle en C₁ à C₆), halogénalkyle en C₁ à C₆ ou alcényle en C₂ à C₆,
un reste cycloalkyle en C₃ à C₇ éventuellement substitué une à cinq fois identiques ou différentes par un radical halogéno, alkyle en C₁ à C₆, alkoxy en C₁ à C₆, halogénalkyle en C₁ à C₆ ou halogénalkoxy en C₁ à C₆,
un reste pyridyle éventuellement substitué une à trois fois identiques ou différentes par un radical halogéno, alkyle en C₁ à C₆, alkoxy en C₁ à C₆, halogénalkyle en C₁ à C₆, halogénalkoxy en C₁ à C₆ ou cyano,
un reste α-pyrrolyle ou β-pyrrolyle éventuellement substitué une ou deux fois identiques ou différentes par un radical halogéno ou alkyle en C₁ à C₄,
un reste phényle substitué par un radical hydroxy, CHO, cyano, carboxyle, poly (alkoxy en C₁ à C₈)-(alkyle en C₁ à C₈) ou (alkoxy en C₁ à C₈)carbonyle, ou bien
un reste phényle, benzyle, phénéth-1-yle, phénéth-2-yle, phénoxyméthyle, phénylthiométhyle, phénylthioéth-1-yle, phénoxyéth-1-yle, phénoxyéth-2-yle ou styryle dont chacun est éventuellement substitué une à cinq fois identiques ou différentes dans le noyau phényle en considérant dans chaque cas les substituants suivants :
halogène,
alkyle en C₁ à C₁₈,
(alkoxy en C₁ à C₈)-(alkyle en C₁ à C₈),
halogénalkoxy en C₁ à C₈,
halogénalkyle en C₁ à C₄,
alkoxy en C₁ à C₁₈, qui est éventuellement interrompu par 1 à 3 autres atomes d'oxygène,
alkylthio en C₁ à C₁₈,
halogénalkylthio en C₁ à C₈,
tri(alkyle en C₁ à C₈)silyle,
phényldi(alkyle en C₁ à C₈)silyle,
3,4-difluorométhylènedioxo,
3,4-tétrafluoréthylènedioxo,
un groupe benzocondensé,
un groupe alcanediyle en C₃ ou C₄ condensé,
benzylimino-oxyméthyle éventuellement substitué par un halogène, un radical alkyle en C₁ à C₄ ou cycloalkyle en C₃ à C₆,
cyclohexyle ou cyclohexyloxy, chacun éventuellement substitué par un radical alkyle en C₁ à C₆, alkoxy en C₁ à C₆, cyclohexyle ou phényle,
pyridyloxy éventuellement substitué une ou deux fois identiques ou différentes par un radical halogéno, alkyle en C₁ à C₄ ou halogénalkyle en C₁ à C₄,
phényl, phényl(alkyle en C₁ à C₆), phénoxy, phénylthio, phényl(alkyloxy en C₁ à C₆) ou benzylthio, chacun éventuellement substitué une à cinq fois identiques ou différentes par un radical halogéno, alkyle en C₁ à C₁₂, halogénalkyle en C₁ à C₄, alkoxy en C₁ à C₁₂, halogénalkoxy en C₁ à C₆, (alkoxy en C₁ à C₆)-(alkyle en C₁ à C₆), (alkoxy en C₁ à C₆)éthylénoxy, alkylthio en C₁ à C₄ ou halogénalkylthio en C₁ à C₆.

3. Procédé suivant la revendication 1, dans lequel
A est un reste phényle qui est substitué une à quatre fois identiques ou différentes par du fluor, du chlore, du brome, un radical alkyle en C₁ à C₃, alkoxy en C₁ à C₃, alkylthio en C₁ à C₃, alkyle en C₁ ou C₂ substitué une à cinq fois identiques ou différentes par du fluor ou du chlore, alkoxy en C₁ à C₄ substitué une à cinq fois identiques ou différentes par du fluor ou du chlore, SCF₃, SCHF₂, nitro, cyano, ou phényle éventuellement substitué une à quatre fois identiques ou différentes par du fluor, du chlore, du brome, un radical alkyle en C₁ à C₄, alkoxy en C₁ à C₄, alkylthio en C₁ à C₄, alkyle en C₁ ou C₂ substitué une à cinq fois identiques ou différentes par du fluor ou du chlore, alkoxy en C₁ à C₄ substitué une à cinq fois identiques ou différentes par du fluor ou du chlore, SCF₃ ou SCHF₂,
un reste naphtyle éventuellement substitué une à trois fois identiques ou différentes par du fluor, du chlore, un radical alkyle en C₁ à C₆, alkoxy en C₁ à C₆, ou un radical alkoxy en C₁ à C₆ substitué une à trois identiques ou différentes par du fluor ou du chlore,
un reste pyridyle éventuellement substitué une ou deux fois identiques ou différentes par du fluor, du chlore, un radical alkyle en C₁ à C₃, alkoxy en C₁ à C₃, CF₃, OCF₃ ou cyano,
un reste thiényle éventuellement substitué une ou deux fois identiques ou différentes par du chlore, du brome, un radical méthyle ou éthyle, ou bien
un reste pyrazolyle éventuellement substitué une à trois fois identiques ou différentes par du fluor, du chlore, du brome ou un radical alkyle en C₁ à C₃, et
B est un reste alkyle en C₁ à C₄, (alkoxy en C₁ à C₄)-(alkyle en C₁ à C₄), (alkylthio en C₁ à C₄)-(alkyle en C₁ à C₄), halogénalkyle en C₁ à C₄ ou alcényle en C₂ à C₆,
un reste cyclopropyle, cyclopentyle ou cyclohexyle, chacun éventuellement substitué une à trois fois identiques ou différentes par du fluor, du chlore, du brome, un radical alkyle en C₁ à C₃, alkoxy en C₁ à C₃, CF₃ ou OCF₃,
un reste pyridyle éventuellement substitué une ou deux fois identiques ou différentes par du fluor, du chlore, un radical alkyle en C₁ à C₃, alkoxy en C₁ à C₃, CF₃, OCF₃ ou cyano,
un reste α-pyrrolyle ou β-pyrrolyle, chacun éventuellement substitué une ou deux fois identiques ou différentes par du fluor, du chlore ou un radical alkyle en C₁ à C₃,
un reste phényle substitué par un radical CHO, cyano, hydroxy, carboxyle, poly(alkoxy en C₁ à C₈)-(alkyle en C₁ à C₆) ou (alkoxy en C₁ à C₈)carbonyle, ou bien
un reste phényle, benzyle, phénéth-1-yle, phénéth-2-yle, phénoxyméthyle, phénylthiométhyle, phénylthioéth-1-yle, phénoxyéth-1-yle, phénoxyéth-2-yle ou styryle, chacun éventuellement substitué une à quatre fois identiques ou différentes dans le noyau phényle, en considérant dans chaque cas les substituants suivants :
fluor, chlore, brome,
alkyle en C₁ à C₁₈,
(alkoxy en C₁ à C₆)-(alkyle en C₁ à C₈),
alkoxy en C₁ à C₈ substitué une à six fois identiques ou différentes par du fluor ou du chlore,
alkyle en C₁ ou C₂ substitué une à cinq fois identiques ou différentes par du fluor ou du chlore,
alkoxy en C₁ à C₁₈ ou -(OC₂H₄)₁₋₃-O-(alkyle en C₁ à C₆),
alkylthio en C₁ à C₁₅,
alkylthio en C₁ à C₈ substitué une à six fois identiques ou différentes par du fluor ou du chlore,
tri(alkyle en C₁ à C₆)silyle,
phényldi(alkyle en C₁ à C₆)silyle,
3,4-difluorométhylènedioxo,
3,4-tétrafluoréthylènedioxo,
un noyau benzocondensé,
un groupe alcanediyle en C₄ condensé,
les groupes 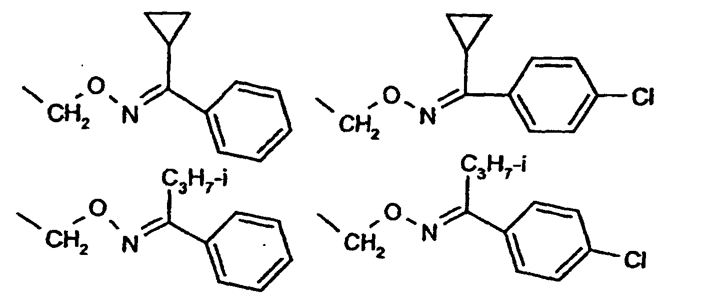 cyclohexyle ou cyclohexyloxy, chacun éventuellement substitué par un radical alkyle en C₁ à C₄, alkoxy en C₁ à C₄, cyclohexyle ou phényle,
pyridyloxy éventuellement substitué une ou deux fois identiques ou différentes par du fluor, du chlore ou un radical CF₃,
phényle, phényl(alkyle en C₁ à C₆), phénoxy, phénylthio, phényl(alkyloxy en C₁ à C₆) ou benzylthio, chacun éventuellement substitué une à quatre fois identiques ou différentes par du fluor, du chlore, du brome, un radical alkyle en C₁ à C₁₂, alkyle en C₁ à C₄ éventuellement substitué une à six fois identiques ou différentes par du fluor ou du chlore, alkoxy en C₁ à C₁₂, alkoxy en C₁ à C₄ éventuellement substitué une à six fois identiques ou différentes par du fluor ou du chlore, (alkoxy en C₁ à C₄)-(alkyle en C₁ à C₄), (alkoxy en C₁ à C₄)éthylénoxy, alkylthio en C₁ à C₄ ou alkylthio en C₁ à C₄ substitué une à six fois identiques ou différentes par du fluor ou du chlore.

4. Procédé suivant la revendication 1, dans lequel
A est un reste phényle qui est substitué une à trois fois identiques ou différentes par du fluor, du chlore, du brome, un radical alkyle en C₁ à C₃, alkoxy en C₁ à C₃, alkylthio en C₁ à C₃, un radical alkyle en C₁ ou C₂ substitué une à cinq fois identiques ou différentes par du fluor ou du chlore, un radical alkoxy en C₁ à C₄ substitué une à cinq fois identiques ou différentes par du fluor ou du chlore, un radical SCF₃, SCHF₂, nitro, cyano, ou un radical phényle éventuellement substitué une à quatre fois identiques ou différentes par du fluor, du chlore, du brome, un radical alkyle en C₁ à C₃, alkoxy en C₁ à C₃, alkylthio en C₁ à C₃, un radical alkyle en C₁ ou C₂ substitué une à cinq fois identiques ou différentes par du fluor ou du chlore, un radical alkoxy en C₁ à C₄ substitué une à cinq fois identiques ou différentes par du fluor ou du chlore, un radical SCF₃ ou SCHF₂, et
B est un reste alkyle en C₁ à C₄, (alkoxy en C₁ ou C₂)-(alkyle en C₁ à C₄), (alkylthio en C₁ ou C₂)-(alkyle en C₁ à C₄), halogénalkyle en C₁ ou C₂ ou alcényle en C₂ à C₄,
un reste cyclopropyle, cyclopentyle ou cyclohexyle chacun éventuellement substitué une à trois fois identiques ou différentes par du fluor, du chlore, un radical CH₃, C₂H₅, OCH₃, CF₃ ou OCF₃,
un reste pyridyle éventuellement substitué une ou deux fois identiques ou différentes par du fluor, du chlore, un radical CH₃, C₂H₅, OCH₃, CF₃, OCF₃ ou cyano,
un reste α-pyrrolyle ou β-pyrrolyle dont chacun est éventuellement substitué une ou deux fois identiques ou différentes par du fluor, du chlore, un radical CH₃ ou C₂H₅,
un reste phényle substitué par un radical CHO, hydroxy, cyano, carboxyle, di(alkoxy en C₁ à C₆)-(alkyle en C₁ à C₄) ou (alkoxy en C₁ à C₆)carbonyle, ou bien
un reste phényle, benzyle, phénéth-1-yle, phénéth-2-yle, phénoxyméthyle, phénylthiométhyle, phénylthioéth-1-yle, phénoxyéth-1-yle, phénoxy-éth-2-yle ou styryle dont chacun est éventuellement substitué une à trois fois identiques ou différentes dans le noyau phényle, en considérant dans chaque cas les substituants suivants :
fluor, chlore, brome,
alkyle en C₁ à C₁₈,
(alkoxy en C₁ à C₆)-(alkyle en C₁ à C₈),
alkoxy en C₁ à C₈ substitué une à six fois identiques ou différentes par du fluor ou du chlore,
alkyle en C₁ ou C₂ substitué une à cinq fois identiques ou différentes par du fluor ou du chlore,
alkoxy en C₁ à C₁₈ ou -(OC₂H₄)₁₋₃-O-(alkyle en C₁ à C₆),
alkylthio en C₁ à C₁₅,
alkylthio en C₁ à C₈ substitué une à six fois identiques ou différentes par du fluor ou du chlore,
tri(alkyle en C₁ à C₆)silyle,
phényldi(alkyle en C₁ à C₆)silyle,
3,4-difluorométhylènedioxo,
3,4-tétrafluoréthylènedioxo,
un noyau benzocondensé,
un groupe alcanediyle en C₄ condensé,
les groupes 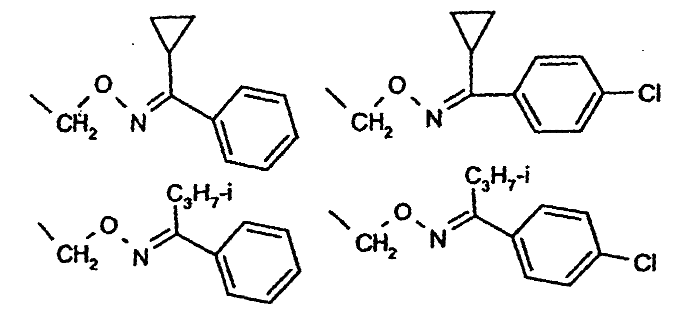
un reste cyclohexyle ou cyclohexyloxy dont chacun est éventuellement substitué par un radical alkyle en C₁ à C₄, alkoxy en C₁ à C₄, cyclohexyle ou phényle,
un reste pyridyloxy éventuellement substitué une ou deux fois identiques ou différentes par du fluor, du chlore ou un radical CF₃,
un reste phényle, phényl(alkyle en C₁ à C₆), phénoxy, phénylthio, phényl(alkyloxy en C₁ à C₆) ou benzylthio dont chacun est éventuellement substitué une à quatre fois identiques ou différentes par du fluor, du chlore, du brome, un radical alkyle en C₁ à C₁₂, alkyle en C₁ à C₄ substitué une à six fois par du fluor ou du chlore, alkoxy en C₁ à C₁₂, alkoxy en C₁ à C₄ substitué une à six fois identiques ou différentes par du fluor ou du chlore, (alkoxy en C₁ à C₄)-(alkyle en C₁ à C₄), (alkoxy en C₁ à C₄)éthylénoxy, alkylthio en C₁ à C₄ ou alkylthio en C₁ à C₄ substitué une à six fois identiques ou différentes par du fluor ou du chlore.

5. Procédé suivant la revendication 1, dans lequel
A est un reste phényle éventuellement substitué une ou deux fois identiques ou différentes par du fluor ou du chlore et
B est un reste phényle substitué une à trois fois identiques ou différentes par du fluor, du chlore, du brome, un radical alkyle en C₁ à C₈, alkoxy en C₁ à C₈, halogénalkyle en C₁ à C₄, halogénalkoxy en C₁ à C₄, alkylthio en C₁ à C₈ ou cycloalkyle en C₃ à C₆, ou bien
un reste phényle substitué une fois par un radical phényle, phényl(alkyle en C₁ à C₃), phényloxy ou benzyloxy, dont chacun est éventuellement substitué une ou deux fois identiques ou différentes dans la partie phényle par un radical fluoro, chloro, bromo, alkyle en C₁ à C₆, alkoxy en C₁ à C₆, halogénalkyle en C₁ à C₄ ou halogénalkoxy en C₁ à C₄,
un reste pyridyle, 2- ou 3-pyrrolyle, chacun éventuellement substitué une ou deux fois identiques ou différentes par du fluor, du chlore, un radical méthyle, éthyle ou trifluorométhyle, ou bien
un reste phényle substitué par un radical CHO, hydroxy, cyano, carboxyle, di(alkoxy en C₁ à C₆)-(alkyle en C₁ à C₄) ou (alkoxy en C₁ à C₆)carbonyle.

6. Procédé suivant l'une des revendications 1 à 5, dans lequel
R¹ et R² sont identiques ou différents et représentent un reste alkyle en C₁ à C₄ ou forment conjointement avec l'atome d'azote auquel ils sont liés, un hétérocyle pentagonal ou hexagonal éventuellement substitué une à cinq fois identiques ou différentes, qui peut contenir éventuellement un ou deux autres hétéro-atomes tels que O, S ou NR, les substituants que l'on peut mentionner étant en particulier des restes alkyle en C₁ à C₄ et alkoxy en C₁ à C₄, et R étant un reste alkyle, ou un reste aryle éventuellement substitué.

7. Procédé suivant l'une des revendications 1 à 5, dans lequel
R¹ et R² sont identiques ou différents et représentent un reste méthyle, éthyle, n-propyle ou isopropyle, ou forment conjointement avec l'atome d'azote auquel ils sont liés, un hétérocycle pentagonal ou hexagonal éventuellement substitué une ou deux fois identiques ou différentes, qui peut contenir éventuellement un autre hétéro-atome tel que O, S ou NR, les substituants que l'on peut mentionner étant des restes alkyle en C₁ à C₄ et alkoxy en C₁ à C₄, et R étant un reste alkyle en C₁ à C₄ ou un reste phényle éventuellement substitué une ou deux fois identiques ou différentes, les subsituants que l'on peut mentionner étant un halogène, un radical alkyle en C₁ à C₄, halogénalkyle en C₁ à C₄, alkoxy en C₁ à C₄, halogénalkoxy en C₁ à C₄, alkylthio en C₁ à C₄ ainsi qu'halogénalkylthio en C₁ à C₄.

8. Composés de formule (IIa) 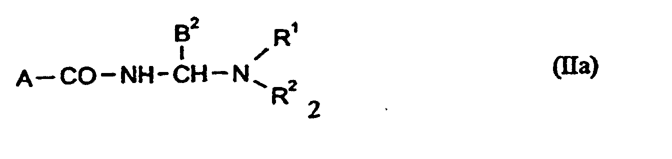 dans laquelle
A, R¹ et R² ont la définition indiquée dans la revendication 1 et
B² a la définition indiquée pour B dans la revendication 1.
